# EUROPEAN PATENT APPLICATION

(11) **EP 1 704 922 A2**
(43) Date of publication of application: **27.09.2006**
(21) Application number: 05020549.1
(22) Date of filing: 04.06.1997
(51) Int. Cl.: B01L 3/14, C12Q 1/68

(54) **System and methods for monitoring PCR processes**

(30) Priority: 04.06.1996 US 658993
(62) Divisional of application: 97926890.1
(71) Applicant: University of Utah Research Foundation, Salt Lake City, Utah 84112 (US)
(72) Inventor: Wittwer, Carl T., Salt Lake City, UT 84109 (US)
(74) Representative: Elsy, David

(57) **Abstract**

A thermal cycling method and device is disclosed. The device comprises a sample chamber whose temperature can be rapidly and accurately modulated over a range of temperatures needed to carry out a number of biological procedures, such as the DNA polymerase chain reaction. Biological samples are placed in glass micro capillary tubes and then located inside the sample chamber. A programmable controller regulates the temperature of the sample inside the sample chamber. Monitoring of the DNA amplification is monitored by fluorescence once per cycle or many times per cycle. The present invention provides that fluorescence monitoring of PCR is a powerful tool for DNA quantification.

## Description

### 1. The Field of the Invention.

This invention relates generally to apparatus for rapid control of the PCR sample with monitoring of the progress of the reaction. More specifically, the present invention relates to thermal cycling apparatus which allows rapid PCR to be rapidly carried out while monitoring the reaction.

### 2. The Background Art.

In numerous areas of industry, technology, and research there is a need to reliably and reproducibly subject relatively small samples to thermal cycling. The need to subject a sample to repeated thermal cycles is particularly acute in biotechnology applications. In the biotechnology field, it is often desirable to repeatedly heat and cool small samples of materials over a short period of time. One such biological process that is regularly carried out is cyclic DNA amplification.

Cyclic DNA amplification, using a thermostable DNA polymerase, allows automated amplification of primer specific DNA, widely known as the "polymerase chain reaction." Automation of this process requires controlled and precise thermal cycling of reaction mixtures usually contained in a plurality of containers. In the past, the container of preference has been a standard, plastic microfuge tube.

Commercial programmable metal heat blocks have been used in the past to effect the temperature cycling of samples in microfuge tubes through the desired temperature versus time profile. However, the inability to quickly and accurately adjust the temperature of the heat blocks through a large temperature range over a short time period, has rendered the use of heat block type devices undesirable as a heat control system when carrying out the polymerase chain reaction.

Moreover, the microfuge tubes which are generally used have disadvantages. The material of the microfuge tubes, their wall thickness, and the geometry of microfuge tubes is a hindrance to rapid heating and cooling of the sample contained therein. The plastic material and the thickness of the wall of microfuge tubes act as an insulator between the sample contained therein and the surrounding medium thus hindering transfer of thermal energy. Also, the geometry of the microfuge tube presents a small surface area to whatever medium is being used to transfer thermal energy. The continued use of microfuge tubes in the art, with their suboptimal geometry, indicates that the benefits of improved thermal transfer (which come by increasing the surface area of a sample container for a sample of constant volume) has heretofore not been recognized.

Furthermore, devices using water baths with fluidic switching, (or mechanical transfer) have also been used as a thermal cycler for the polymerase chain reaction. Although water baths have been used in cycling a polymerase chain reaction mixture through a desired temperature versus time profile necessary for the reaction to take place, the high thermal mass of the water (and the low thermal conductivity of plastic microfuge tubes), has been significantly limiting as far as performance of the apparatus and the yields of the reaction are concerned.

Devices using water baths are limited in their performance. This is because the water's thermal mass significantly restricts the maximum temperature versus time gradient which can be achieved thereby. Also, the water bath apparatus has been found to be very cumbersome due to the size and number of water carrying hoses and external temperature controlling devices for the water. Further the need for excessive periodic maintenance and inspection of the water fittings for the purpose of detecting leaks in a water bath apparatus is tedious and time consuming. Finally, it is difficult with the water bath apparatus to control the temperature in the sample tubes with the desired accuracy.

U.S. Patent No. 3,616,264 to Ray shows a thermal forced air apparatus for cycling air to heat or cool biological samples to a constant temperature. Although the Ray device is somewhat effective in maintaining a constant temperature within an air chamber, it does not address the need for rapidly adjusting the temperature in a cyclical manner according to a temperature versus time profile such as is required for biological procedures such as the polymerase chain reaction.

U.S. Patent No. 4,420,679 to Howe and U.S. Patent No. 4,286,456 to Sisti et al. both disclose gas chromatographic ovens. The devices disclosed in the Howe and Sisti et al. patents are suited for carrying out gas chromatography procedures but do not provide thermal cycling which is substantially any more rapid than that provided by any of the earlier described devices. Rapid thermal cycling is useful for carrying out many procedures. Devices such as those described in the Howe and Sisti et al. patents are not suitable for efficiently and rapidly carrying out such reactions quickly.

In particular, the polymerase chain reaction (PCR) is fundamental to molecular biology and is the first practical molecular technique for the clinical laboratory. Despite its usefulness and popularity, the current understanding of PCR is not highly advanced. Amplifications must be optimized by trial and error and protocols are often followed blindly. The limited understanding of PCR found in the art is a good example of how those skilled in the art are content to utilize a powerful technique without reflection or comprehension.

PCR requires temperature cycling of the sample. Not only does the prior art, as explained above, carry out temperature cycling slowly, the prior art also ignores the underlying principles which allow PCR to work and could be used to make PCR even more useful. Thus, it would be an great advance in the art to provide methods and apparatus which are particularly adaptable for rapidly carrying out PCR and analyzing the reaction which is taking place, particularly if such reaction is analyzed as it is taking place, that is in real time.

### BRIEF SUMMARY AND OBJECTS OF THE INVENTION

In view of the above described state of the art, the present invention seeks to realize the following objects and advantages.

It is an object of the present invention to provide an apparatus for accurately controlling the temperature of biological samples.

It is a further object of the present invention to provide a thermal cycling apparatus for quickly and accurately varying the temperature of biological samples according to a predetermined temperature versus time profile.

It is another object of the present invention to provide an apparatus suitable for subjecting a number of different biological samples to rapid thermal cycling.

It is also an object of the present invention to provide a thermal cycling apparatus having a thermal transfer medium of low thermal mass which can effectively subject samples to a large temperature gradient over a very short period of time.

It is a further object of the present invention to provide an apparatus which can subject a biological sample to rapid thermal cycling using air as a thermal transfer medium.

It is another object of the present invention to provide a thermal cycling apparatus which will heat samples located in a fluid chamber therein, by means of an internal heater, and will subsequently cool the samples by moving ambient fluid into the chamber, at the proper time in the thermal cycle, to cool the samples.

It is an object of the present invention to provide a system and method for performing PCR rapidly and for simultaneously monitoring the reaction.

It is another object of the present invention to provide a system and method for performing PCR rapidly and also continuously monitor the reaction and adjusts the reaction parameters while the reaction is ongoing.

These and other objects and advantages of the invention will become more fully apparent from the description and claims which follow, or may be learned by the practice of the invention.

The present invention is an apparatus particularly suited for subjecting biological samples to rapid thermal cycling in order to carry out one or more of a number of procedures or processes. In one of its preferred forms, the apparatus includes a means for holding a biological sample. The means for holding a biological sample, or a sample chamber, is provided with an insulation means for retaining thermal energy and also a means for heating the interior of the sample chamber. Preferably, a high wattage incandescent lamp functions as a means for heating the interior of the sample chamber. A thermal insulator lines the interior of the sample chamber and functions to retain the heat generated by the lamp within the sample chamber and serves as an insulation means.

In order to rapidly cool the sample chamber, the preferred apparatus includes a means for forcing air into the sample chamber and a means for dispersing the air forced into the sample chamber. A high velocity fan functions to force air into the sample chamber and a rotating paddle functions to disperse the air into the chamber. A means for venting allows the air to escape from the sample chamber taking the unwanted heat with it. The present invention allows heating and cooling of a sample to take place both quickly and uniformly.

The apparatus of the present invention includes a control means for operating the apparatus through the desired time versus temperature profile. The present invention is particularly well suited for carrying out automated polymerase chain reactions.

Other embodiments of the present invention include a closed loop hot fluid compartment and a reaction compartment. The reaction compartment is located within the hot fluid compartment and can be accessed through a venting door for allowing the insertion of samples in capillary tubes which may contain a reaction mixture for the polymerase chain reaction. A heating coil is also located in the compartment and is regulated by a programmable set-point process controller via a thermocouple sensor which is also located in the compartment at a position directly adjacent the reaction compartment.

The heat coil is located up-stream of the reaction compartment, and a fan is located up-stream of the heat coil, such that, fluid blown across the heat coil by the blower unit passes through the reaction compartment and into the intake side of the blower unit in closed loop fashion. Baffles may be located between the heat coil and the reaction compartment in order to cause uniform homogenous mixing of the heated fluid before it passes through the reaction compartment.

Alternately, the fan may be placed downstream of the heating coil but before the reaction compartment. In this case, the fan blades serve as baffles to mix the heated fluid. At the correct time in the predetermined thermal cycle, the controller activates a solenoid that opens the venting door which vents fluid out of the compartment and allows cool (ambient) fluid to enter and cool the samples.

The controller of the present invention allows the chamber, and subsequently the samples located in the sample compartment therein, to pass through a predetermined temperature cycle corresponding to the denaturation, annealing and elongation steps in the polymerase chain reaction. In use, the apparatus of the present invention allows rapid optimization of denaturation, annealing, and elongation steps in terms of time and temperature, and shortened time periods (ramp times) between the temperatures at each step.

The present invention particularly decreases the total time required for completion of polymerase chain reaction cycling over prior art thermal cycling devices while at the same time significantly increasing specificity and yield.

In accordance with another aspect of the present invention, the present invention provides methods and apparatus for continuous fluorescence monitoring of DNA amplification. In preferred embodiments of the present invention, optical components are combined with structures to provide rapid temperature cycling in order to continuously monitor DNA amplification by a variety of different fluorescence techniques. Glass capillary sample tubes, and composite plastic/glass sample tubes, allow rapid heat transfer from air (30 cycles in less than 15 min) and simultaneous optical monitoring of the reaction. Fluorescence is acquired continuously from a single sample or alternately from multiple samples on a rotating carousel with all of the samples being simultaneously subjected to rapid thermal cycling.

In accordance with another aspect of the present invention, fluorescence during DNA amplification was monitored by: 1) the double strand-specific dye SYBR™ Green 1, 2) a decrease in fluorescein quenching by rhodamine after exonuclease cleavage of a dual-labeled hybridization probe, and 3) resonance energy transfer of fluorescein to Cy5™ by adjacent hybridization probes. Fluorescence data acquired once per cycle allow quantification of initial template copy number if fluorescence specificity and amplification efficiency are known.

Furthermore, in contrast to measuring fluorescence once per cycle, embodiments of the present invention are disclosed which monitor temperature, time and fluorescence continuously throughout each cycle thus producing a 3-dimensional spiral. This 3-dimensional spiral can be reduced to temperature vs. time, fluorescence vs. time, and fluorescence vs. temperature plots. Fluorescence vs. temperature plots of hybridization probes discriminate between the cumulative, irreversible signal of exonuclease cleavage and the temperature-dependent, reversible hybridization of adjacent probes. Using double strand dyes, product denaturation, reannealing and extension can be followed within each cycle:

The present invention provides that fluorescence monitoring of PCR is a powerful tool for DNA quantification. Cycle-by-cycle monitoring using dsDNA dyes does not require unique probes and allows quantification over a large dynamic range. Sequence specific fluorescent probes offer enhanced specificity and can be used to quantitate very low copy numbers of template. There are at least two classes of sequence specific, fluorescent oligonucleotide probes that are useful in PCR. The mechanism of signal generation naturally separates them into either hydrolysis or hybridization probes. By monitoring the fluorescence once each cycle, either probe system can be used for quantitation. Continuous monitoring of fluorescence allows acquisition of melting curves and product annealing curves during temperature cycling.

In accordance with another aspect of the present invention, fluorescence monitoring optical components are combined with a rapid thermal cycler to be used to acquire DNA melting curves during PCR by fluorescence monitoring of double-strand DNA specific dyes. Plotting fluorescence as a function of temperature as the thermal cycler heats through the dissociation temperature of the product gives a DNA melting curve. The shape and position of this DNA melting curve is a function of GC/AT ratio, length, and sequence, and can be used to differentiate amplification products separated by less than 2°C in melting temperature. Desired products can be distinguished from undesired products, including primer dimers. Analysis of melting curves can be used to extend the dynamic range of quantitative PCR. The present invention provides apparatus and methods for rapid cycle PCR with combined amplification and complete quantitative analysis in under fifteen minutes and more preferably in under ten minutes.

The present invention provides a method for real time monitoring of a polymerase chain reaction amplification of a target DNA sequence and, as well, a method for analyzing a target DNA sequence of a biological sample for DNA sequence polymorphisms, heterozygosity or mutations. The method comprises the steps of amplifying the target sequence by polymerase chain reaction in the presence of two nucleic acid probes that hybridize to adjacent regions of the target sequence, exciting the sample with light at a wavelength absorbed by the donor fluorophore and detecting the fluorescence emission from the sample. One of the probes is labeled with an acceptor fluorophore and the other probe is labeled with a donor fluorophore of a fluorescence energy transfer pair such that upon hybridization of the two probes with the target sequence, the donor and acceptor fluorophores are within 0 to 25 nucleotides of one another. The polymerase chain reaction itself comprises the steps of adding a thermostable polymerase and primers for the targeted nucleic acid sequence to the biological sample and thermally cycling the biological sample between at least a denaturation temperature and an elongation temperature. In one alternative embodiment the fluorescence from the sample is monitored as a function of the temperature of the sample. In a preferred embodiment the donor fluorophore is fluorescein. one acceptor fluorophore particularly suitable for use with fluorescein is Cy5.

As another embodiment of this invention there is provided a method of real time monitoring of a polymerase chain reaction amplification of a targeted nucleic acid sequence in a biological sample. This method embodiment is also useful for analyzing a target DNA sequence in a biological sample for DNA sequence polymorphisms, heterozygosity or mutations. The method comprises the steps of
(a) adding to the biological sample an effective amount of two nucleic acid primers and a nucleic acid probe, wherein one of said primers and the probe are each labeled with one member of a fluorescence energy transfer pair comprising an acceptor fluorophore and a donor fluorophore, and wherein the labeled probe hybridizes to an amplified copy of the target nucleic acid sequence within 0 to 25 nucleotides of the labeled primer;
(b) amplifying the targeted nucleic acid sequence by polymerase chain reaction, said polymerase chain reaction comprising the steps of adding a thermostable polymerase and primers for the targeted nucleic acid sequence and thermally cycling the biological sample between at least a denaturation temperature and an elongation temperature;
(c) illuminating the biological sample with light of a selected wavelength that is absorbed by said donor fluorophore and detecting the fluorescence emission of the sample.

That method can be of further advantage by employing the additional step of monitoring the temperature dependent fluorescence of the sample.

Data relating to the temperature dependent fluorescence of the biological sample can be used as basis for adjustment of the thermal cycling conditions to optimize yield or specificity of the reaction. Thus there is also provided an improved method of amplifying a targeted nucleic acid sequence of a biological sample, wherein the method comprises the steps of
(a) adding to the biological sample an effective amount of two nucleic acid probes that hybridize to adjacent regions of the target sequence, one of said probes being labeled with an acceptor fluorophore and the other probe labeled with a donor fluorophore of a fluorescence resonance energy transfer pair such that upon hybridization of the two probes with the target sequence, the donor and acceptor fluorophores are within 0 to 25 nucleotides of one another;
(b) amplifying the targeted nucleic acid sequence using polymerase chain reaction, comprising thermally cycling the biological sample using predetermined temperature and time parameters;
(c) illuminating the biological sample with a selected wavelength of light that is absorbed by said acceptor fluorophore during the polymerase chain reaction;
(d) monitoring fluorescent emissions from said sample; and
(e) adjusting the temperature and time parameters in accordance with the data generated from step (d) to optimize product yield or specificity.

In each of the methods of this invention utilizing a fluorescence resonance energy transfer pair as a source of a fluorescent signal indicative of the status of a polymerase chain reaction, good results are obtained where the resonance energy transfer pair comprises fluorescein as the donor and Cy5 as the acceptor. The methods are carried out advantageously in a device designed to thermally cycle biological samples in optically transparent capillary tubes at high temperature ramp rates, with minimum hold times at a maximum temperature, and wherein sample fluorescence is measured or detected through the end of the capillary tube.

The present invention also provides a device for monitoring the fluorescence of a sample. The device comprises
a chamber;
a capillary tube having a high surface area to volume ratio and comprising an optically transparent material;
a capillary tube holder;
a light source mounted in said chamber and positioned to illuminate the capillary tube through an end of the capillary tube; and
a light detector mounted in said chamber and positioned to measure fluorescence from the capillary tube through an end of the capillary tube.

In one embodiment of that device the capillary tube holder is in the form of a carousel for holding a plurality of capillary tubes. The carousel is rotatably mounted in said chamber, and the device further comprises a stepper motor for rotating the carousel; and means for coupling said carousel to said motor. In another embodiment the device is also provided with a heater and a fan in air flow communication with the chamber. The heater and the fan are controlled by a programmable controller to rapidly cycle the temperature of the chamber .

The present invention is also directed to a device for conducting PCR reactions. The device comprises
a chamber;
a heater and a fan mounted in said device and in air flow communication with the chamber;
carousel for holding a plurality of capillary tubes, said carousel being rotatably mounted in said chamber, said capillary tubes comprising an optically transparent material;
a light source mounted in said chamber and positioned to illuminate at least one of the capillary tubes through an end of a capillary tube; and
a light detector mounted in said chamber and positioned to measure fluorescence from at least one of the capillary tubes through an end of a capillary tube.

There is also provided a method for enhancing detection and efficiency of acquisition of fluorescence in a sample comprising a fluorophore. The method makes use of total internal reflection to optimize acquisition and monitoring of fluorescent emissions. The method comprises the steps of placing the sample in a capillary tube comprising an optically transparent material and detecting fluorescence from the sample through an end of the capillary tube. The ratio of he surface area to the volume of the capillary tube is preferably greater than or equal to 4mm⁻¹. In the circumstance where the fluorescence is induced by fluorophore-excitatory illumination of the sample, the method further comprises the step of illuminating the sample through an end of the capillary tube, preferably along the optical path of the detected fluorescence.

In one preferred embodiment the fluorescence of the biological sample is temperature dependent and the method further comprises the step of heating or cooling the biological sample during detection of fluorescence to monitor the temperature dependent fluorescence in the sample. The sample can contain a fluorescence energy transfer pair, and the fluorescence of at least one fluorophore of the fluorescence energy transfer pair is detected. In one embodiment the sample comprises nucleic acids including nucleic acid probes. One of said probes is labeled with an acceptor fluorophore, and the other probe is labeled with a donor fluorophore of a fluorescence energy transfer pair. The fluorescence detected is that of the donor fluorophore or the acceptor fluorophore or both.

Another aspect of this invention is a handling system for a sample. The system comprises a capillary tube having a sample delivery port and a funnel cap for filling said capillary tube. The capillary tube comprises an optically transparent material, wherein the ratio of the surface area to the volume of the capillary tube is greater than or equal to 4mm⁻¹. The funnel cap has a sample receiving orifice and a sample transfer orifice and means for releasably engaging the sample delivery port of the capillary tube so that the sample transfer port of the funnel cap and the sample delivery port of the capillary tube are in alignment. The sample handling system can also include a plug for frictional fit sealing engagement with the sample receiving port of the funnel cap.

Another important embodiment of this invention is a system for performing PCR and monitoring the reaction in real time. The system comprises
a chamber;
a heater and a fan mounted in said device and in air flow communication with the chamber and a controller for cycling the temperature in the chamber according to initial predefined temperature and time parameters;
a carousel for holding a plurality of capillary sample tubes, said carousel being rotatably mounted in said chamber, said capillary tubes comprising an optically transparent material, and having a high surface area to volume ratio that provides for rapid thermal transfer;
a light source mounted in said chamber and positioned to illuminate at least one of said capillary tubes through an end of the capillary tube; and
a light detector mounted in said chamber and positioned to measure fluorescence from at least one of said capillary tubes through an end of the capillary tube;
means for displaying the status of the reaction based detected fluorescence.

In one preferred embodiment the system further comprises means for adjusting the controller such that one or more reaction parameters are adjusted in real time. The capillary sample tubes can have an inner diameter ranging from about 0.02mm to about 1.0mm. In another embodiment the device further comprises a stepper motor for rotating the carousel to position the capillary tubes held by said carousel for illumination and fluorescence detection, preferably through an end of the capillary sample tubes.

### BRIEF DESCRIPTION OF THE DRAWINGS

In order to better appreciate how the above-recited and other advantages and objects of the invention are obtained, a more particular description of the invention briefly described above will be rendered by reference to specific embodiments thereof which are illustrated in the appended drawings. Understanding that these drawings depict only a typical embodiment of the invention and are not therefore to be considered limiting of its scope, the invention will be described and explained with additional specificity and detail through the use of the accompanying drawings in which:
Figure 1 shows a perspective view of a thermal cycling apparatus adapted for thermal cycling of biological samples and adapted especially for use in cyclic DNA amplification, according to the concepts of the present invention.
Figure 2 is a side elevation view of the fluid chamber portion of the apparatus of Figure 1.
Figure 3 is an interior plan view of the fluid chamber portion of the apparatus illustrated in Figure 1.
Figure 4 shows an interior plan view of the fluid chamber of another embodiment of the present invention.
Figure 5 shows an optimized temperature versus time profile for a polymerase chain reaction using the thermal cycling device of the present invention.
Figure 6 shows graphically the effect of annealing time on polymerase chain reaction specificity and yields using the thermal cycling device of the present invention.
Figure 7 shows graphically the effect of denaturation time on polymerase chain reaction yields using the thermal cycling device of the present invention.
Figures 8A-B, which are perspective and elevational cross sectioned views, respectively, of another preferred embodiment of the present invention.
Figure 8C is a diagrammatic representation of the relationship of the heat producing element and the capillary tubes holding the biological samples in the embodiment illustrated in Figures 8A-B.
Figure 9A shows the results of four different temperature/time profiles (A-D) and their resultant amplification products after thirty cycles (A-D).
Figure 9B shows a cycle of another preferred temperature/time profile used by the present invention. Figures 9C-G show exemplary cycle of other preferred temperature/time profiles used by the present invention.
Figure 10 provides a block diagram of a temperature slope control circuit in accordance with the present invention.
Figure 11 is a schematic view of a preferred rapid temperature cycler with fluorescence detection in accordance with the present invention.
Figure 11A is a temperature v. time chart of showing one preferred operation of the apparatus of Figure 11.
Figure 12 is a chart showing 2D plots of temperature vs. time, fluorescence vs. time, and fluorescence vs. temperature also shown as a 3D plot.
Figure 13 is a fluorescence vs. temperature projection for a 536 base pair fragment of the human β-globin gene.
Figure 14 is a cycle number vs. fluorescence plot obtained in accordance with an aspect of the present invention.
Figure 15 is a cycle number vs. fluorescence ratio plot obtained in accordance with an aspect of the present invention.
Figure 16 is a fluorescence ratio vs. temperature plot obtained in accordance with the an aspect of the present invention.
Figure 16A is a fluorescence ratio vs. cycle number plot obtained in accordance with an aspect of the present invention.
Figure 17 is a fluorescence ratio vs. temperature plot obtained in accordance with an aspect of the present invention.
Figure 18 is a fluorescence vs. cycle number plot for a number of different initial template copies obtained in accordance with an aspect of the present invention.
Figures 19A-D represent two preferred arrangements for providing sequence specific detection of PCR products in accordance with the present invention.
Figure 20 is a fluorescence ratio (fluorescein/rhodamine) vs. cycle number plot of DNA amplification monitored with a dual-labeled hydrolysis probe obtained in accordance with an aspect of the present invention.
Figures 21A-D provide a comparison of three fluorescence monitoring techniques for PCR, including dsDNA dye SYBR® Green I (A), a dual-labeled fluorescein/rhodamine hydrolysis probe (B), and a fluorescein-labeled hybridization probe with a Cy5-labeled primer (C); Figure 21D shows the coefficient of variation for the three monitoring techniques represented in Figures 21A-C.
Figure 22 provides plots showing the application of interpolation methods for quantification of PCR fluorescence curves.
Figure 23A provides a plot showing a linear change in fluorescence ratio with temperature and a parallel increase in fluorescence as more probe is hydrolyzed.
Figure 23B provides a plot showing that the fluorescence ratio from hybridization probes varies radically with temperature.
Figure 24 provides a plot showing the temperature dependence of product strand status during PCR in accordance with the present invention.
Figure 24A is a temperature vs. florescence plot of PCR with SYBR® Green I.
Figure 25 provides a plot showing the native and competitive components of a product melting curve in accordance with the present invention.
Figure 26 provides a plot of rate of product reannealing for different concentrations of PCR product in accordance with the present invention.
Figure 27 provides a plot showing the initial determination of a rate constant.
Figure 28 is a graph representing an equilibrium PCR paradigm and a kinetic PCR paradigm.
Figure 29 shows that monitoring over a 10-fold dynamic range in the log-linear portion of a routine amplification has been accomplished.
Figure 30 shows an embodiment of the present invention configured for continuous monitoring of a single sample.
Figure 31 shows the optical layout of another embodiment in accordance with the present invention to provide continuous monitoring of a PCR sample.
Figure 32 sets forth charts showing the effectiveness of light piping by viewing the tip rather than the side of capillary sample tubes.
Figure 33 is a chart showing that the observed polarization when using capillary sample tubes depends on the precise positioning of the tube with respect to the excitation beam.
Figure 34 shows the results obtained using one polarization probe in accordance with the present invention.
Figure 35 shows fluorescence PCR results from a probe with five intervening bases between fluorescein and rhodamine labels.
Figure 36 shows the effect of probe concentration on sensitivity.
Figure 37 plots cycle number vs. fluorescence ratio in PCR monitored by resonance energy transfer and hydrolysis probes in accordance with the present invention.
Figure 38 is a plot of emission wavelength vs. fluorescence for resonance energy transfer of a dual labeled fluorescein/Cy5 probe in accordance with the present invention.
Figure 39 is a plot showing cycle number vs. fluorescence ratio above background in PCR monitored by resonance energy transfer with hybridization probes.
Figure 40 is a plot of temperature vs. fluorescence ratio in PCR monitored by adjacent hybridization probes.
Figure 41 is a plot of temperature vs. fluorescence ratio in PCR monitored by hydrolysis probe.
Figures 42A is a plot of time vs. fluorescence showing the inverse relationship between temperature and fluorescence.
Figures 42B is a plot of time vs. temperature showing the inverse relationship between temperature and fluorescence.
Figure 43A is a plot of temperature vs. fluorescence for the melting curves of three different purified PCR products.

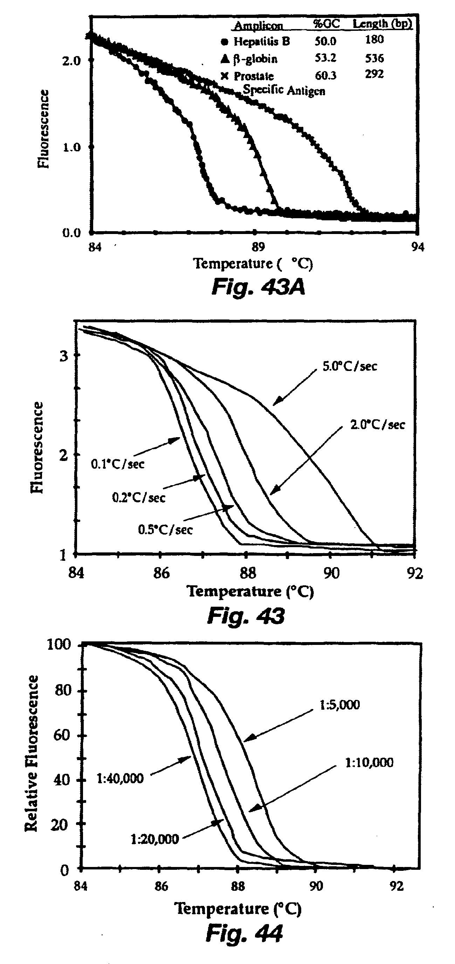

Figure 43 is a plot of temperature vs. fluorescence showing the apparent Tₘ of PCR products is dependent on the heating rate.
Figure 44 is a plot of temperature vs. relative fluorescence showing Tₘ of PCR products is dependent on the dsDNA dye concentration.

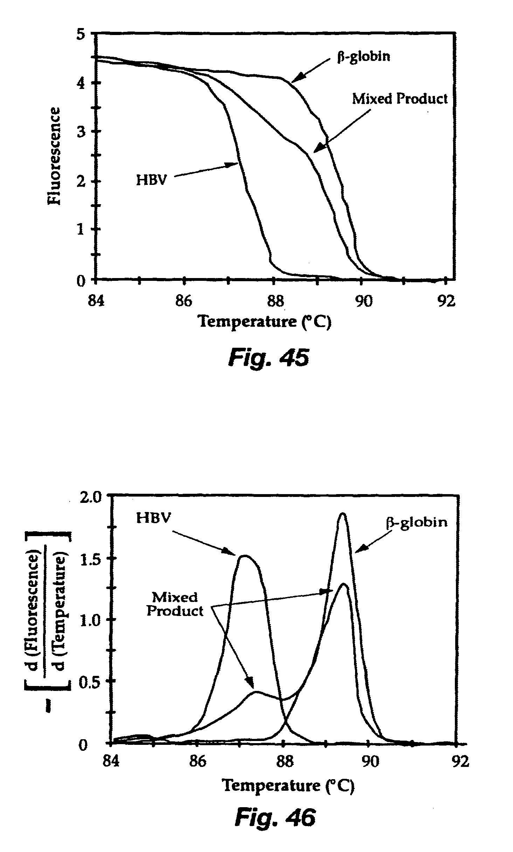

Figure 45 is a plot of temperature vs. fluorescence showing the melting curve obtained when two purified PCR products differing in Tₘ by about 2°C are mixed.
Figure 46 is a plot showing how the relative amount of each PCR product can be quantified.

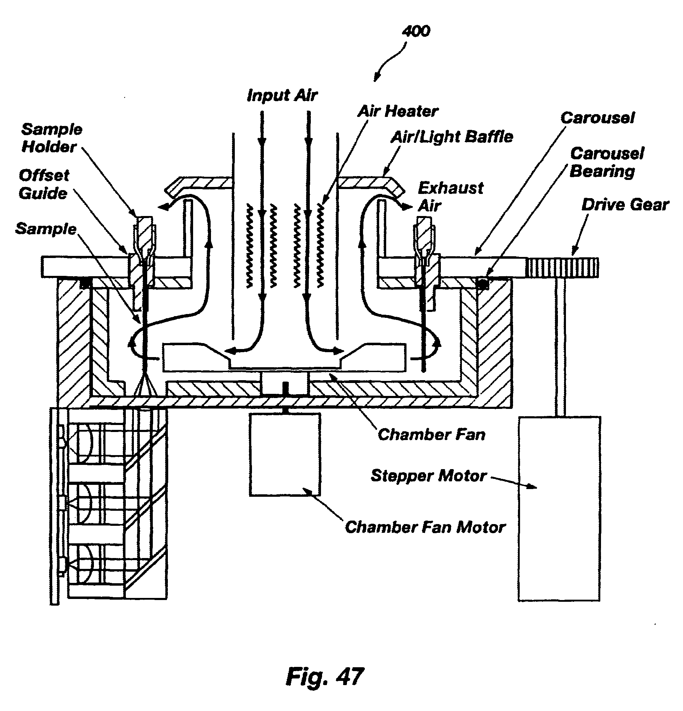

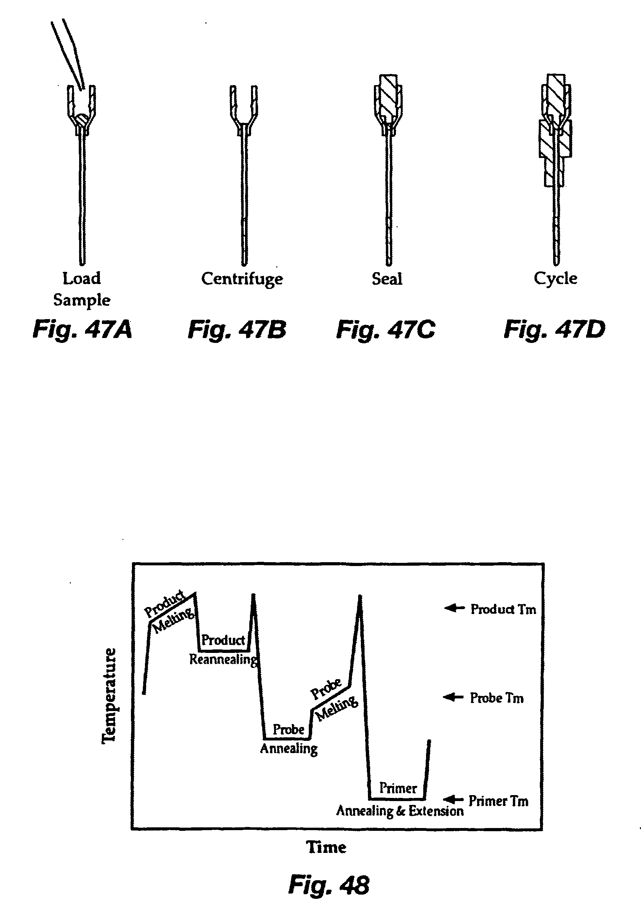

Figure 47 is a schematic representation of another embodiment of the present invention which is a rapid temperature cycler with fluorescence detection at the tip of capillary sample tubes.
Figures 47A-D show composite plastic/glass containers into which samples are loaded.
Figure 48 illustrates useful temperature vs. time segments for fluorescence hybridization monitoring.

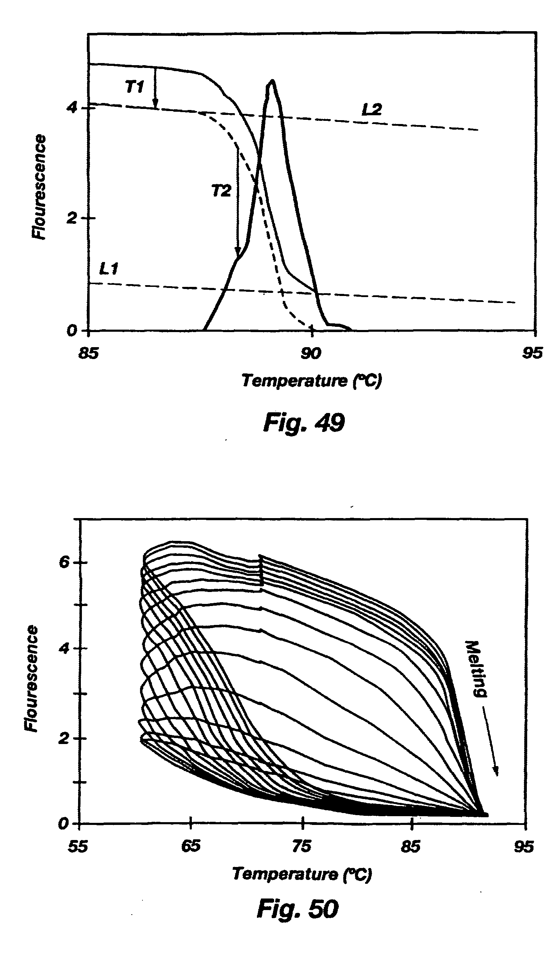

Figure 49 shows melting curve information displayed as melting peaks.
Figure 50 plots fluorescence from dsDNA specific dyes as a function of temperature during PCR to allow monitoring of strand status during individual cycles of the reaction.

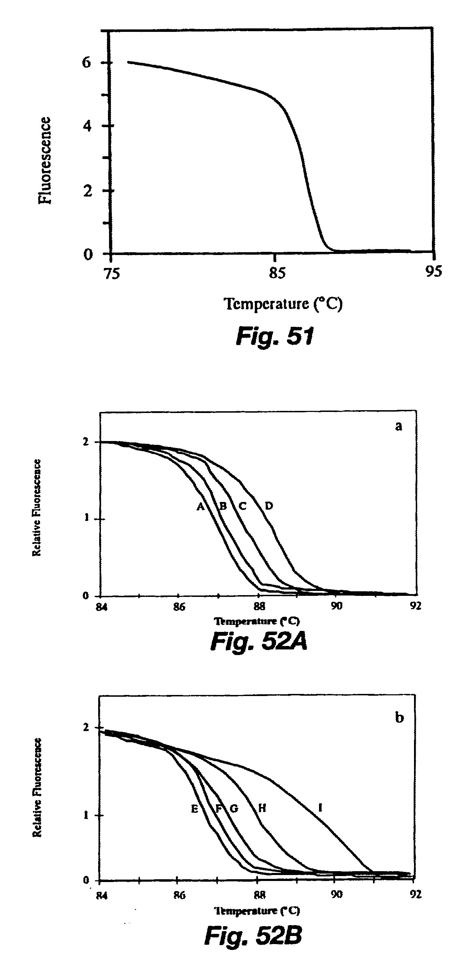

Figure 51 plots a melting curve acquisition cycle.
Figure 52A plots the absolute position of PCR product melting curves.
Figure 52B plots how rapid temperature transitions through the denaturation temperature shifted melting curves to higher apparent Tₘ.

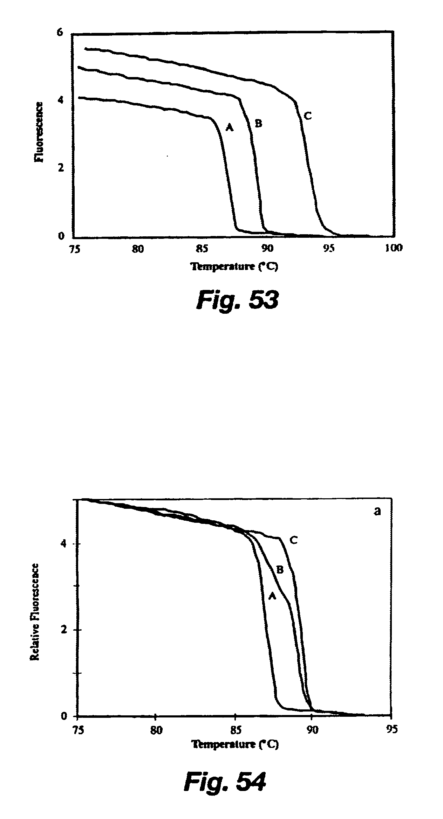

Figure 53 plots melting curves for three different purified PCR products.
Figure 54 plots the overlapping melting curves resulting when purified PCR products were mixed.

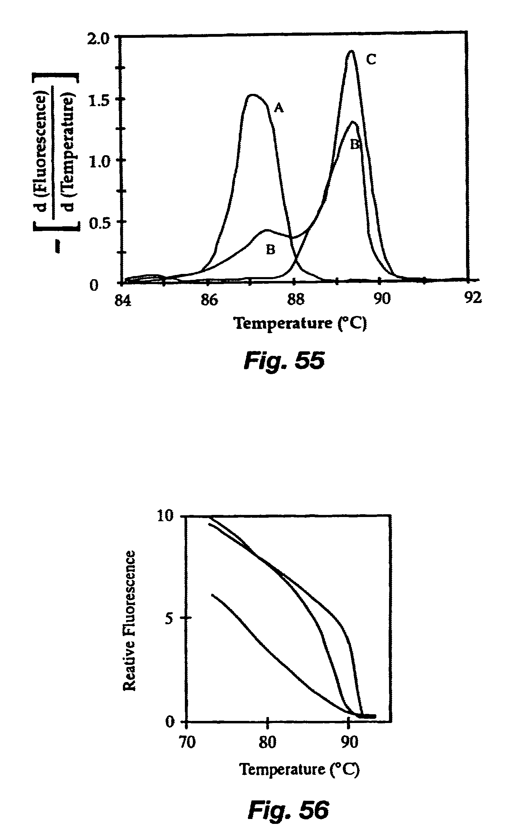

Figure 55 plots the results after conversion into melting peaks showing that it is possible to resolve mixtures of reaction products that differed in T, by less than 2°C into separate peaks.
Figure 56 plots a melting curve analysis used to differentiate intended product from nonspecific products such as primer dimers.

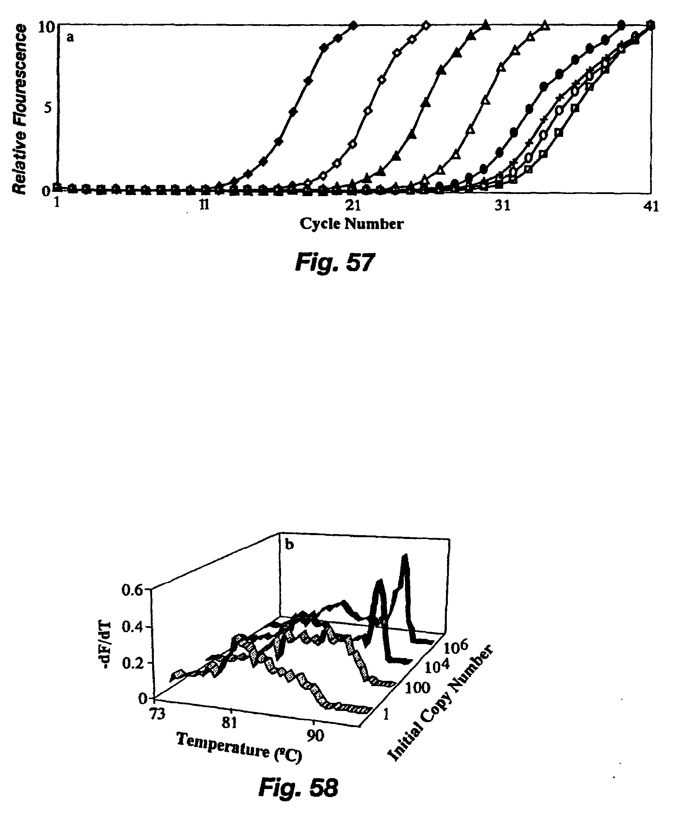

Figure 57 plots relative fluorescence acquired once each cycle after polymerase extension of the product for a series of reactions varying in initial template concentration.

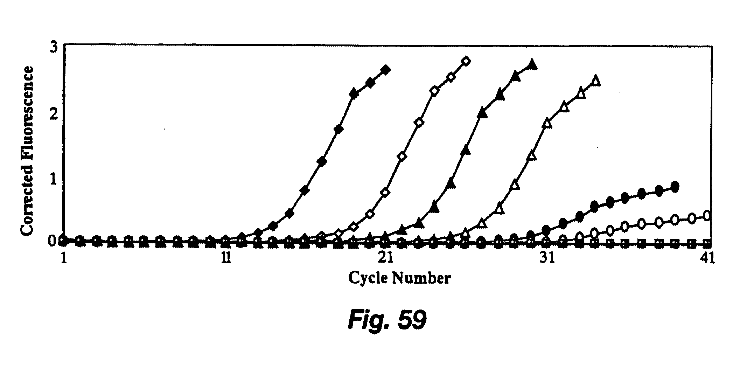

Figure 58 plots melting peaks acquired for a number of samples.
Figure 59 plots the data points of Figure 57 multiplied by the appropriate ratio to give the corrected plot.

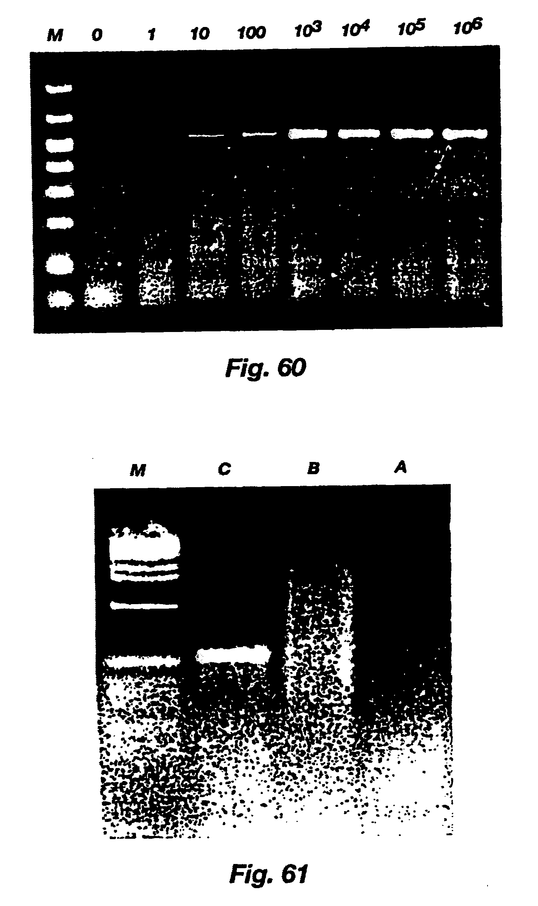

Figure 60 shows the results of Figure 58 correlate well with electrophoresis results.
Figure 61 shows melting curves represented in Figure 56 can be resolved into product peaks for estimates of the relative amount of different products present.

### DETAILED DESCRIPTION OF THE PREFERRED EMBODIMENTS

Reference will now be made to the drawings wherein like structures will be provided with like reference designations.

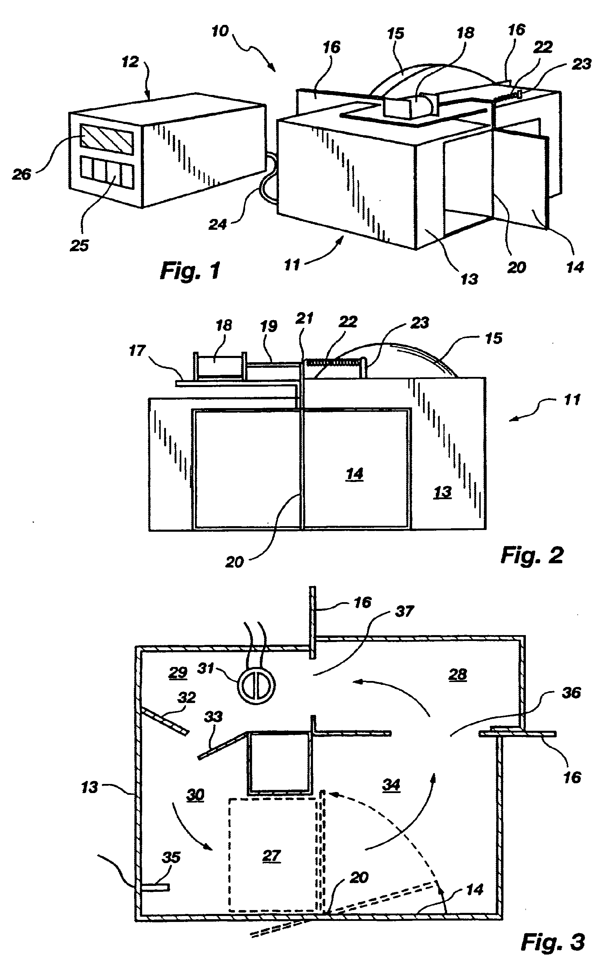

As shown in Figure 1, the thermal cycling device 10 includes a closed loop fluid (most preferably air) chamber, generally designated at 11, which is adapted to accept samples to be cycled through vent door 14. The closed loop fluid chamber 11 includes a plurality of compartments each of which will be described shortly. The device 10 also includes a controller 12 which can be programmed by means of input keys 25 and display 26 to cause the chamber 11 to be cycled through a series of temperatures over a predetermined period of time. The thermal cycling of chamber 11 can be used to carry out numerous procedures and is particularly suited for amplification of primer specific DNA from samples containing reaction mixture as will be explained below.

The closed loop fluid chamber 11 is enclosed in a generally box shaped configuration by housing 13. Blower mounting boards 16, if desired, can be located so as to section off a smaller rectangular section of the chamber 11 and function to support and secure a generally cylindrically shaped lower housing 15 thereto. Alternatively, the fan of the blower 28 may be housed integrally within chamber housing 13.

The interior of blower housing 15 contains the blades and shaft of the blower. The blower motor (not shown) is located externally of blower housing 15, and therefore exteriorly of the enclosed chamber 11. In this configuration, the blades and shaft are the only parts of the blower which become exposed to the circulating hot fluid within chamber 11. It would be disadvantageous to mount the motor within the chamber which would subject the motor to temperature variations and also would add the thermal mass of the motor to that which is subject to heating and cooling. The reduction of thermal mass exposed to the fluid in chamber 11 is important to the overall performance of the device 10 in its function of subjecting samples placed therein to predetermined temperature versus time profiles as will be more fully explained below.

The blower 28 is a well known type blower usually identified as an "in line" type blower which preferably employs a propeller type fan due to its generally low thermal mass, or if desired, a squirrel cage type fan, the fan preferably having a 75 cubic feet per minute minimum capacity.

The solenoid platform 17 has secured thereto a solenoid 18. The solenoid armature 19 is attached to upper end 21 of rod 20 which is rigidly attached to vent door 14 and rotatably attached to housing 13 at points above and below the vent door 14. The rod 20 therefore allows vent door 14 to freely rotate relative to the housing 13 about the rod's longitudinal axis.

A spring 22 is attached at one of its ends to the housing 13 by support post 23. The opposite end of spring 22 is attached to the top end 21 of rod 20 directly adjacent the attachment of solenoid armature 19. The spring 22 is drawn between these two attachment points so as to be in tension. The spring 22 therefore tends to draw top end 21 toward the support post 23, which in turn tends to rotate vent door 14 to its closed position. When solenoid 18 is actuated, armature 19 tends to pull top end 21 of the rod 20 in the direction of the solenoid 18, which is opposite the direction of pull of spring 22, and which tends to open the vent door 14.

Controller, generally designated at 12, is electrically attached to the chamber 11 by means of a transmission cable 24. The cable 24 also supplies power to the blower motor (not shown), and to the heat coil 31. Further, the controller 12 also is connected to thermocouple sensor 35 for receiving signals corresponding to temperature data, and to solenoid 18 for triggering the solenoid armature.

Controller 12 can be any well known type of temperature controller unit which is programmable to control the heat coil 31, vent door 14, and blower so as to achieve predetermined temperatures as a function of time within the chamber 11, and which is also capable of being programmed to actuate a relay output for driving a solenoid at predetermined time periods and chamber temperature levels. A preferred temperature controller 12 for use in the embodiment of Figures 1-3 is a Partlow MIC-6000 proportional temperature controller, available through Omega Engineering Inc, of Stanford, Connecticut, as the Model No. CN8600 process controller.

As shown in Figures 2 and 3, the interior of chamber 11 is sectioned off into four main compartments. The blower compartment 28 is formed of the blower housing 15 and the blower mounting plates 16. The entirety of blower compartment 28 is filled with the fan and shaft portions of a blower as has been described above. The blower can be any of a number of well-known designs, as has been described above, and has therefore been omitted from Figure 3 for purposes of clarity. It is sufficient for the present invention to understand that the fan located in blower compartment 28 draws fluid into the blower compartment 28 through inlet opening 36 and pushes the fluid out of exit opening 37.

It is preferred that the fluid be driven by the blower at a rate of at least 75 cubic feet per minute. It is important however, in regard to the present invention, to realize that the fluid located in chamber 11 only contacts the fan and a portion of the drive shaft of the blower, the blower motor itself being located outside of the blower housing 15 so as to avoid any contact thereof with fluid in the chamber 11. This is important since it is critical to the speed of operation of the invention to minimize the material which contacts the fluid inside the chamber 11 so as to minimize the thermal mass of material which must be heated and/or cooled thereby during the cycling process. By minimizing the thermal mass which must be heated or cooled by the fluid, the response time necessary to bring the contents of chamber 11 to a uniform temperature is greatly diminished.

Fluid exiting blower compartment 28 through outlet opening 37 enters heating compartment 29. Fluid passing into heating compartment 29 must pass by heating coils 31. If heating coils 31 gets hotter than the fluid passing into heating compartment 29, the fluid will become heated thereby as it is forced through the compartment. The heating coil is preferably a 1,000 watt (125 VAC) nichrome wire coil wound around a microsupport. However, any heating unit suitable for heating the type of fluid present in the chamber may be used. The particular heating coil of embodiment of Figures 1-3 is manufactured by Johnstone Supply, of Portland, Oregon.

The heating coil is activated by an output relay included in the controller 12. The preferred relay is a 25 A, 125 VAC solid state relay manufactured by Omega Engineering Inc. of Stanford, Connecticut as Model No. Omega SSR 240 D25.

Fluid passing through heating compartment 29 becomes incident on baffles 32 and 33 before passing into the reaction compartment 30. Baffles 32 and 33 tend to break up any laminar fluid flow and generate turbulence therein to effectively mix the fluid so that it arrives in reaction compartment 30 at an homogenous temperature.

Thermocouple sensor 35 provides an electrical input signal to controller 12 which corresponds to the fluid temperature in the reaction compartment 30. Temperature monitoring during operation of the thermal cycling device 10 is preferably achieved by a 30-gauge iron-constantan "J-type" thermocouple. The controller uses this information to regulate the heat coil 31 according to the predetermined temperature versus time profiles programmed therein and to actuate solenoid 18, as will be explained momentarily.

The fluid passing from the reaction compartment 30 to the return air compartment 34 must pass through sample compartment 27 (as shown in dashed lines). Sample compartment 27 will also be explained momentarily.

The fluid in return compartment 34 has been slightly cooled due to the heat transfer therefrom into samples located in sample compartment 27. The fluid in return compartment 34 is drawn through inlet opening 36 into blower compartment 28 where it is again forced, by action of the fan, out through outlet opening 37 into the heating compartment 39. Thus, the fluid chamber 11, when operating with vent door 14 closed, is a closed loop fluid chamber which continuously recirculates the fluid along a closed loop path through each compartment thereof in order to bring the contents therein to a uniform temperature. Continuous circulation of the air in the air chamber 11 allows the samples in sample compartment 27 to be brought to a predetermined temperature as quickly as possible, and then to be held at that temperature, if desired.

When the device 10 must be used to not only heat material located in the reaction compartment 27, but also to subsequently cool these materials as quickly as possible to a temperature at or above the ambient fluid (air) temperature, the controller 12 can be programmed to actuate solenoid 18 to cause vent door 14 to open and allow large quantities of ambient fluid to immediately flood the compartment 11 while heated fluid therein simultaneously escapes.

Deactivation of the heating coil 31 while continuing activation of the blower with vent door 14 open, will draw ambient fluid into return compartment 34 and from there into the blower compartment 28. The blower will then push this ambient fluid through heating compartment 29 where it will pass directly into reaction compartment 30 without being heated by coil 31. The ambient fluid then passes through the sample compartment 27 and escapes out of chamber 11 through the vent door 14. Due to the minimum thermal mass of material located in chamber 11, and the action of the blower fan, vast quantities of ambient fluid will be forced past the sample compartment 27, and from there out of the chamber 11. Thus, rapidly cooling of samples or material located in the reaction compartment 27 is obtained.

The sample compartment 27 is sized so as to allow a plurality of samples, such as hollow elongate glass tubes containing a sample therein, to be easily located in a space apart orientation so that fluid may pass evenly around each sample. If desired, sample compartment 27 may be sized and configured so as to allow insertion of a rack, basket, or the like which has been configured so as to accept a plurality of samples in uniform spaced apart configuration so as to simplify loading the samples into the sample chamber 27.

Access to sample compartment 27 is accomplished by rotation of the vent door 14 to its open position. Once the vent door 14 is rotated to approximately 90 degrees from it's closed position, the sample compartment 27 is easily accessible therethrough. Also, as can be seen, rotation of vent door 14 approximately 90 degrees from its closed position causes return fluid compartment 34 to be substantially closed off from reaction compartment 30. Thus, when the device 10 of the present invention is in a "cooling" mode, ambient fluid enters directly into return fluid compartment 34 and is forced through the blower compartment 28, heating compartment 29, reaction compartment 30, and sample compartment 27 substantially along the same path as the closed loop fluid flow path described above. The fluid is then forced out of the air chamber 11 and prevented from passing back into air return compartment 34 by the positioning of the vent door 14 between the sample compartment 27 and the return fluid compartment 34.

Thus, the vent door 14 not only allows ambient fluid to enter the chamber 11, it can also prevent the fluid from recirculating in a loop fashion through the chamber 11. Instead, fluid is forced to pass through the sample compartment 27 and then out of the chamber 11 to aid in the rapid cooling of the sample contents and chamber 11.

When the device 10 of the present invention is used for cyclic DNA amplification, repetitive cycling through a temperature versus time profile is required. Samples containing a reaction mixture for the polymerase chain reaction generally must be cycled approximately 30 times through a temperature versus time profile which corresponds to the denaturation, annealing and elongation phases of the amplification process.

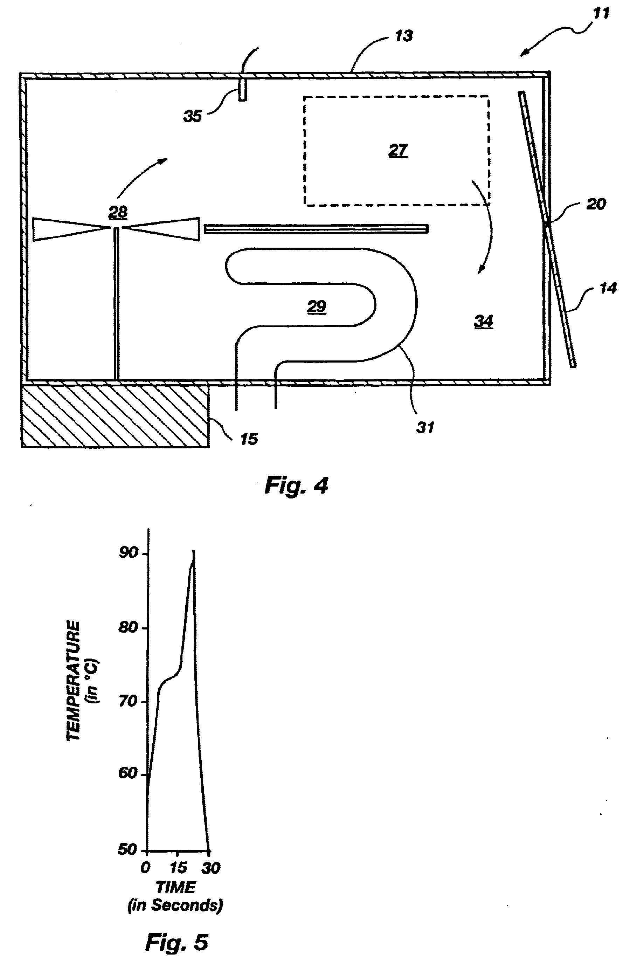

The device 10 of the present invention, due to its lower overall thermal mass, is capable of cycling samples through significantly shortened temperature versus time profiles compared to the prior art. The DNA amplification application of the embodiment of Figures 1-3 can pass through a temperature versus time profile cycle in 30-60 seconds (see Figure 5). This same cycle using prior art devices would take approximately 5-10 times longer. These low cycle times have proven also to increase yield and specificity of the polymerase chain reaction over prior art cycling.

### EXAMPLE 1

The polymerase chain reaction was run in a 10 µℓ volume with 50 ng template DNA, 0.5 mM of each deoxynucleotide 500 nM of each oligonucleotide primer in a reaction buffer consisting of 50 mM Tris-HCl (pH 8.5 at 25°C), 3.0 mM magnesium chloride, 20 mM HCl, and 500 µg/ml bovine serum albumin (5). Thermos aquatics polymerase (0/4 units of Taq polymerase - Stratagene™) was added, the samples placed in 8 cm long, thin-walled capillary tubes (manufactured by Kimble, Kimax 46485-1), and the ends fused with a laboratory gas burner so that an air bubble was present on both ends of each tube.

The capillary tubes were then placed vertically in a holder constructed of 1 mm thick "prepunched perfboard" (manufactured by Radio Shack). The mixture was cycled 30 times through denaturation (90-92°C), annealing (50-55°C), and elongation (72-75°C) for the times indicated in the temperature versus time profile of Figure 5. Temperature monitoring of the capillary tubes was done with a miniature thermocouple (IT-23, Sensortek, Clifton, NJ) placed in 10 µℓ of deionized water and connected to a thermocouple monitor (BAT-12, Sensortek). Amplification products were fractionated by electrophoresis on a 1.5% agarose gel. Good results were obtained.

Due to the fact that the device 10 of the present invention cycles air as the heat transfer medium instead of water, it has the advantage that heat transfer occurs through a low heat capacity medium (air) which can be warmed very rapidly.

The response time for sample cooling is very fast due to the use of thin walled glass capillary tubes for holding samples, instead of plastic microfuge tubes as has been done in the past with prior art processes, and by minimizing the thermal mass of material inside the chamber 11 (see Figure 5). Such response times can allow for optimization of the denaturation and annealing, and elongation steps in the polymerase chain reaction, in terms of time and temperature.

Further, shortened "ramp" times are obtained, i.e., the time required to bring the temperature of the sample from one temperature level to the next temperature level corresponding to phases in the amplification process is shortened. This decreases the time required for a complete amplification, as well as allowing specific study of annealing, denaturation and enzyme kinetics within a polymerase chain reaction protocol.

The baffles 32 and 33 (as shown in Figure 3) may be used if desired to achieve adequate temperature homogeneity within the sample compartment 27. As shown in this embodiment, baffles 32 and 33 decrease the temperature variation in the reaction compartment 30 from about 10°C, to about 2°C. If desired, further (or more complicated) baffles may be used to further decrease the temperature variation in reaction compartment 30. Alternately, as shown in Figure 4 the fan may be positioned downstream from the heating coil 31, but before the sample compartment 27 to achieve more uniform mixing.

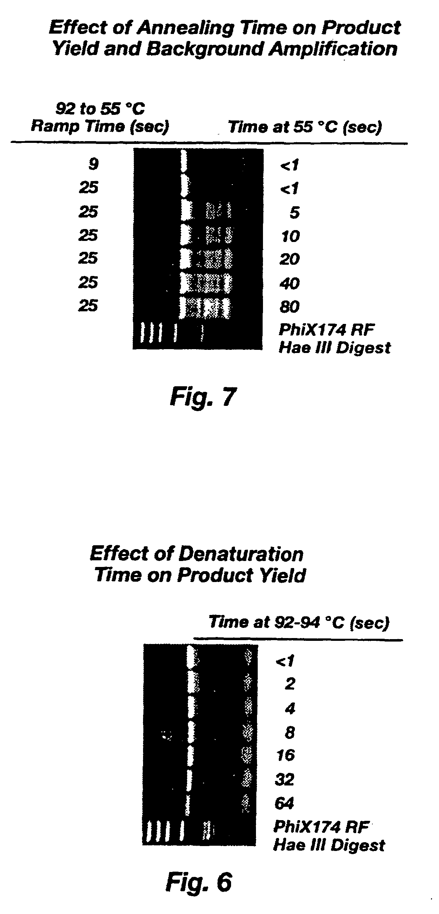

Amplification products obtained through the use of apparatus 10 are qualitatively and quantitatively similar to those obtained through the manual water bath cycling method. However, advantages in specificity and yield are possible with rapid thermal control of the reaction mixture. Figure 6 shows the effect of varying the denaturation time of the temperature versus time profile of Figure 5 as used with the thermal cycling apparatus 10 of the present invention on DNA amplification yields. The brighter vertical lines each correspond to a particular time at a denaturation temperature. As can be seen, the yield is greatest at the shortest possible denaturation time. Such a rapid response is not possible with prior art systems.

Figure 7 shows the effect of the temperature versus time profile of Figure 5 as used with the thermal cycling apparatus 10 on specificity (i.e., one specific product yield as opposed to a plurality of similar or "shadow" products) . As can be seen, the shorter the ramp and annealing time, the greater the product specificity. The rapid temperature response of the apparatus 10 allows improved specificity and yield which is not possible with prior art systems.

As has been shown, by decreasing the thermal capacity (thermal mass) of the apparatus 10, the present invention can markedly decrease the total time required for the polymerase chain reaction. In addition, the use of small samples reduces the amounts of expensive reagents which must be used by up to about 90% thus further reducing the cost of carrying out procedures using the present invention. For example, capillary tubes 108 having inner diameters in the range from about 0.25mm to about 1.0mm can desirably be used. In some applications, capillary tubes 108 having inner diameters in the range from about 0.02mm to about 0.1mm can also be desirably used.

The apparatus 10 of the present invention is useful for amplifying DNA from any source. Although particular configurations and arrangements of the present invention have been discussed in connection with the specific embodiments of the thermal cycling device 10 as constructed in accordance with the teachings of the present invention, other arrangements and configurations may be utilized. For example, various fluids other than air, of generally low thermal mass, may alternatively be used in the device 10.

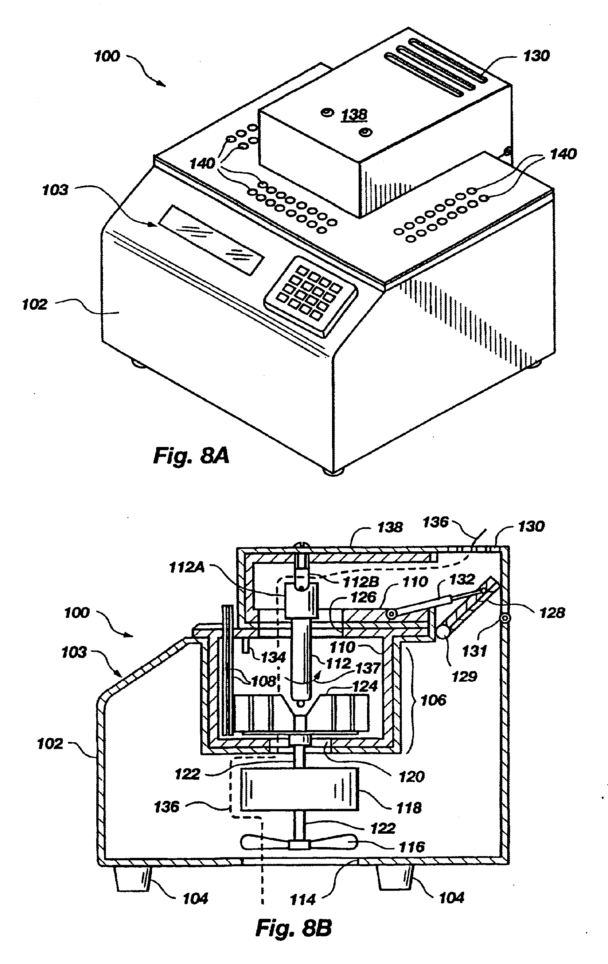

Another embodiment of the present invention is represented in Figures 8A-C. Figure 8A is a perspective view and Figure 8B is an elevational cross sectioned view of the additional embodiment. It will be understood that many of the earlier explained components and teachings also have application in the embodiment illustrated in Figures 8A-C. Thus, only the pertinent additional information concerning this embodiment will be provided below. Importantly, in the embodiment of Figures 8A-C, the heat producing element is adjacent to the biological sample containers allowing faster heating and cooling of biological samples as explained below.

As will be appreciated shortly, the apparatus of Figures 8A-C provides even greater improvement over the prior art in the speed at which thermal cycling can be carried out, e.g., 15 or 30 cycles of DNA amplification in 30, 15, 10 to 5, or even fewer, minutes. Furthermore, the apparatus 100 provides better thermal homogenization throughout the samples than previously possible.

Shown in Figure 8A is the general configuration of the housing 102 of the embodiment. The housing 102 rests on feet 104 (best seen in Figure 8B) and functions to hold the other described structures in place and to isolate those structures which become hot from the surrounding environment. Included in the embodiment 100 of Figure 8A are input keys 25 and a display 26 as in the previously described apparatus 10. The previously described control structures can readily be modified or used as a pattern for a control means for use in the embodiment of Figures 8A-C.

As shown best in the cross sectional view of Figure 8B, a sample chamber is designated by bracket 106. A lid 138 connected to the housing 102 by a hinge 131 can be opened to allow access to the sample chamber 106. The sample chamber 106 is preferably cylindrical in shape but can be of any shape or size required by the particular application

The sample chamber 106 is lined with a black colored foan. material 110 whose surface has light absorbing characteristics with the bulk of the thickness of the foam having insulating characteristics. The black foam material can be one which is readily available in the art and one fabricated from a plastic material. The foam 110 is preferably a material which is readily cooled by the air passing there over, i.e., the material has low thermal conductivity and a porous surface .

The dark or black porous surface of the material converts shorter wavelength radiation striking the surface into longer wavelength radiation, i.e., heat, which is radiated into the sample chamber.

The foam 110 functions to thermally isolate the sample chamber from the surrounding air space in the housing and also to convert the light emitted by lamp 112 into thermal energy. The foam 110 can be replaced with other structures. For example, a material having a black, dark, or other nonreflective surface, such as a thin sheet of polycarbonate having one surface painted black, can be backed by an insulative material, such as a fiberglass or foam material. The black or dark surface, which can be painted on a number of different substrates, converts shorter wavelength radiation striking it into thermal radiation while the insulative material thermally isolates the sample chamber from the surrounding environment. Thus, using the teachings provided herein, those skilled in the art can utilize many different materials and structures as a lining for the sample chamber.

The lamp 112 is preferably a 500 watt halogen lamp. If appropriate control devices are used, higher power lamps or a plurality of lamps, such as four 500 watt halogen lamps, can be used. A lamp socket 112A is attached to the housing 102 by a support 112B. The lamp 112 is able to very rapidly and uniformly heat the sample chamber 106 to the desired temperature. Other sources of heat, i.e. infrared radiation, such as the earlier described nichrome wire element, can also be used within the scope of the present invention.

Represented in Figure 8B are two thin-walled capillary tubes 108 such as those described earlier. While two thin-walled capillary tubes 108 are shown, the sample chamber 106 can hold many such tubes. The thin-walled capillary tubes 108 have several important advantages over previously used devices as described earlier and, together with the sample chamber 106, function as the one presently preferred example of a means for holding a biological sample.

It will be appreciated that many other structures performing equivalent or similar functions can also be used. The thin-walled capillary tubes 108 are preferably left partially extending out of the sample chamber through apertures 140 for ease of access but may be completely contained within the sample chamber 106 as may numerous other fluid holding structures which are suited to particular applications. The preferred thin-walled capillary tubes 108 have a capacity of about 10 µℓ. As will be understood, the volume of the sample should be keep small, and the surface area of the sample holding structure relatively large, and together they should present a relatively small thermal mass. It is also preferred that the sample holding structure contain a volume anywhere from about 0.1 µℓ to about 10,000 µℓ but those skilled in the art will appreciate that other volumes of samples can also be used within the scope of the present invention if the different thermal mass of the structure is considered.

The lamp 112 and the insulative foam 110 together provide rapid and uniform heating of the sample contained in the thin-walled capillary tubes 108 and the air contained within the sample chamber 106. A thermocouple 134 is included within the sample chamber 106 to sense the temperature within the chamber and is used to maintain the desired temperature within the sample chamber as earlier described.

The thermocouple 134 is preferably one available in the art whose thermal response substantially matches the thermal response of the biological sample and the container holding the same. Such thermocouples can be commercially obtained from sources such as Idaho Labs which manufactures a line of thermocouples referred to as metal sheathed, J-type thermocouples. The matching of the thermal response of the thermocouple to that of the biological sample and container can be preferably carried out by inserting a micro thermocouple, such as the model IT-23 thermocouple available from PhysiTemp as known in the art, into a typical biological sample being held by the chosen container and subjecting the sample and the thermocouple under test to the same temperature changes. The thermocouple under test, or some external criteria, can be changed until the thermal response of the thermocouple suitably matches the thermal response of the sample and its container.

The arrangement represented in Figure 8B provides more uniform heating and cooling of the sample than previously available devices. In previously available devices, transfer of heat throughout the sample is carried out by convection through the sample. Convection induced movement of the sample within whatever structure is used to hold the sample is caused by temperature gradients or differences in the generally small biological samples (e.g., 10-100 µℓ).

The effect of temperature gradients within the sample become more pronounced and more difficult to control as the cycle time for a sample decreases. The existence of uneven temperatures within a sample, and particularly the reliance on "mixing by convection" within the sample by the prior art devices, generally increases the cycle time for a sample and likely has deleterious effects on the biological sample. The apparatus 100 is capable of providing heating and cooling such that thermal differences within a 10 µℓ sample are maintained at not greater than ±1°C at all times during a 30 second cycle.

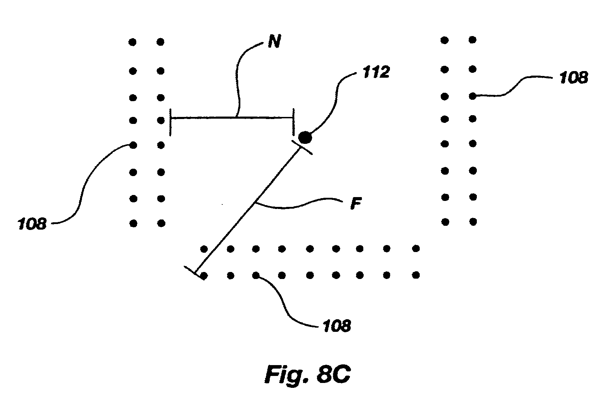

In order to promote uniform heating and cooling, it is preferred that the thin-walled capillary tubes 108 be at least somewhat uniformly spaced from the heat source, for example, lamp 112 in apparatus 100. Figure 8C provides a diagrammatic top view of the lamp 112 and the plurality of thin-walled capillary tubes 108 as arranged in the apparatus 100 represented in Figures 8A-B.

In the arrangement represented in Figure 8C, the thin-walled capillary tubes 108 which are farthest from the lamp 112 (as indicated by line F) are preferably no more than substantially 40%, and more preferably no more than substantially 25%, farther from the lamp 112 than the distance between the lamp 112 and those thin-walled capillary tubes 108 which are closest to the lamp 112 (as indicated by line N) . For example, the distance indicated by line N can be about 7.3 cm while the distance indicated by line F can be about 8.5 cm.

It will be appreciated that the arrangement of the thin-walled capillary tubes 108 can be other than that represented in the figures, for example, circular or semi-circular. Moreover, it will appreciated that the point from which to measure the distance between the heat producing element and the sample containers will vary as the type and size of the heat producing element varies. For example, the heat producing element may comprise a plurality of lamps or electric resistive elements which vary in shape and size. In some embodiments, it may also become important to consider the distance from the sample chamber wall the sample containers are positioned. In the illustrated embodiment, the apertures 140 (see Figure 8A) function as a means for holding the sample containers but other structures performing equivalent functions can also be used in accordance with the present invention.

The apparatus 100 also cools the samples contained in the capillary tubes 108 very rapidly and uniformly. In order to cool the sample chamber 106, air from outside the housing 102 is draw into the interior of the housing through a lower housing portal 114 by a fan 116 which is connected to a motor shaft 122 driven by a motor 118. Since rapid cooling of the sample chamber is desired, it is preferred that the combination of the motor 118 and the fan 116 be able to move sufficient volumes of air into the sample chamber 106 and then disperse that air inside the sample chamber 106, as will be explained shortly. Arrangements other than the motor 118 and fan 116 illustrated in Figure 8B can also be used within the scope of the present invention.

The use of air as the thermal transfer medium, in contrast to other gases and liquids, has the advantages of being inexpensive, readily available, easily mixed, and never making a mess. In the case of the described embodiments, the high surface area-to-volume ratio of the sample containing capillary tubes provides for rapid thermal transfer using air as the thermal transfer medium.

During cooling portions of the thermal cycle, the action of the fan 116 draws ambient temperature air into the housing 102. A vent door 128, articulating on hinge 129, is provided. The vent door 128 is automatically opened by way of a solenoid 132 so that the interior of the housing 102 is sealed off from the upper housing portal 130. In some embodiments, the solenoid 132 is preferably replaced by a stepper motor as is known in the art. The use of a stepper motor allows the vent door 128 to be accurately and incrementally opened and closed in accordance with the needs for heating and cooling the samples. Those skilled in the art will be able to derive an appropriate control mechanism for use with a stepper motor, for example an SC-149 stepper motor controller (available from Alpha Products) as known in the art, using the information set forth herein.

Due to the arrangement of the lower sample chamber portal 120 and the larger cross sectional area and position of the upper sample chamber portal 126, room temperature air is moved into the sample chamber 106 and is dispersed and mixed within the sample chamber 106 by a paddle 124 which is connected to the motor shaft 122. The paddle 124 should rotate at a relatively high rate, for example, fast enough to create air velocities of around preferably about 250, more preferably 500, and most preferably 1000 meters per minute within the sample chamber 106. With the paddle 124, which can be a single or a multivane paddle, rotating at a high speed, air is moved, or drawn, into the sample chamber 106 and vented out of the sample chamber 106 following the path indicated by the dashed line 136. The rotation of the paddle 124 also promotes mixing of the air entering the sample chamber 106 and ensures the most efficient transfer of thermal energy from the surfaces of the thin-walled capillary tubes 108 to the air passing through the sample chamber 106. It will be appreciated that structures other than those illustrated herein can perform equivalent functions.

As the solenoid 132 is actuated to open the vent door 128, all of the room temperature air moved into the sample chamber 106 is exhausted through a sample chamber upper portal 126 and then through the upper housing portal 130 carrying the heat from the sample chamber 106 to the surrounding atmosphere. The rapid mixing of the air that passes through, and is disbursed in, the sample chamber 106 results in rapid and uniform cooling of the samples.

### Example 2

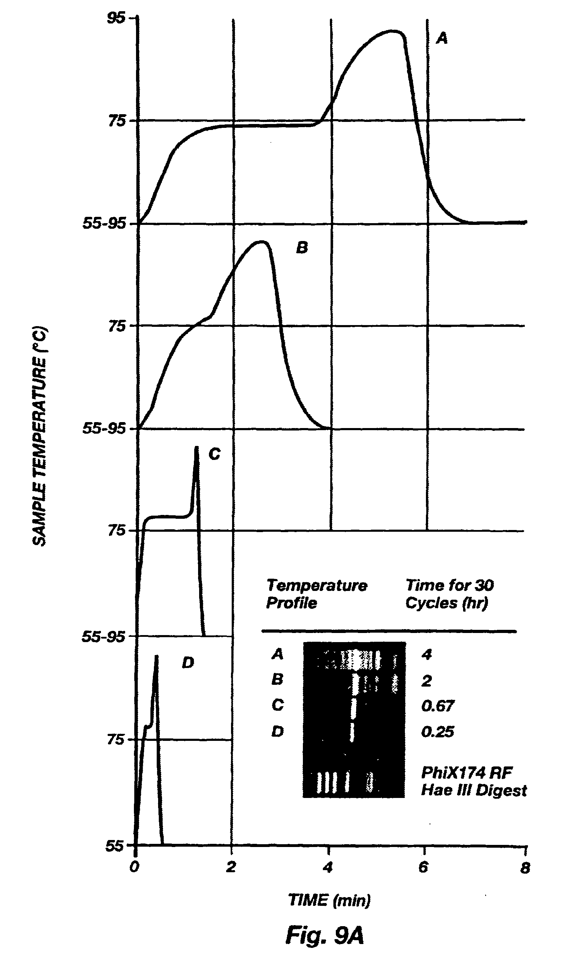

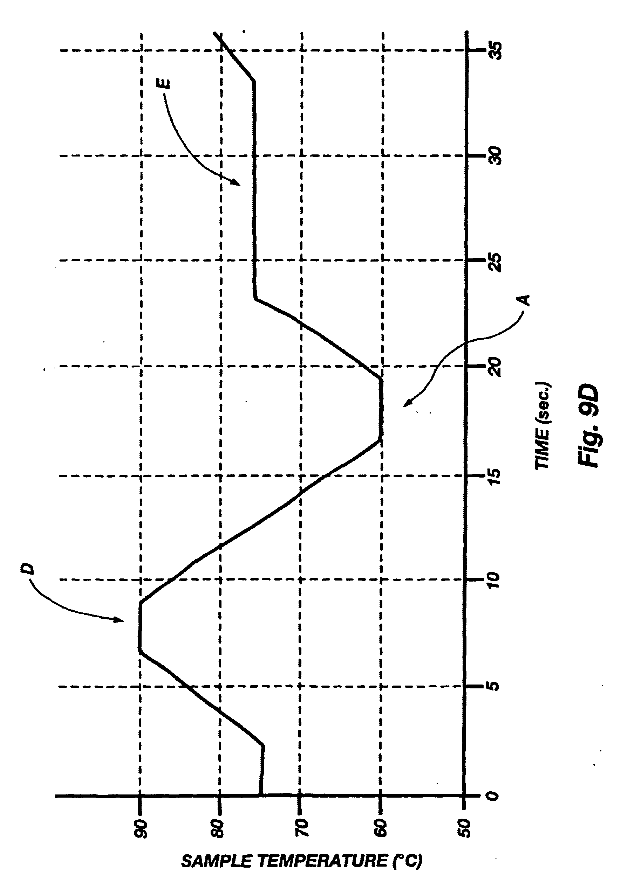

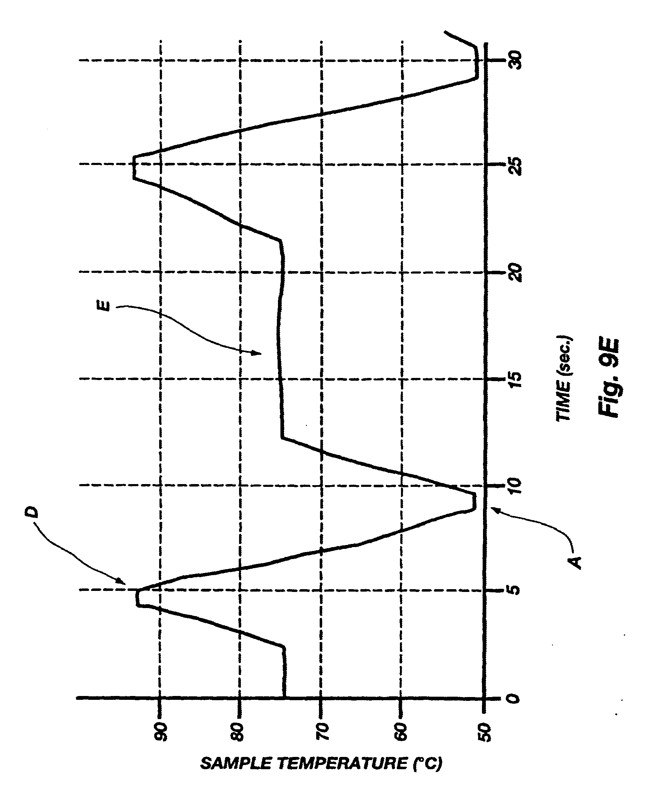

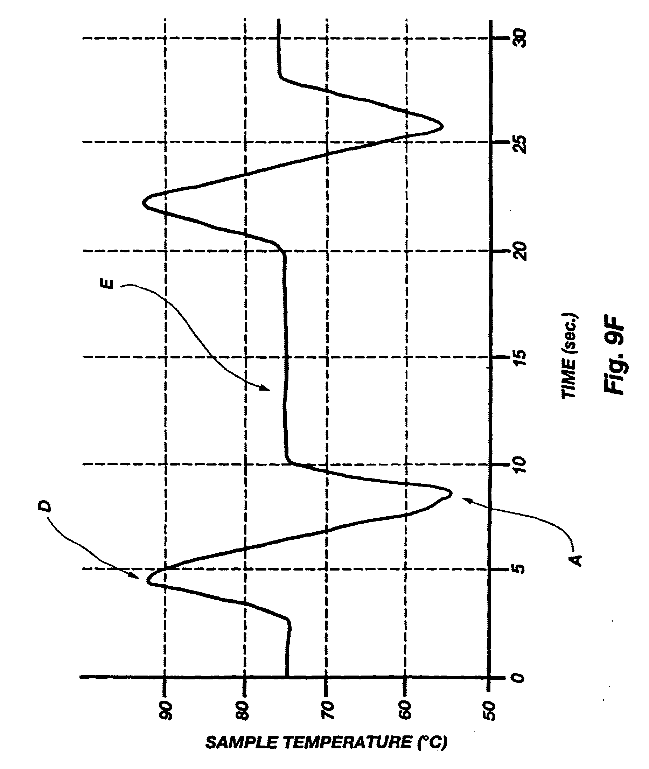

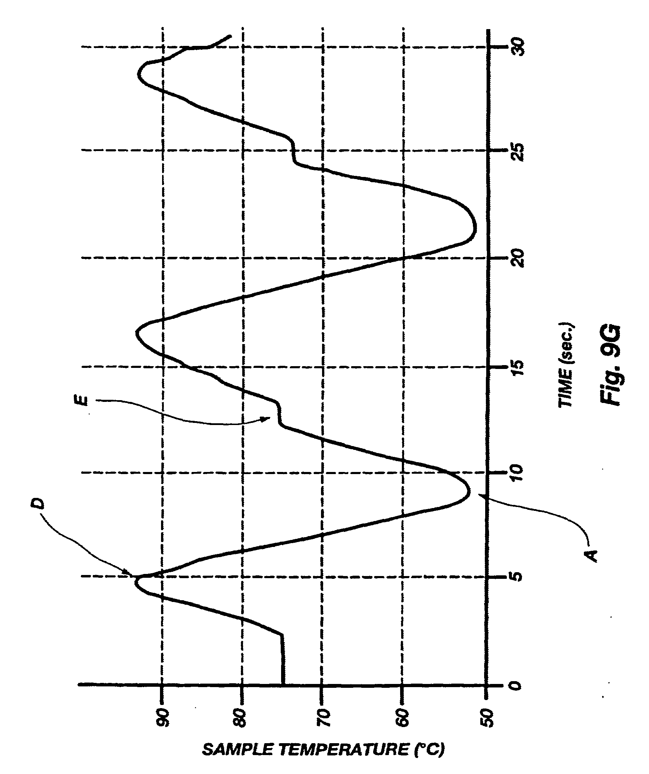

Figure 9A shows the results of four different temperature/time profiles (A-D) and their resultant amplification products after thirty cycles (A-D). The profiles A and B in Figure 9A were obtained using a prior art heating block device using the prior art microfuge tube. As can be seen in Figure 9A, the transitions between temperatures are slow and many nonspecific bands are present in profiles A and B. Profile B shows improvement in eliminating some of the nonspecific bands (in contrast to profile A) by limiting the time each sample remains at each temperature thus indicating that shorter times produce more desirable results.

Profiles C and D were obtained using the apparatus of Figures 8A-B. As can be seen in Figure 9A, amplification is specific and, desirably, even though yield is maximal in C (60 second elongation) it is still entirely adequate in D (10 seconds elongation).

The optimal times and temperatures for the amplification of a 536 bp fragment of β-globin from human genomic DNA were also determined. Amplification yield and product specificity were optimal when denaturation (93°C) and annealing (55°C) were less than 1 second. No advantage was found to longer denaturation or annealing times. The yield increased with longer elongation times at (77°C) but there was little change with elongation times longer than 10-20 seconds. These unexpected results indicate that the previously available devices used for DNA amplification are not maximizing the conditions needed to optimize the physical and enzymatic requirements of the reaction.

Further information can be obtained from: Wittwer, Carl T., Marshall, Bruce C., Reed, Gudrun B., and Cherry, Joshua L., "Rapid Cycle Allele-Specific Amplification with Cystic Fibrosis ΔF_{50B} Locus," 39 clinical Chemistry 804 (1993) and Wittwer, Carl T., Reed, Gudrun H., and Rire, Kirk M., "Rapid DNA Amplification," THE POLYMERASE CHAII REACTION 174 (1994) which are both now incorporated herein by this reference.

From the information provided in Figure 9A, it can be seen that the embodiments of the present invention subject the samples placed therein to rapid thermal cycling wherein the temperature of the sample is increased and decreased at a rate preferably at least as great as 0.5°C/second. In the case of the present invention carrying out the polymerase chain reaction, the temperature change is preferably carried out over an approximate range of between 30°C to 50°C. It is preferred that the thermal cycles be carried out quickly enough to complete at least thirty thermal cycles in forty minutes and more preferably complete thirty thermal cycles in twenty minutes and most preferably complete thirty thermal cycles in ten minutes.

The apparatus 100 more preferably increases and decreases the temperature of the sample at a rate at least as great as 1.0°C/second and even more preferably at a rate at least as great as 4.0°C/second and most preferably at a rate at least as great as 10.0°C/second. Critically, the biological sample, not just the surrounding medium and/or the sample container, must undergo the specified thermal change. The previously available devices, while having the drawback of not able to perform thermal changes as rapidly as the present invention, also did not recognize the problem of changing the temperature of the sample, not just the temperature of the surrounding medium and container, rapidly and uniformly.

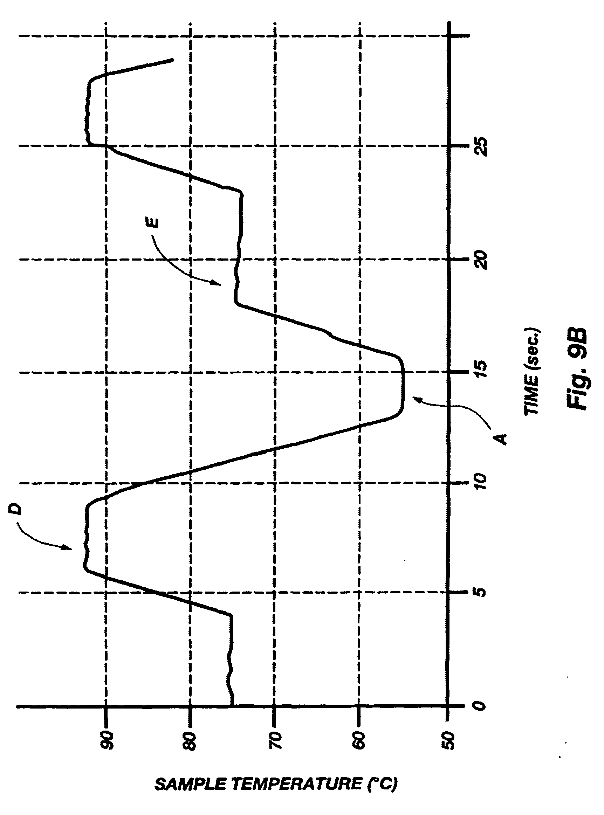

Referring now to the chart of Figure 9B, the method of the present invention can desirably achieve thermal cycling preferably at a rate at least as great as 10°C/sec., and more preferably at a rate at least as great as 20°C/sec., over a temperature range of greater than about 20°C, more preferably over a temperature range of greater than about 30°C, and most preferably over a temperature range of about 40°C. Figure 9B shows the temperature in °C of the biological sample, not just the surrounding air or container, as the biological sample undergoes the thermal cycle. Figure 9B shows a PCR sample beginning at about 74°C and being heated to a denaturation temperature, indicated at D, of about 92°C for 2 seconds. The sample is then cooled to an annealing temperature, indicated at A, of about 55°C for two seconds. The transition between the denaturation temperature and the annealing temperature covers a range of 37°C in just under 4 seconds providing a rate at least as great as 10°C/sec. The sample is then warmed to an extension temperature of 74°C for five seconds as indicated at E in Figure 9B. The cycling of the sample through the denaturation temperature, the annealing temperature, and the extension temperature is repeated thirty time or as many times as desired.

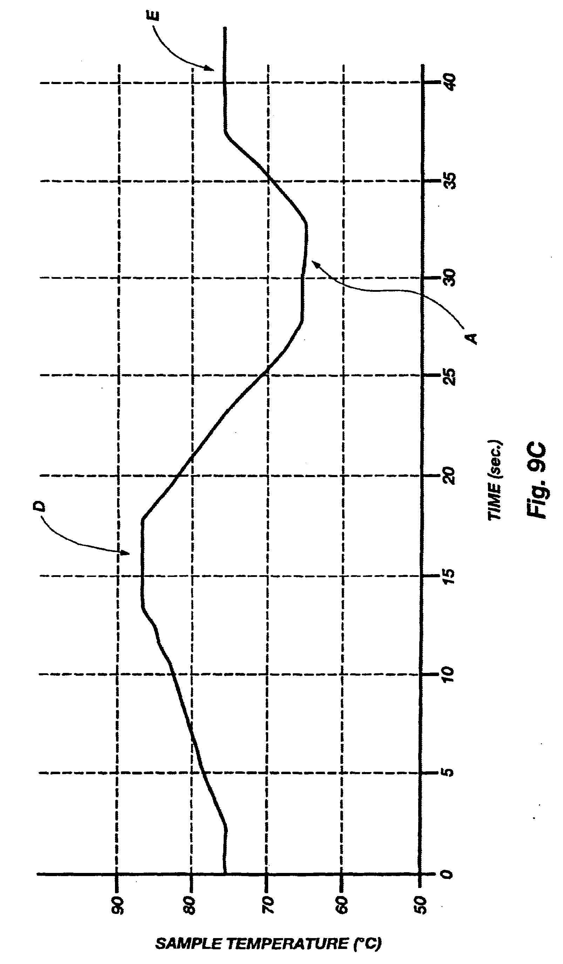

Figures 9C-G show exemplary cycles of other preferred temperature/time profiles which are achieved by the present invention. It will be understood that those skilled in the art can alter the represented temperature/time profiles to carry out specific processes in accordance with the present invention. Those skilled in the art will also appreciate that the previously available devices and methods, such as devices which conduct heat to and from the sample via a solid or liquid, cannot provide, and do not suggest or teach, the temperature/time profiles described herein. Furthermore, it will be appreciated that the previously available devices and methods utilizing air and the transfer medium, for example previously available chromatographic ovens, cannot provide, and do not suggest or teach, the temperature/time profiles described herein.

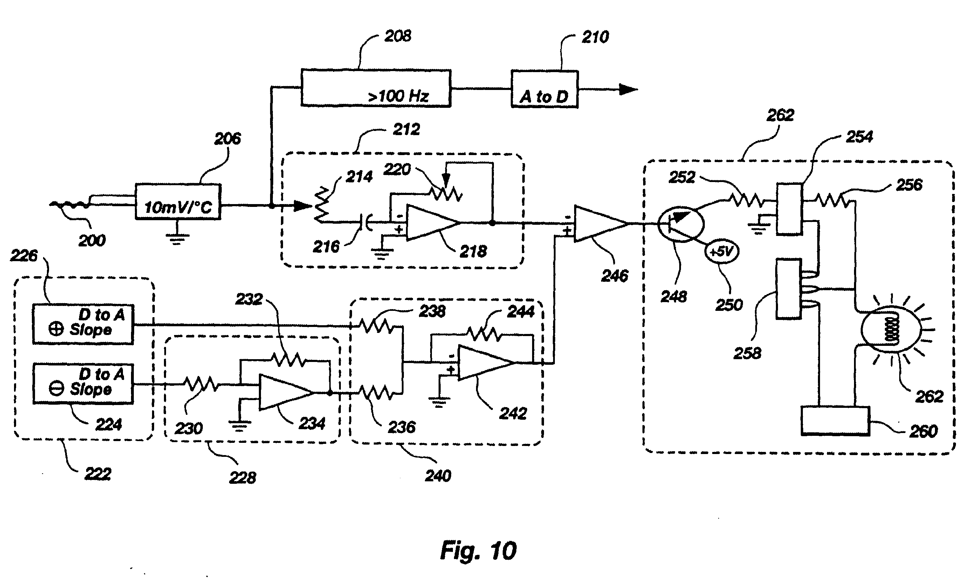

In order to provide the fastest thermal cycling time, it is preferred that the lamp (112 in Figures 8A and 8B) be rated at 2000 watts or a plurality of lamps be included which provide similar output. It is also preferred to include a temperature slope control circuit which is represented in Figure 10 in conjunction with an A-bus controller/acquisition system using an 8052 micro controller board with a clock and high level program interpreter available from Alpha Products (model no. SP-127) of Darian, Connecticut. Exemplary programming code used in connection with the described microcontroller is included in the Programming Code Appendix attached hereto and incorporated herein. The programming code provided in the Appendix is a BASIC52 file for serial downloading into the microcontroller and provides exemplary temperature slope control during thermal cycling. Use of the 2000 watt heat producing device and the describe control structures allows rates of 20°C/sec. to be desirably obtained.

The preferred arrangement for the temperature slope control circuit represented in Figure 10 will be explained with the understanding the additional necessary components not explicitly illustrated in Figure 10 can readily be supplied by those skilled in the art.

The temperature slope control circuit of Figure 10 includes a thermocouple 200 matched to the sample temperature response as explained earlier. The thermocouple 200 is connected to an integrated circuit 206, which preferably is one known in the art as an AD595, whose output is conveyed to a 4th order low pass filter 208 with a cutoff frequency of 100 Hz and to a 12 bit analog-to-digital convertor 210 whose output is used to provide a digital display of the temperature.

The output of the circuit 206 is also conveyed to a measured slope circuit 212. The measured slope circuit 212 preferably includes a 353 operational amplifier 218, a 100 KΩ potentiometer 214, a 1 MΩ potentiometer 230, and a 22 µF capacitor. The measured slope circuit 212 outputs a signal to the inverting input of a 353 operational amplifier 246.

A slope set circuit 222 includes a positive slope set digital-to-analog converter 226 and a negative slope set digital-to-analog converter 224. The digital-to-analog converters 224 and 226 are preferably 8-bit digital-to-analog converters referred to in the art as DA147. The slope set circuit can preferably receive instructions from another digital device (not illustrated in Figure 10) such as a personal computer. The output of the slope set circuit 228 is communicated to a summing circuit 240.

The summing circuit 240 preferably includes 100 KΩ resistors 236, 238, and 244 and a 353 operational amplifier 242. The output of the summing circuit 240 is conveyed to the non-inverting input of the operational amplifier 246 and represents the desired slope of the temperature change. The output of the operational amplifier 246 is provided to a transistor 248 contained within a power switching circuit 262.

The power switching circuit 262 includes a 5 VDC supply 250 providing current to the transistor 248. The transistor 248 has its emitter connected to a 3010 circuit 254 by way of resistor 252 which is preferably a 330 Ω resistor. The 3010 circuit 254 includes an output connected in series with a resistor 256 which preferably is a 180 Ω resistor. A triac 258 is preferably used to control the current delivered to a lamp 262, or other heat producing device, from a source of AC current 260.

The temperature slope control circuit represented in Figure 10, in cooperation with the other described system components, provides thermal cycling of biological samples as great as 20°C/sec over a temperature range of 30°C, and most preferably over a temperature range of 40°C, with homogeneity being maintained throughout the biological sample.

It will be appreciated that the apparatus described herein can readily be used for many different applications including: polymerase chain reaction processes; cycle sequencing; and, other amplification protocols such as the ligase chain reaction. The present invention also advantageously provides an apparatus for accurately controlling the temperature of samples located in the sample chamber and quickly and accurately varies the temperature of samples located in a chamber according to a predetermined temperature versus time profile.

### Continuous Fluorescence Monitoring of DNA Amplification

As indicated earlier, the polymerase chain reaction can be performed rapidly. Using the methods and apparatus described herein, the necessary number of temperature cycles can routinely be completed in much less than time than possible with the prior art, for example in less than 15 minutes. By minimizing denaturation and annealing times, the specificity and yield of rapidly cycled amplifications are also improved to an extent not previously possible. Moreover, in addition to facilitating rapid heat transfer, the use of optically clear capillary tubes allows for continuous fluorescence monitoring DNA amplification in accordance with the present invention.

Fluorescent probes can be used to detect and monitor DNA amplification. Useful probes include double-stranded-DNA-specific dyes and sequence-specific probes. With the intercalater ethidium bromide, UV-excited red fluorescence increases after amplification in capillary tubes or microfuge tubes. Sequence-specific fluorescence detection is possible with oligonucleotide hybridization probes. For example, dual-labeled fluorescein/rhodamine probes can be cleaved during polymerase extension by 5'-exonuclease activity, separating the fluorophores and increasing the fluorescein/rhodamine fluorescence ratio.

Fluorescence can be measured after temperature cycling is complete, once per cycle as a monitor of product accumulation, or continuously within each cycle. In contrast to the present invention, the previously available sequence-specific methods have been limited to endpoint analysis.

The present invention allows cycle-by-cycle monitoring for quantification of initial template copy number. To carry out such cycle-by-cycle monitoring, fluorescence is acquired during the extension or combined annealing/extension phase of each cycle and related to product concentration. A quantitative assay for hepatitis C RNA using the intercalater, YO-PRO-1™ is known in the art. See Ishiguro, T., J. Saitch, H. Yawata, H. Yamagishi, S. Iwasaki, and Y. Mitoma, 1995, Homogeneous quantitative assay of hepatitis C virus RNA by polymerase chain reaction in the presence of a fluorescent intercalater, Anal. Biochem. 229:207-213. Prior to the present invention, continuous fluorescence monitoring within each cycle has not been efficiently and accurately carried out.

Disclosed herein is a rapid temperature cycler integrated with 2-color fluorescence optics that provides continuous fluorescence monitoring. As will be more fully discussed below, three different preferred fluorescence techniques for following DNA amplification are provided herein as specific examples of the present invention. Those skilled in the art will be familiar with the use of ethidium bromide in fluorescence techniques which can be used in accordance with the present invention. In the presently preferred embodiment described below, it is preferred that SYBR® Green I, which is well known in the art and available from Molecular Probes of Eugene, Oregon, be used as a double-strand-specific dye. In accordance with the present invention, time, temperature and fluorescence is acquired every 200 msec. Such data reveal fine details of product denaturation, reannealing and extension during rapid cycling not available in the previously available apparatus and methods. Moreover, as will be fully discussed below, the results obtained using a 5'-exonuclease quenching probe, which is well known in the art, is compared to the results obtained using double-strand-specific fluorescence. Furthermore, as will also be fully discussed below, the use of a novel fluorescence scheme based on adjacent hybridization probes with resonance energy transfer from fluorescein to a known cyanine dye, Cy5™, is disclosed. Mujumdar, R.B., L.A.Ernst, S.R. Mujumdar and A.S. Waggoner, 1989, Cyanine dye labeling reagents containing isothiocyanate groups, Cytometry 10:11-19.

As will be appreciated by those skilled in the art, once-per-cycle monitoring of multiple samples undergoing DNA amplification is a powerful quantitative tool. As will be appreciated by an understanding of this disclosure, continuous monitoring within a cycle can identify the nature of probe fluorescence and provide insight into DNA amplification mechanics not previously available in the art.

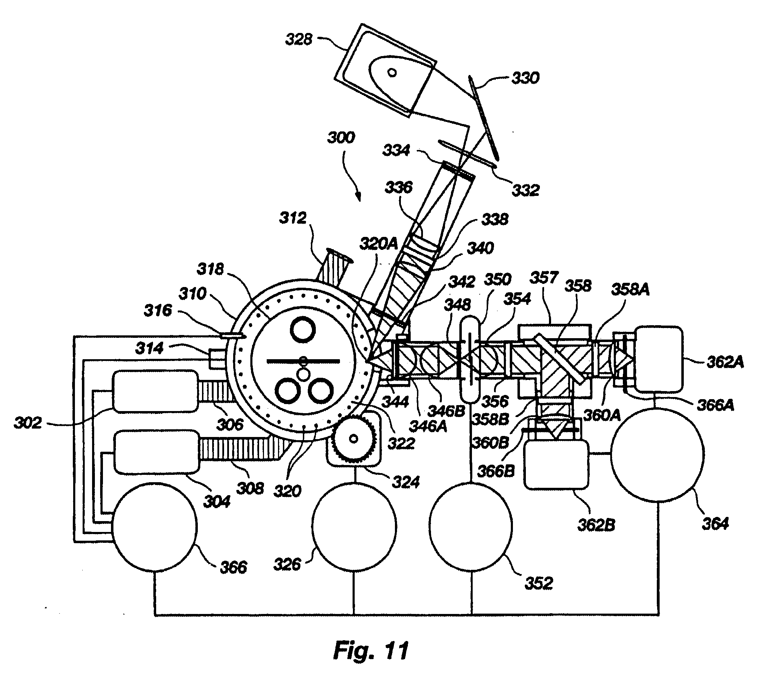

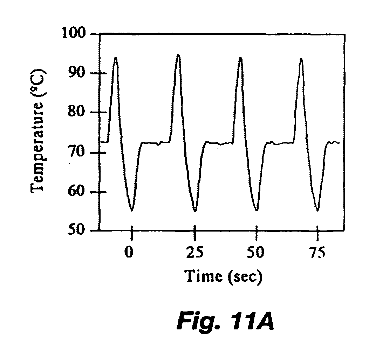

Referring now to Figure 11, a schematic view of a preferred rapid temperature cycler with fluorescence detection, generally designated at 300, is provided. A forced air hot air source 302 is preferably a commercially available device including a 1600 watt heating coil and fan. A forced air cool air source 304 is preferably a 2200 rpm shaded pole blower available in the art from Dayton of Niles, Illinois, model no. 4C006B. It is preferred that the cool air source 304 provide ambient temperature air, but it is within the scope of the present invention to utilize means for providing fluid that is at a temperature lower than ambient air temperature. In the embodiment of Figure 11, corrugated black nylon tubing 306 and 308 having a 2.5 cm diameter is used to connect the forced air hot air source 302 and the forced air cool air source 304, respectively, to a sample chamber 310. A vent 312 and an exhaust fan 314 move air out of the sample chamber 310. The interior of the sample chamber 310 is shielded from outside light.

Temperature of the samples within the sample chamber 310 is monitored by a tubular, metal-sheathed thermocouple 316, available from Idaho Technology of Idaho Falls, Idaho, model no. 1844, matched in thermal response to samples held in capillary tubes. Temperature homogeneity within the sample chamber 310 is achieved by a central sample chamber fan 318. The sample chamber fan preferably includes a 1.7 X 11 cm fan blade available from Idaho Technology, model no. 1862, and a motor available from Idaho Technology, model no. 1861, which creates air velocities of at least 800 to 1000 m/minute within the sample chamber 310.

Within the sample chamber 310, a plurality of samples held in capillary tubes, some of which are indicted at 320, are placed vertically on a rotatable carousel 322. The carousel 322 is preferably 14 cm in diameter and rotated by a 400 step/rev stepper motor 324 controlled by a microstepping drive module 326. The stepper motor 324 is preferably one available from New England Affiliated Technologies of Lawrence, Massachusetts, model no. 2198364, and the microstepping drive module 326 is preferably on also available from New England Affiliated Technologies, model no. MDM7 microstepping drive module, which provides 12,800 steps per rotation of the carousel 322.

Still referring to Figure 11, a fluorescence excitation source 328 is provided. The excitation path will now be described. The fluorescence excitation source 328 is preferably a 75 watt xenon arc source focused with an elliptical reflector, the xenon arc source being preferably available from Photon Technology International of South Brunswick, New Jersey, model no. A1010, with f/2.5 elliptical reflector. Alternatively, a light emitting diode can be used. The light emitted by the fluorescence excitation source 328 is focused to about 2 mm using an adjustable iris 334 such a one available in the art from Rolyn (Covina, California), model no. 75.0125. The light emitted from the fluorescence excitation source 328 impinges upon a cold mirror 330, which is preferably available from Rolyn, model no. 60.4400, and passes through heat absorbing glass 332, which is preferably one available from Rolyn, model no. 65.3130. After collimation through a planoconvex lens 336, preferably available from Rolyn, model no. 10.0260, a 450-490 nm bandpass interference filter 338, preferably available from Omega Optical of Brattleboro, Vermont, model no. 470RDF40, a focusing planoconvex lens 340, preferably available from Rolyn, model no. 10.0260, and a 1 mm silica window 342, preferably available from Omega, to prevent condensation on the just described optical components during temperature cycling. Using the described excitation path, a 5-7 mm section of one capillary sample tube 320A is illuminated.

The collection path for collecting the fluorescence emitted from the sample 320A will be described next by still referring to Figure 11. The optics of the collection path include a 1 mm silica window 344 also included to prevent condensation on the other optical components. Two opposed aspheric lenses 346A&B, preferably available from Rolyn, model no. 17.1175, which function to focus emitted fluorescence onto a 2 x 10 mm slit 348, which can be fabricated from cutting exposed X-ray film, which functions as a spatial filter. After the spatial filter 348, the emitted fluorescence is imposed upon a 35 mm electronic shutter 350 operated via an electronic shutter control 352. The 35 mm electronic shutter 350 is preferably a Uniblitz shutter model no. VS35 and the electronic shutter control 352 is preferably driver model no. D122, both available from Vincent Associates of Rochester, New York. A collimating aspheric lens 354, preferably on available from Rolyn model no. 17.1175, is also provided.

A filter 356 is also included when detection of SYBR® Green I emissions is desired. The filter 356 is a 520-580 nm band pass filter, available from Omega model no. 550RDF60, used for single color acquisition. For detection of other emissions a combination of dichroic filter 358 and wavelength filters 358A and 358B are used in a filter block. For separation of fluorescein and rhodamine emissions, a dichroic filter 358 is used which preferably consists of a 560 nm long pass dichroic filter, preferably available from Omega, model no. 560 DRLP, and a 520-550 nm band pass filter (358A), preferably available from Omega, model no. 535DF30, for detection of fluorescein, and a 580-620 nm band pass filter (358B), preferably available from Omega, model no. 600DF40, for detection of rhodamine. For separation of fluorescein and Cy5 emissions, the dichroic filter 358 preferably is a 590 nm long pass dichroic filter, available from Omega, model no. 590 DRLP, and filters 358A&B preferably consist of a 520-550 nm band pass filter (358A), available from Omega, model no. 535DF30, for detection of fluorescein, and a 660-680 nm band pass filter (358B), available from Omega, model no. 670DF20, for Cy5 detection.

Still referring to Figure 11, after being subjected to the filter 358, the fluorescence is focused through two planoconvex lenses 360A & 360B, each preferably available from Edmund of Barrington, New Jersey, model no. 32970, onto photomultiplier tubes 362A and 362B. The photomultiplier tubes 362A and 362B are preferably ones available from Hamamatsu of Middlesex, New Jersey, model no. R928, and are housed in a housing, preferably one available from Photon Technology International, model no. 714, with analog acquisition. A PMT and data acquisition control module 364 is also preferably provided. Manual PMT shutters 366A and 366B, as known in the art, are also provided.

The forgoing described optical components are preferably 5 cm in diameter and mounted in 5 cm universal lens mounts, such as those available from Rolyn, model no. 90.0190. As will be appreciated by those skilled in the art, many of the necessary structural components were machined from black Delrin™.

Using the apparatus represented in Figure 11, DNA amplification was performed in 50 mM Tris, pH 8.5 (25°C), 3 mM MgCl₂, 500 µg/ml bovine serum albumin, 0.5 µM of each primer, 0.5 mM of each deoxynucleoside triphosphate and 0.2 U of Taq polymerase per 5 µl sample unless otherwise stated in the following examples. Human genomic DNA (denatured for 1 min by boiling) or purified amplification product was used as DNA template. Purified amplification product was obtained by phenol/chloroform extraction and ethanol precipitation, see Wallace, D.M. 1987. Large- and small-scale phenol extractions and precipitation of nucleic acids, p. 33-48, in S.L. Berger and A.R. Kimmel (Eds.), Guide to Molecular Cloning Techniques (Methods in Enzymology, Vol. 152), Academic Press, Orlando, followed by removal of primers by repeated washing through a Centricon 30 microconcentrator (available from Amicon of Danvers, Massachusetts). Template concentrations were determined by absorbance at 260 nm. A₂₆₀/A₂₈₀ ratios of templates were greater than 1.7.

Primers were synthesized by standard phosphoramidite chemistry as known in the art, namely, using Pharmacia Biotech Gene Assembler Plus (Piscataway, New Jersey). The 180 base pair fragment of the hepatitis B surface antigen gene was amplified using primers 5'-CGTGGTGGACTTCTCTCAAT-3' (SEQ ID NO:1), and 5'-AGAAGATGAGGCATAGCAGC-3' (SEQ ID NO:2) (Genbank sequence HVHEPB). SYBR® Green I was obtained from Molecular Probes (Eugene, Oregon). The β-actin primers and fluorescein/rhodamine dual probe were obtained from Perkin Elmer (Foster City, California) (no. N808-0230,). The human β-globin primers RS42/KM29 (536 base pairs) and PC03/PC04 (110 base pairs) are described in Wittwer, C.T., G.C. Fillmore and D.R. Hillyard, Automated polymerase chain reaction in capillary tubes with hot air, Nucl. Acids. Res. 17:4353-4357 which is now incorporated herein by reference.

The single labeled probes 5'-CAAACAGACACCATGGTGCACCTGACTCCTGAGGA-fluorescein-3' (SEQ ID NO:3) and 5'-Cy5-AAGTCTGCCGTTACTGCCCTGTGGGGCAAG-phosphate-3' (SEQ ID NO:4) were synthesized using a fluorescein phosphoramidite (available from Glen Research of Sterling, Virginia, no. 10-1963) a Cy5™ phosphoramidite (available from Pharmacia no. 27-1801-02), and a chemical phosphorylation reagent (available from Glen Research no. 10-1900). These adjacent probes hybridize internal to the PC03/PC04 β-globin primer pair on the same DNA strand and are separated by one base pair. Probes were purified by reverse phase C-18 high pressure liquid chromatography and homogeneity checked by polyacrylamide electrophoresis and absorbance (A₂₆₀ and the absorbance maximum of the fluorophore). Hybridization probes (β-actin and β-globin) were used at 0.2 µM each.

In the pertinent examples described herein, amplification samples of 5 µℓ were loaded into capillary sample tubes 230. The capillary sample tubes preferably are those available from Idaho Technology, model no. 1705, having dimensions of 1.02 mm O.D. and 0.56 mm I.D. Once loaded, the capillary sample tubes were sealed with a butane flame. The surface of the capillary sample tube was cleaned with optical grade methanol before it was loaded into the carousel 322 of the rapid temperature cycler with fluorescence detection 300.

Control of the components represented in Figure 11 was achieved by use of a graphical programming language known as LabView (available from National Instruments, Austin, Texas) and a 12-bit multifunction input/output card (available from National Instruments under the designation AT-MIO-E2) in a PC compatible computer 366 utilizing an 80486 Intel® microprocessor running at a clock speed of 120 MHZ. Analog output channels on the input/output card were used to control the sensitivity, *i*.*e*. the PMT voltage, of each photomultiplier tube 362A&B. Analog input channels on the input/output card receive the signals from each photomultiplier tube 362A&B. The PC compatible computer, through the input/output card controls the position, rate and direction of movement of the carousel. As will be appreciated, when multiple capillary sample tubes are loaded, the carousel 322 rapidly positions each capillary sample tube 320 sequentially at the monitoring location for 100 msec acquisitions. For continuous monitoring of a single capillary sample tube 320A, data are averaged and acquired every 200 msec. Time, temperature, and 2 channels of fluorescence are continuously displayed as fluorescence vs. cycle number and fluorescence vs. temperature plots. The carousel should be positioned where maximal fluorescence and signals are acquired. When a single capillary sample tube 320A was analyzed, the signals were acquired every 200 msec with an integrating time constant on the photomultiplier tubes 364 being set at 50 msec. When multiple photomultiplier tubes 366A&B were used, the time constant was set at 0.5 msec and the carousel rotated once to locate the precise position where each capillary sample tube 320 provided maximum fluorescence in each channel. After positioning the capillary sample tube 320A at a location where maximum fluorescence was obtained, the sensitivity of each PMT 362A&B was adjusted and the carousel rotated again to count and locate the position of all the capillary sample tubes 320. Signals were obtained once each amplification cycle during extension by sequentially positioning each capillary sample tube 320 on the carousel 322 at the monitoring position for 100 msec. Temperature control programming was modified from a commercial rapid temperature cycler available from Idaho Technology under the designation Rapidcycler using an 8051 cross compiler available from Systronics, Salt Lake City, Utah, designated BC151 and Dallas development system (also available from Systronics under the designation DPB2).

In practice, the temperature response of the rapid temperature cycler with fluorescence detection 300 is similar to response obtained with the embodiment of the present invention disclosed in Figures 8A&B allowing 20-30 second cycles (30 cycles in 10-15 min) as represented in the temperature vs. time chart of Figure 11A. when a double strand-specific fluorescent dye is present during amplification, fluorescence generally increases as more double stranded product is made, see Higuchi, R., G. Dollinger, P.S. Walsh, and R. Griffith, 1992, Simultaneous amplification and detection of specific DNA sequences, Bio/Technology 10:413-417.

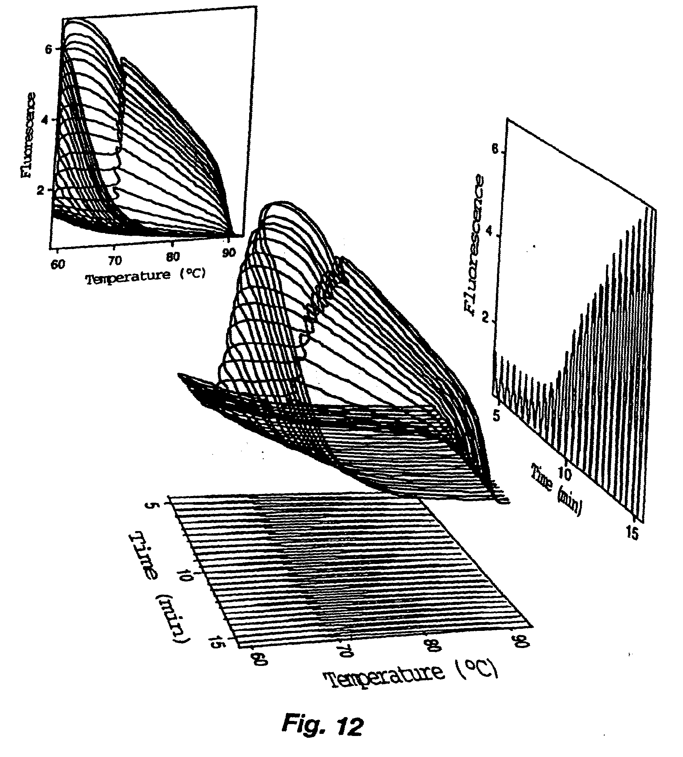

Fluorescence also depends on temperature, a confounding effect during temperature cycling that is usually eliminated by considering fluorescence once per cycle at a constant extension temperature. However, if temperature, time and fluorescence are acquired every 200 msec during rapid cycle amplification, a three dimensional spiral is traced in space as represented in Figure 12. The 3D curve represented in Figure 12 is also projected in Figure 12 as 2D plots of temperature vs. time, fluorescence vs. time, and fluorescence vs. temperature. The temperature vs. time projection of Figure 12 repeats each cycle and provides essentially the same information as set forth in Figure 11A. Because fluorescence varies inversely with temperature, the fluorescence vs. time projection shown in Figure 12 at early cycles is a scaled mirror image of the temperature vs. time plot. As product accumulates, the fluorescence increases at all temperatures with double stranded product. However at denaturation temperatures, fluorescence returns to baseline since only single stranded DNA is present.

The fluorescence vs. temperature projection of double stranded dyes shown in Figure 12 eliminates the time axis and shows the temperature dependence of strand status during DNA amplification. The fluorescence vs. temperature projection shown in Figure 12 is for a 180 base pair fragment of hepatitis B virus DNA.

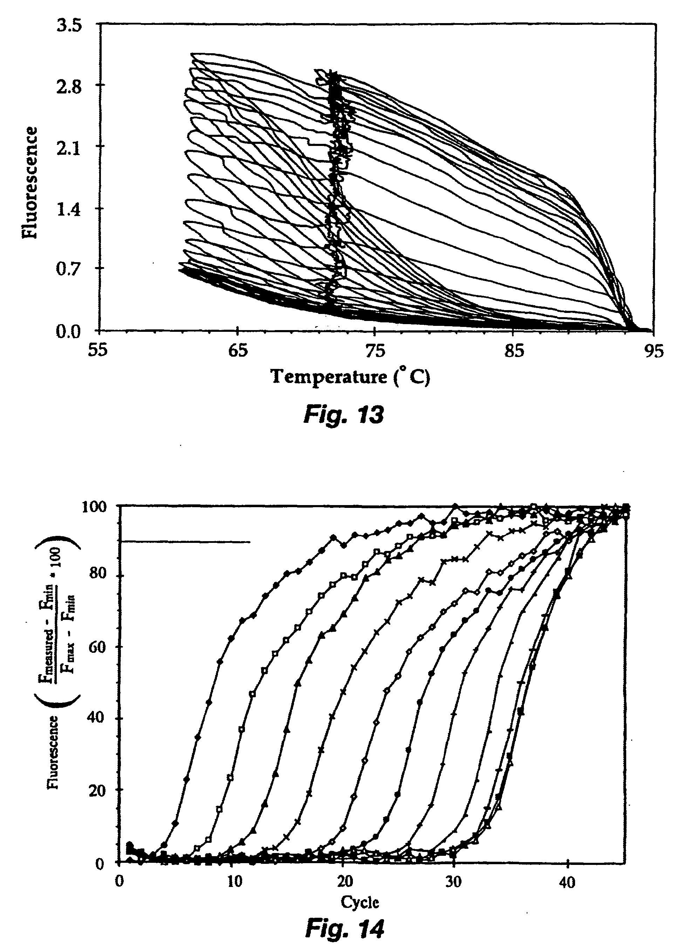

The fluorescence vs. temperature projection shown in Figure 13 is for a 536 base pair fragment of human β-globin DNA. Early cycles represented in Figure 13 appear identical, with a nonlinear increase in fluorescence at lower temperatures. As amplification proceeds, later cycles appear as rising loops between annealing and denaturation temperatures that show significant hysteresis. That is, the observed fluorescence during heating is greater than that during cooling. As the sample is heated, fluorescence is high until denaturation occurs (apparent as a sharp drop in fluorescence). As the sample cools from denaturation to annealing temperatures, double strand signal increases rapidly. Fluorescence continues to increase during extension while the temperature is held constant.

When multiple samples are monitored once each cycle with SYBR® Green I, a 10⁷-10⁸ range of initial template concentration can be discerned as represented in Figure 14. When the data are normalized as the percent maximal fluorescence of each capillary sample tube 320, one hundred initial copies are clearly separated from ten copies. However, the difference between one and ten copies is marginal, and no difference is observed between zero and one average copies per capillary sample tube 320.

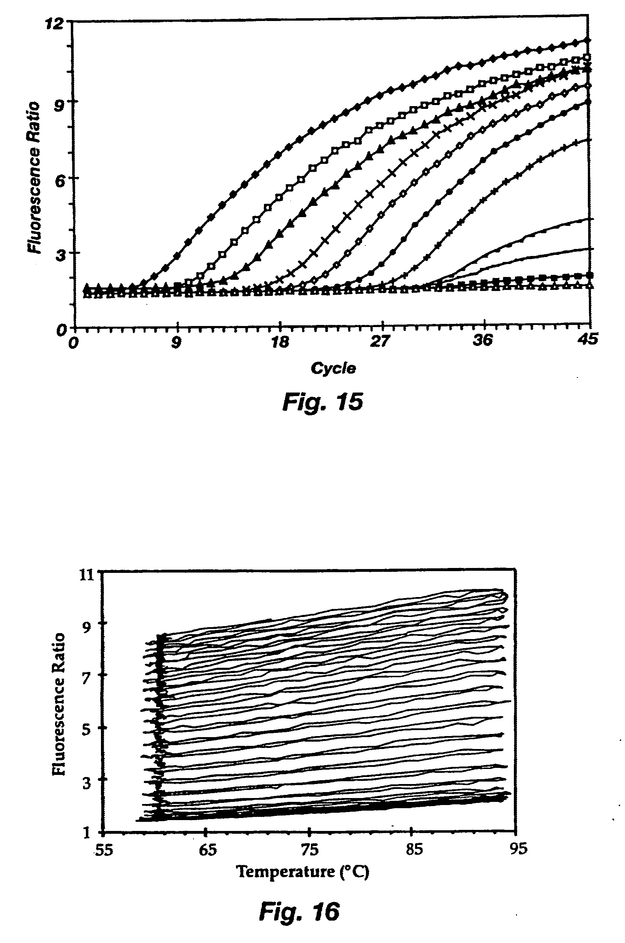

In contrast, sequence-specific probes have a similar dynamic range but, as shown in the plot of Figure 15, appear to discriminate even a single initial template copy from negative controls. Signal generation with 5'-exonuclease probes is dependent not only on DNA synthesis, but requires hybridization and hydrolysis between the fluorophores of the dual-labeled probe. This hydrolysis reduces quenching and the fluorescence ratio of fluorescein to rhodamine emission increases. Whereas the fluorescence from double strand dyes levels off with excess cycling, the signal from exonuclease probes continues to increase with each cycle. Even though no net product is being synthesized, probe hybridization and hydrolysis continue to occur. As the template copy number decreases below 10³, signal intensity decreases, but low copy numbers can still be quantified because the negative control signal is stable.

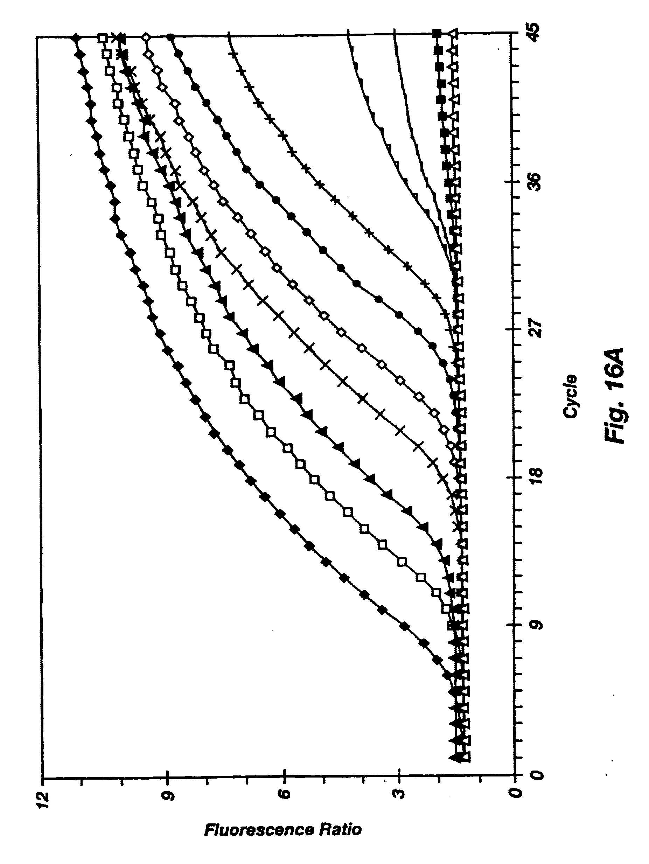

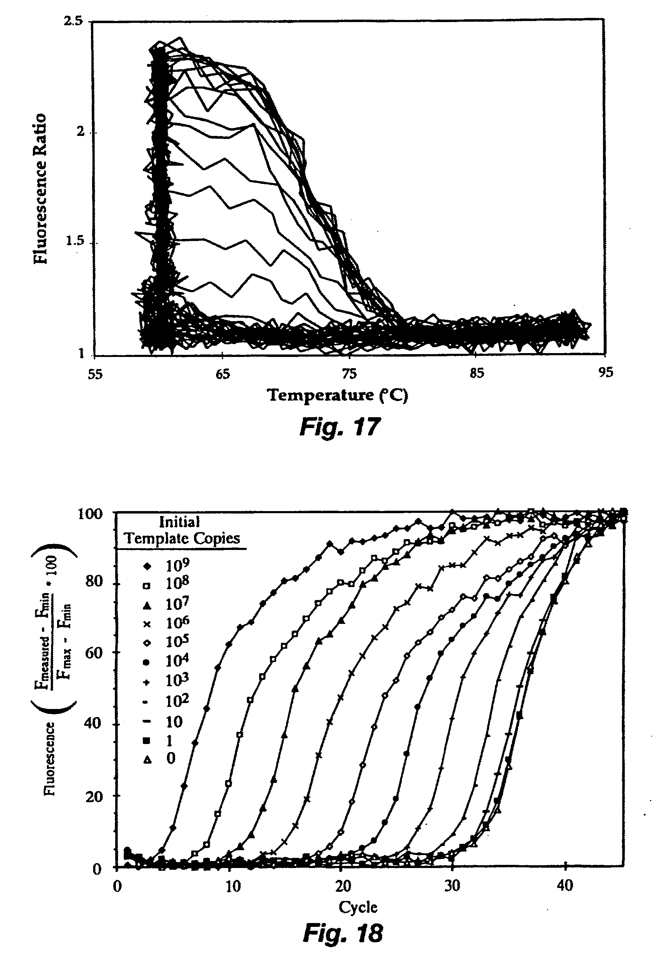

Fluorescence vs. temperature plots of 5'-exonuclease probes confirm that probe hydrolysis is the mechanism of signal generation. In Figure 16, a fluorescence vs. temperature plot of 2-temperature cycling is shown with the β-actin exonuclease probe. Each cycle the fluorescence ratio varies linearly with temperature and there is little if any hysteresis. The signal increases each cycle during the annealing/extension phase when probe hydrolysis is presumed to occur. Although the fluorescence of both fluorescein and rhodamine decreases with increasing temperature (data not shown), the rate of change is greater for rhodamine, resulting in an increasing ratio with increasing temperature. No temperature-dependent hybridization effects are apparent with the 5'-exonuclease probe. Figure 16A shows a plot of fluorescence ratio vs. cycle number plot for different initial template copy number, according to the legend in Figure 18.

In contrast, when the fluorescence signal is dependent only on hybridization, fluorescence ratio vs. temperature plots show a different pattern with obvious hysteresis during 2-temperature cycling, as plotted in Figure 17. Two adjacent hybridization probes are present, an upstream probe labeled 3' with fluorescein and a downstream probe labeled 5' with Cy5™. The probes are separated by a 1 base pair gap. During the annealing/extension phase, the probes hybridize to accumulating product and the Cy5™ to fluorescein fluorescence ratio increases. During heating to product denaturation temperatures, the probes appear to dissociate between 65 and 75°C, returning the fluorescence ratio to background levels. The change in fluorescence ratio during hybridization is largely due to an increase in Cy5™ fluorescence from resonance energy transfer.

From the foregoing discussion, it will be appreciated that fluorescence monitoring during DNA amplification is an extraordinarily powerful analytical technique. Using the productive and cost efficient apparatus to provide real time monitoring, sequence-specific detection and quantification can be achieved in five to twenty minutes, depending on the number of initial template copies present. Double-strand-specific dyes such as ethidium bromide or SYBR® Green I can be used as generic indicators of amplification. SYBR® Green I dye is preferred over ethidium bromide in many applications because it has an excitation maximum near fluorescein and often provides a stronger signal with DNA than visible excitation of ethidium bromide. Double strand dyes depend on the specificity inherent in the amplification primers. As will be appreciated by those skilled in the art, nonspecific amplification at high cycle numbers can limit detection sensitivity to about one hundred initial template copies (see Figure 14) while the present invention may provide further improvements in amplification specificity that could further improve this performance.

When low copy number detection and quantification are needed, additional specificity can be provided by fluorescent probes that require hybridization for signal generation. Cleavage of a dual-labeled exonuclease probe is one technique (see Lee, L.G., C.R. Connell and W. Bloch, 1993, Allelic discrimination by nick-translation PCR with fluorogenic probes. Nucl. Acids Res. 21:3761-3766 and Livak, K.J., S.J.A. Flood, J. Marmaro, W. Giusti and K. Deetz, 1995, Oligonucleotides with fluorescent dyes at opposite ends provide a quenched probe system useful for detecting PCR product and nucleic acid hybridization, PCR Meth. Appl. 4:357-362) that appears capable of distinguishing a single template copy from a negative control (see Figure 15). Although the final fluorescence signal is decreased when low copy numbers are amplified (presumably because of decreased amplification efficiency), quantification between zero and one hundred copies appears possible. The signal generated by exonuclease probes is cumulative and only indirectly related to product concentration. Hence, the fluorescence signal continues to increase even after the amount of product has reached a plateau. Appropriate standards to control for efficiency of amplification and cleavage would be necessary for absolute quantification.

The design, synthesis, and purification of 5'-exonuclease probes requires care. Hybridization is a necessary, but not sufficient, condition for exonuclease-derived signal; all probes are not cleaved efficiently. See 8. Lee, L.G., C.R. Connell and W. Bloch, 1993, Allelic discrimination by nick-translation PCR with fluorogenic probes, Nucl. Acids Res. 21:3761-3766, and Livak, K.J., S.J.A. Flood, J. Marmaro, W. Giusti and K. Deetz, 1995, Oligonucleotides with fluorescent dyes at opposite ends provide a quenched probe system useful for detecting PCR product and nucleic acid hybridization, PCR Meth. Appl. 4:357-362, both of which are now incorporated herein by reference. Synthesis of the dual-labeled probes involves manual addition of the rhodamine label, usually followed by reverse phase high pressure liquid chromatography.

Instead of depending on hydrolysis of a dual-labeled probe, hybridization can be detected directly through resonance energy transfer as outlined by Morrison. See Morrison, L.E., 1992, Detection of energy transfer and fluorescence quenching, p. 311-352, In L.J. Kricka (Ed.), Nonisotopic DNA probe techniques, Academic Press, San Diego, which is now incorporated herein by reference. Using 2 adjacent probes labeled separately with fluorescein and Cy5™, energy transfer to Cy5™ increases with product accumulation each amplification cycle (Figure 17). In contrast to exonuclease probes, probe synthesis is relatively simple because amidites for both fluorescein and Cy5™ are available for direct incorporation during automated synthesis. The prior art does not disclose using fluorescein and Cy5™ as a resonance energy transfer pair. Although the spectral overlap is small, the molar absorption coefficient and absorption wavelengths of Cy5™ are high compared to rhodamine. All three factors (overlap, absorptivity, and wavelength) contribute to the overlap integral that determines energy transfer rates. Cy5™ also has low absorbance at fluorescein excitation wavelengths, reducing direct excitation of the acceptor.

Conventional endpoint analysis of DNA amplification by gel electrophoresis identifies product size and estimates purity. However, because amplification is at first stochastic, then exponential, and finally stagnant, the utility of endpoint analysis is limited for quantification. Cycle-by-cycle monitoring provided by the present invention affords relatively simple quantification and closed tube fluorescence monitoring has further advantages as will be understood by those skilled in the art.

In contrast to endpoint and cycle-by-cycle analysis, the present invention can also monitor fluorescence throughout each temperature cycle. As previously discussed, continuous fluorescence monitoring with double-strand-specific dyes can be used to control temperature cycling parameters. Amplification can be stopped after a certain amount of product is synthesized, avoiding over amplification of alternative templates. The extension phase of each cycle need only continue as long as fluorescence increases. Product denaturation can be assured in real time, increasing the temperature until the fluorescence reaches baseline. Limiting the time product is exposed to denaturation temperatures is especially useful for amplification of long products.

Additional uses of continuous monitoring with fluorescent dyes is within the scope of the present invention. For example, with precise temperature control and double-strand-specific dyes, product purity could be estimated by melting curves. With rapid temperature control, absolute product concentration could be determined by reannealing kinetics. The only requirements are fluorescence capability, the ability to change temperatures rapidly, and strict intra-sample temperature homogeneity. The high surface area to volume ratio of capillary sample tubes, combined with the ease of mixing air, allows precise control of sample temperature at a speed not possible with other designs. For example, sample temperature vs. time plots in capillary tubes show sharp spikes at denaturation and annealing temperatures whereas several seconds are required for all of the sample to reach equilibrium in the conical plastic tubes used in the prior art. Moreover, although high surface area to volume ratios are possible on etched silicon or glass chips, reported cycle times have yet to approach those in achieved by the present invention using capillary sample tubes and it is difficult to achieve temperature homogeneity over the large chip surface exposed to the sample.

Using the present invention, many aspects of DNA amplification which have heretofore been little understood are discernable. For example, product denaturation occurs in less than one second, yet the prior art calls for ten seconds to one minute of denaturation. Observing product melting by real time fluorescence monitoring with double strand dyes in accordance with the present invention (see Figures 12 and 13) shows that use of shorter denaturation times is effective. As another example, many causes of the known "plateau effect" have been proposed, but few data are available to distinguish between alternatives. As shown in Figure 13, product reannealing is very rapid. In fact, during later cycles of amplification, a majority of product is reannealed each cycle during cooling before the primer annealing temperature is reached. This occurs with cooling rates of 5-10°C/seconds carried out by the present invention. Product reannealing with slower, prior art temperature cyclers this undesirable effect is greater. Product reannealing appears to be a major, and perhaps the sole, cause of the "plateau effect."

### Fluorescence Monitoring of Rapid Cycle PCR for Ouantification Using Cycle-by-Cycle Monitoring

In the prior art, the analysis of PCR products is usually performed after amplification is complete. When used for quantitative PCR, such endpoint methods require good estimates of initial template concentration for accurate results. Fluorescence monitoring of a PCR sample for quantitative PCR has been demonstrated with ethidium bromide. The present invention provides the advantage of fluorescence monitoring of a PCR sample at least every cycle. Fluorescence is usually acquired once per cycle during a combined annealing/extension phase. Ethidium bromide fluorescence is enhanced when intercalated into dsDNA and is a measure of product concentration. Monitoring the amount of product once per cycle with dsDNA dyes is an important advance of the present invention that allows a wide dynamic range of initial template concentrations to be analyzed.

In accordance with an aspect of the present invention, it is preferred that double-stranded-DNA-specific dyes be used. For example, SYBR® Green I is a preferred sensitive dye for following PCR product accumulation. In Figure 18, quantification of initial template copy number is possible over a one hundred and eight fold range. In Figure 18, the fluorescence curves are displaced horizontally according to the initial template concentration. As represented in the plot of Figure 18, the sensitivity is limited at low initial template concentrations because amplification specificity is not perfect. When no template is present, undesired products such as primer dimers are eventually amplified. Thus, there is little difference between 0, 1, and 10 average initial template copies.

Although quantification of very low copy numbers has not previously been achieved with dsDNA dyes, the present invention can utilize such dyes, which is advantageous because, inter alia, of the simplicity of using these dyes. The dsDNA dyes can be used for any amplification and custom fluorescently-labeled oligonucleotides are not necessary when using such dyes. Quantification of very low copy numbers with dsDNA dyes requires very good amplification specificity or, as provided by the present invention, means to differentiate the desired product from nonspecific amplification.

Still referring to Figure 18, a fluorescence vs. cycle number plot of DNA amplification monitored with the dsDNA dye SYBR® Green I dye is provided. The plot of Figure 18 was obtain using a 536 base pair fragment of the human β-globin gene which was amplified from 0 to 10' average template copies in the presence of a 1:10,000 dilution of SYBR® Green I. Purified template DNA was obtained by PCR amplification, phenol/chloroform extraction, ethanol precipitation, and ultrafiltration (Centricon 30 available from Amicon of Danvers, Massachusetts) and quantified by absorbance at 260 nm. Each temperature cycle was 28 seconds long (95°C maximum, 61°C minimum, 15 seconds at 72°C, average rate between temperatures 5.2°C/second). All samples were amplified simultaneously and monitored for 100 milliseconds between seconds 5 and 10 of the extension phase. The display was updated each cycle for all samples and forty-five cycles were completed in 21 minutes. The data for each capillary sample tube were normalized to a percentage of the difference between minimum and maximum values for each tube (represented by the Y-axis in Figure 18).

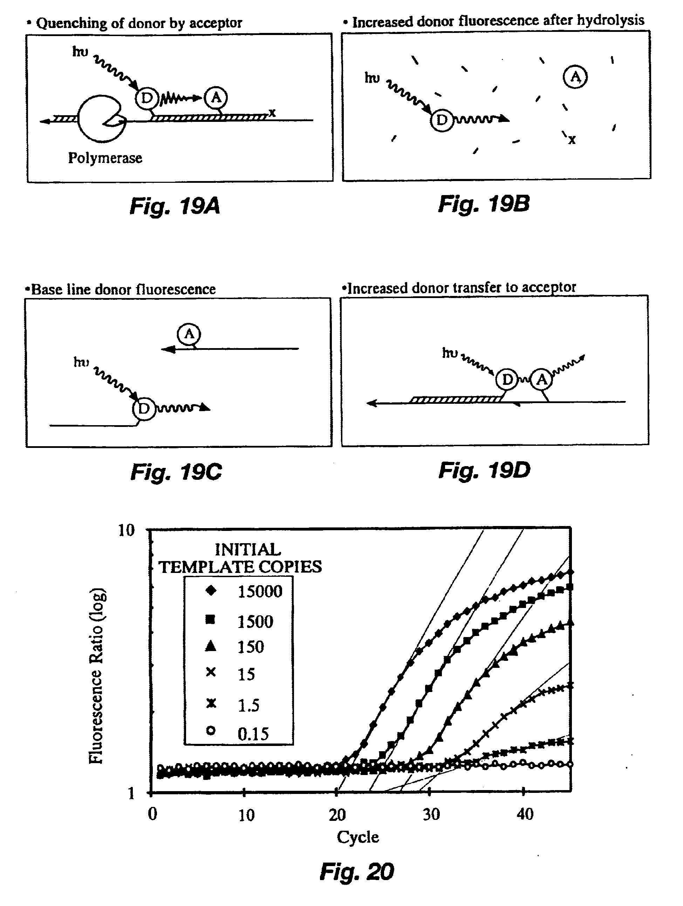

In accordance with another aspect of the present invention, sequence specific fluorescence monitoring is also provided. Sequence specific detection of PCR products is obtained by the present invention by way of including two different fluorophores on oligonucleotide probes. Two arrangements are presently preferred within the present invention as represented in Figures 19A-D. Both arrangements require a donor with an emission spectrum that overlaps the absorbance spectrum of an acceptor.

Figures 19 A-D depict two different preferred arrangements for sequence-specific fluorescence monitoring during PCR. Figure 19A shows a donor (D) which is initially quenched by an acceptor (A) because both are together on a single oligonucleotide probe. After hydrolysis by polymerase 5'-exonuclease activity, the donor and acceptor are separated and the fluorescence of the donor increases (Figure 19B). In the arrangement shown in Figure 19C, the donor is on a probe and the acceptor is on one of the primers. The dyes on the probe and primer are approximated by hybridization. This results in resonance energy transfer to the acceptor, increasing the acceptor fluorescence (Figure 19D).

If the donor and acceptor are synthesized on the same oligonucleotide, the acceptor dye quenches the fluorescence of the donor because of their proximity (Figure 19A). During temperature cycling, some of the probe hybridizes to single stranded PCR product and may be hydrolyzed by polymerase 5'-exonuclease activity. Because the donor and acceptor are no longer linked, the donor is released from acceptor quenching and the donor fluorescence increases. Because the fluorescence signal is dependent on probe hydrolysis instead of hybridization, this class of probes is referred to herein as "hydrolysis" probes.

In accordance with the arrangement depicted in Figures 19C&D, a different fluorescence scheme is possible if the donor and acceptor are placed on different oligonucleotides. In Figures 19C&D, the donor is placed at the 3' end of a probe and one primer is labeled with the acceptor. There is minimal interaction between the dyes because they are on separate oligonucleotides. During temperature cycling, the probe hybridizes near the labeled primer and resonance energy transfer occurs. Because the fluorescence signal is dependent on probe hybridization, this class of probes is referred to herein as "hybridization" probes.

Whether the donor fluorescence is merely quenched or actually increases acceptor fluorescence depends on the specific fluorophores and solvent conditions. An increase in donor fluorescence is usually observed during PCR with hydrolysis probes, whereas an increase in acceptor fluorescence is usually monitored with hybridization probes.

Further information and data on hydrolysis probes will now be provided. In contrast to dsDNA dyes, sequence specific probes can quantify very low initial template numbers as represented by Figure 20. As will be appreciated from the information set forth herein, a single template copy can be distinguished from the absence of template. The efficiency of amplification decreases as the initial copy number drops below 1,500. Significantly, the fluorescence signal continues to increase even after many cycles because hydrolysis is cumulative and not strictly related to product concentration.

Still referring to Figure 20, a fluorescence ratio (fluorescein/rhodamine) vs. cycle number plot of DNA amplification monitored with a dual-labeled hydrolysis probe is plotted. The thin lines indicate the log-linear portion of each curve. A 280 base pair fragment of the human β-actin gene was amplified from 0.15 to 15,000 (average) copies of human genomic DNA per tube. The hydrolysis probe which was utilized (included at 0.2 µM) is available from Perkin Elmer of Foster City, California. Each temperature cycle was 26 sec long (94°C maximum, 60°C for 15 seconds, average rate between temperatures 6.2°C/sec). All samples were amplified simultaneously and monitored for 100 milliseconds between seconds 5 and 10 of the annealing/extension phase. The display was updated each cycle for all tubes and 45 cycles completed in under 20 min.

Further information and data on hybridization probes will now be provided. In contrast to hydrolysis probes, the fluorescence signal from hybridization probes is not cumulative and develops anew during each annealing phase. The fluorescence is a direct measure of product concentration because the hybridization is a pseudo-first order reaction (see Young B. and Anderson M., (1985), Quantitative analysis of solution hybridization, In: Nucleic Acid Hybridization: A Practical Approach, Hames B., Higgins s., eds., pp. 47-71, Washington D.C. IRL Press). Because the concentration of probe is much greater than the product, the fraction of product hybridized to probe is independent of product concentration.

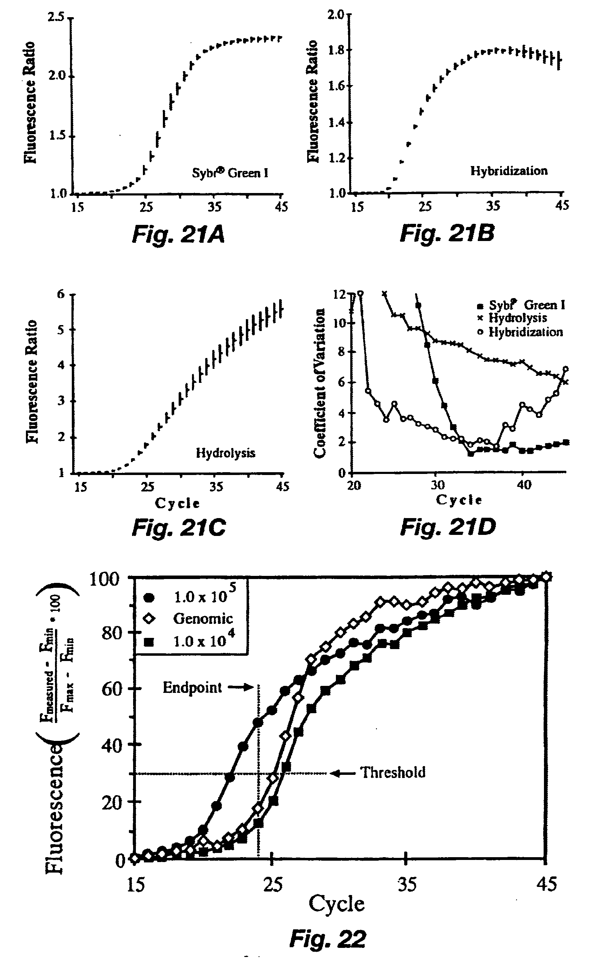

The fluorescence signals from SYBR® Green I, hydrolysis probes, and hybridization probes are directly compared in Figures 21A-D. All probes have nearly the same sensitivity with detectable fluorescence occurring around cycle 20. With extended amplification, the signal continues to increase with the hydrolysis probe, is level with SYBR® Green I, and slightly decreases with the hybridization probe. The decreasing signal after 35 cycles with hybridization probes may be caused by probe hydrolysis from polymerase exonuclease activity. Although the change in fluorescence ratio from the hydrolysis probe is greater than that from the hybridization probe (Figures 21B and 21C), the coefficient of variation of fluorescence from the hydrolysis probes is greater (Figure 21D). That is, the fluorescence resulting from the hybridization probe is more precise than the hydrolysis probe, even though the absolute signal levels are lower.

Figures 21A-D provide comparison of three fluorescence monitoring techniques for PCR. The fluorescence probes are the dsDNA dye SYBR® Green I (Figure 21A), a dual-labeled fluorescein/rhodamine hydrolysis probe (Figure 21B), and a fluorescein-labeled hybridization probe with a Cy5-labeled primer (Figure 21C). All amplifications were performed in ten replicates with 15,000 template copies (50 ng of human genomic DNA/10 *µ*l). The temperature cycles were 31 sec long (94°C maximum, 60°C for 20 sec, average rate between temperatures 6.2°C/sec). Fluorescence was acquired for each sample between seconds 15 and 20 of the annealing/extension phase. The mean +/- standard deviation are plotted for each point. SYBR® Green I was used at a 1:10,000 dilution in the amplification of a 205 base pair human β-globin fragment from primers KM29 and PC04. The hydrolysis probe and conditions are those represented in Figure 20. The hybridization probe, TCTGCCGTTACTGCCCTGTGGGGCAAG-fluorescein (SEQ ID NO:5) (from fluorescein-CPG available from Glen Research of Sterling, Virginia) was used with KM29 and the Cy5-labeled primer CAACTTCATCCACGTNCACC (SEQ ID NO:6) where N was an amino-modifier C6dT (Glen Research) labeled with Cy5 (Mono Reactive available from Amersham of Arlington Heights, Illinois). The precision of the three fluorescence monitoring techniques are compared in Figure 21D. The data are plotted as the coefficient of variation (standard deviation/mean) of the fluorescence ratio above baseline (taken as the average of cycles 11-15).

Algorithms for quantification using cycle-by-cycle monitoring will now be discussed. Although quantitative information about initial template concentrations is contained in fluorescence curve sets such as plotted in Figures 18 and 20, those skilled in the art will not readily understand how to best extract this quantitative information. Referring to Figure 22, therein are shown curves for the amplification of 10⁴ and 10⁵ copies of purified template (taken from Figure 18) compared to the amplification of 50 ng of genomic DNA. Review of the data provided herein informs one of skill in the art that the genomic DNA contains slightly more than 10⁴ copies of the target, but not how many more copies. In accordance with the present invention, the number of copies can be determined.

Referring to Figure 22, plots are provided that compare interpolation methods for quantification of PCR fluorescence curves. Illustrated in Figure 22 is endpoint interpolation which compares the fluorescence of unknowns and standards at a given cycle number. Also illustrated in Figure 22 is threshold interpolation which determines the cycle number at which each curve exceeds a given fluorescence value.

In accordance with the present invention, disclosed herein are two preferred methods for interpolation of the data. One preferred method is to interpolate entire curves, attempting to use all relevant data points. This method of curve fitting approach provides the most accurate results. Another preferred method is to interpolate the unknown along single vertical or horizontal lines. Single vertical interpolation is similar to conventional endpoint analysis. At a certain cycle number, the unknown fluorescence is interpolated between known values. Provided below in Table 1 is a list resulting from vertical interpolation of cycles 20-24. Interpolation along horizontal lines requires establishing a fluorescence threshold. Interpolation from thresholds of 10, 20, 30 and 40% are also listed in Table 1.

**TABLE 1 Single line interpolation methods for fluorescence quantification. (See Figure 22)**

| Endpoint | | Threshold | |
|---|---|---|---|
| Cycle | Copies | Fluorescence(% ) | Copies |
| 20 | 41,000 | --- | --- |
| 21 | 12,000 | 10 | 17,000 |
| 22 | 12,000 | 20 | 14,000 |
| 23 | 13,000 | 30 | 15,000 |
| 24 | 14,000 | 40 | 18,000 |
| Average | 18,000 | Average | 16,000 |

As will be appreciated, endpoint or threshold interpolations do not use much of the available data and can be highly affected by single aberrant data points, e.g. endpoint interpolation at cycle 20. The present invention provides an improved method which involves interpolating curves that fit the data well. For example, an exponential fit for data within the log-linear portion of each curve should follow: F=AN(1+E)", where F is the fluorescence signal, A is a fluorescence scaling factor, N is the starting copy number, E is the amplification efficiency, and n is the number of cycles. A and E could first be determined for values of N that bracket the unknown. Then, the best value of N for the unknown could be determined. Using this approach, the gene copy number in 50 ng of genomic DNA is 1.7 x 10⁴, near the middle of values in Table 1 and close to the accepted value of 1.5 x 10⁴. Other curve fits may be better than the exponential example given above. The exponential fit necessarily relies on only the log-linear cycles (7 out of 45) and these fall in a region where the fluorescence precision is poor (Figure 21D).

Even further, in accordance with the present invention, a sigmoidal curve is used which includes parameters that describe the lag phase, the log-linear phase, and the plateau phase, thus using all the available data. The fit is weighted to reflect the expected precision of the data points. Different parameters are necessary for different fluorescence probes-because of variation in the curve shapes (Figures 21A-D).

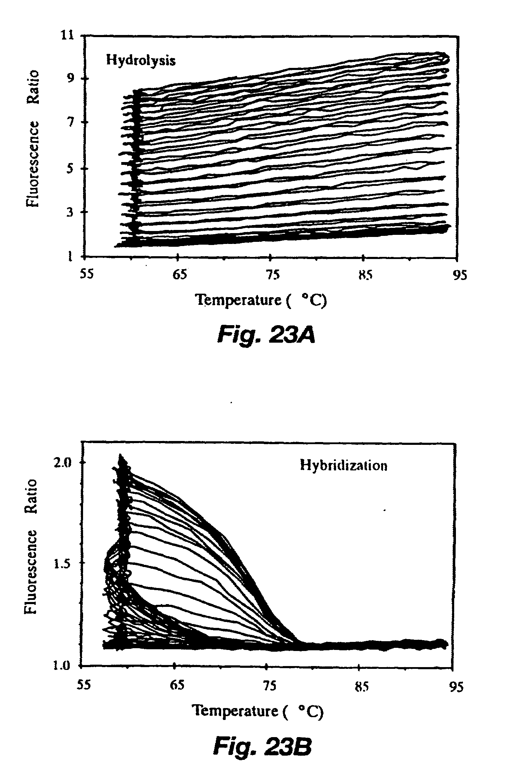

The present invention's feature of continuous monitoring, that is, monitoring many times within each PCR cycle, will now be discussed. While fluorescence monitoring during PCR can be done once each cycle at a constant temperature, the present invention provides the important advantage of providing continuous monitoring throughout the PCR cycle. Temperature is important because fluorescence changes as a function of temperature. However, in accordance with the present invention, monitoring fluorescence during temperature changes is very informative. For example, if fluorescence is acquired continuously throughout temperature cycling, hydrolysis probes show a linear change in fluorescence ratio with temperature and a parallel increase in fluorescence as more probe is hydrolyzed (See Figure 23A). In contrast, the fluorescence ratio from hybridization probes varies radically with temperature (See Figure 23B). During probe hybridization at low temperatures, the ratio increases, followed by a decrease to baseline when the hybridization probe melts off the template.

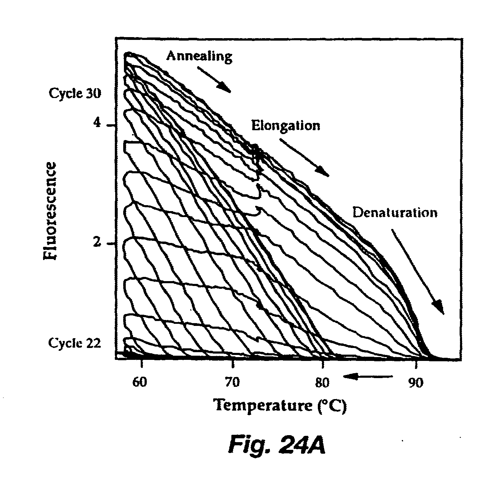

The temperature dependence of product strand status during PCR is revealed by fluorescence vs. temperature plots using SYBR® Green I as shown in Figure 24. As amplification proceeds, temperature cycles appear as rising loops between annealing and denaturation temperatures. As the sample is heated, fluorescence is high until denaturation occurs. As the sample cools, fluorescence increases, reflecting product reannealing. When the temperature is constant during extension, increasing fluorescence correlates with additional DNA synthesis. Figure 24 provides a fluorescence vs. temperature plot of DNA amplification with the dsDNA dye SYBR® Green I. A 536 base pair fragment of the human β-globin gene was amplified from 10⁶ copies of purified template and a 1:30,000 dilution of SYBR® Green I. Other conditions are essentially the same as those described in connection with Figure 18. Cycles 15-25 are displayed. The fluorescence vs. temperature data have been transformed to remove the effect of temperature on fluorescence.

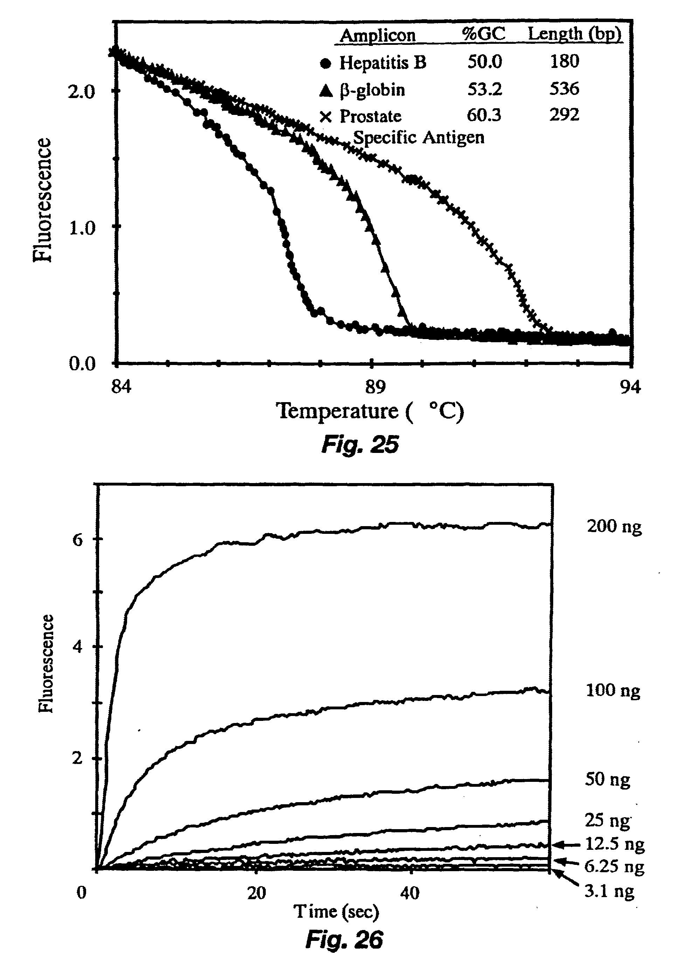

The present invention's ability to monitor product denaturation and product reannealing leads to even additional methods for DNA quantification. These additional methods of the present invention for DNA,quantification require fluorescence monitoring within individual temperature cycles and cannot be used when fluorescence is only acquired once per cycle. For example, PCR products have characteristic melting curves dependent on product GC/AT ratio and length (See Figure 25). Significantly, using empirical algorithms to predict melting temperatures, various PCR products should melt over a 50°C range. Since nonspecific products will melt differently than the desired PCR product, dsDNA fluorescence can be separated into specific and nonspecific components. Using the methods of the present invention allows quantification of very low copy numbers using generic dsDNA dyes like SYBER® Green I or ethidium bromide. Figure 25 shows the melting curves of three purified PCR products. PCR products were purified and quantified as explained in connection with Figure 18. A 1:10,000 dilutions of SYBR® Green I was added to 50 ng of DNA in 10 µℓ of 50 nM Tris, pH 8.3 and 3 mM MgCl₂. The temperature was increased to 0.2°C/second and the fluorescence acquired and plotted against temperature.

The present invention's feature of using melting curves for competitive quantification will now be explained. In accordance with the present invention, an additional use for melting curves is competitive quantitative PCR. These methods of the present invention require a competitive template for coamplification with the native template. Control templates are designed that differ in melting temperature from the natural amplicon. A melting temperature shift of 3°C can be obtained by changing the product GC percentage by 7-8% (see Wetmur, J., (1995), Nucleic acid hybrids, formation and structures of, In: Molecular Biology and Biotechnology: A Comprehensive Desk Reference, Meyers R., ed. pp. 605-608, New York: VCH, which is now incorporated herein by reference). A 3°C shift in the melting temperature is enough to separate the native and competitive components of the product melting curve (see Figure 25). These melting curves, acquired during amplification, are differentiated to melting peaks (see Hillen, W., Goodman T., Benight, A., Wartell R. and Wells, R., (1981), High resolution experimental and theoretical thermal denaturation studies on small overlapping restriction fragments containing the Escherichia coli lactose genetic control region, J. Biol. Chem. 256, 2761-2766, which is now incorporated herein by reference) and used to determine the relative amounts of competitor and template. Using this method should eliminate the need to analyze the sample after temperature cycling.

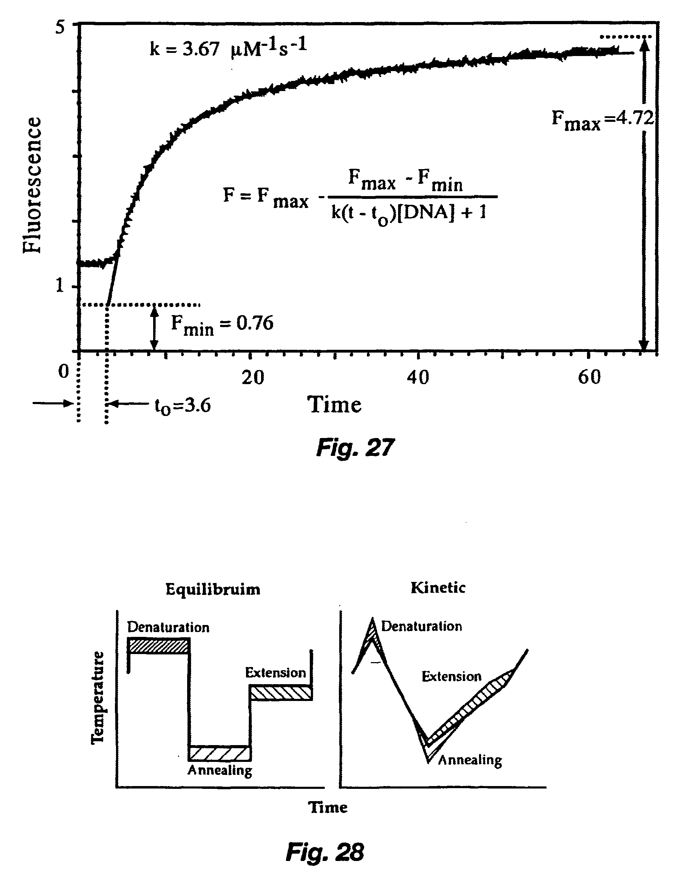

The hybridization kinetics for absolute quantification in accordance with the present invention will now be discussed. The present invention also allows monitoring of product-product reannealing after denaturation and provides a method for direct, absolute DNA quantification. If the sample temperature is quickly dropped from the denaturation temperature and held at a lower temperature, the rate of product reannealing will follow second order kinetics (see Young, B. and Anderson, M., 1985, Quantitive analysis of solution hybridization, In: Nucleic Acid Hybridization: A Practical Approach, Hames, B., Higgins, S. eds., pp. 47-71, Washington, D.C.: IRL Press, which is now incorporated herein by reference). When different concentrations of DNA are tested, the shape of the reannealing curve is characteristic of the DNA concentration (See Figure 26). Figure 26 shows reannealing curves for different concentrations of PCR product. Different amounts of a purified 536 base pair fragment of DNA (see Figure 18) were mixed with a 1:30,000 dilution of SYBR® Green I in 5 µℓ of 50 mM Tris, pH 8.3 and 3mM MgCl₂. The samples were denatured at 94°C and then rapidly cooled to 85°C. For any given PCR product and temperature, a second order rate constant can be measured. Once the rate constant is known, an unknown DNA concentration can be determined from experimental reannealing data. Initial determination of a rate constant is demonstrated in Figure 27. Cooling is not instantaneous, and some reannealing occurs before a constant temperature is reached. Rapid cooling, which can be carried out by the apparatus of the present invention, maximizes the amount of data available for rate constant or DNA concentration determination. Figure 27 shows determination of a second order reannealing rate constant. The curve was fit by non-linear least squares regression with Fₘₐₓ, Fₘᵢₙ, t₀ and k as the floating parameters. With the rate constant (k) determined, DNA concentrations can subsequently be determined on unknown samples.

This method of the present invention requires pure PCR product, but this can be assured by melting curves also obtained during temperature cycling. The present invention's method of quantification by reannealing kinetics is advantageously independent of any signal variation between capillary sample tubes.

### Continuous Monitoring of Rapid Cycle PCR

From the forgoing discussion, it will be appreciated that the present invention achieves several advantages not heretofore available in the art. For example, the present invention provides single-color fluorescence methods to monitor product purity, quantify template, and detect mutations during PCR. The presence of double-stranded DNA (dsDNA) can be followed during PCR with fluorescent dyes. Within each cycle, fluorescence decreases when dsDNA is denatured and increases with product reannealing and primer extension. Although these dyes are not sequence specific, product purity can be assessed by analysis of the shape of DNA melting curves. Pure PCR products have sharp melting transitions, while impurities melt over a broad temperature range.

Competitive quantitative PCR requires differentiation of a natural amplicon from a competitor. Melting curves can be used to distinguish products that have different melting temperatures. Initial template copy number will be quantified by competitive amplification with an artificial control template that differs in melting temperature from the natural amplicon.

Melting curves can also be used for mutation detection during PCR. The heteroduplexes formed during amplification of heterozygous DNA melt at lower temperatures than completely complementary products. The ability to identify deletions and single base mutations will be demonstrated using melting curve analysis.

Continuous monitoring of dsDNA formation is used for direct, absolute DNA quantification. If denatured DNA is rapidly cooled and then held at a constant reannealing temperature, the absolute DNA concentration is determined by the rate of reannealing.

The present invention also provides dual-color fluorescence methods that depend on probe hybridization (not hydrolysis) for sequence-specific detection during PCR. The annealing kinetics and melting of hybridization probes provides information not available with probes that rely on exonuclease hydrolysis between fluorophores. Initial template copy number will be quantified by competitive amplification using probe melting temperature to discriminate between templates. Single base mutations will be detected by probe melting temperature shifts. Determination of absolute product concentration is demonstrated by analysis of probe annealing kinetics.

Furthermore, the present invention provides an inexpensive instrument that can be used in commercial applications and that continuously monitors fluorescence during rapid cycle amplification. The thermal cycler of the present invention is capable of amplification in 10-20 minutes and is coupled to optics modules that detect one, two or three fluorophores. The preferred embodiments of the present invention monitor 24 samples 1 to 10 times/second. In accordance with the present invention, it is preferred that samples are prepared in a 96 well format in composite plastic/capillary vessels and then transferred to the apparatus (see Figure 11) for thermal cycling and analysis. The preferred embodiments include a user friendly graphical interface, which analyzes and displays fluorescence data continuously on a display.

The present invention also uses fluorescence feedback for real time control and optimization of amplification. Fluorescence is used to control temperature cycling. With continuous monitoring of dsDNA-specific dyes, extension will be terminated each cycle after fluorescence stops increasing. Denaturation conditions are automatically controlled by increasing the temperature only until the product is completely melted. Primer annealing is monitored with resonance energy transfer to Cy5-labeled oligonucleotides. Temperature cycling is automatically terminated after a certain amount of product is made.

The advantages of rapid temperature cycling are now recognized by the research community. Fast temperature cycling with minimal annealing and denaturation times improves quantitative PCR and increases the discrimination of allele specific amplification. Rapid cycling for cycle sequencing reduces sequencing ambiguities and minimizes "shadow banding" in dinucleotide repeat amplifications. For long PCR up to 35 kb, yield is improved when the sample is exposed as little as possible to high denaturation temperatures.

From the foregoing, it will be appreciated that an efficient and economical rapid cycler for PCR would be a great advance in the industry. The prior art devices are expensive and cumbersome. For example, one device, the Perkin Elmer 9600, can run 30 cycles of a two temperature profile (60°C, 94°C) in a little over an hour, but the machine is expensive and temperature homogeneity within samples is a problem, with significant intra-sample convection. See wittwer, C.T., G.B. Reed and K.M. Ririe, Rapid cycle DNA amplification, in K. Mullis, F. Ferre, and R. Gibbs (Eds.), The Polymerase Chain Reaction, Springer-Verlag, Deerfield Beach, Florida, pp. 174-181, 1994 and Haff L., J.G. Atwood, J. DiCesare, E. Katz, E. Picozza, J.F. Williams, T. Woudenberg, A high-performance system for automation of the polymerase chain reaction, BioTechniques 10:102-112, 1991, which are both now incorporated herein by reference. Another example is a device developed by Kodak which uses a closed vessel system for PCR amplification and detection that utilizes a "PCR pouch" of heat sealed plastic. In the Kodak device the sample is temperature cycled as a sandwich between two heating/cooling plates. The time for 30 cycles of a two temperature profile (62°C, 94°C) is approximately 30 min. Both of these devices inherently present significant drawbacks and disadvantages.

In accordance with the present invention, a kinetic paradigm for PCR is appropriate. Instead of thinking about PCR as three reactions (denaturation, annealing, extension) occurring at three different temperatures, a kinetic paradigm for PCR may be more appropriate (Figure 28). With a kinetic paradigm, the temperature vs. time curve consists of continuous transitions between overlapping reactions. A complete analysis would require knowledge of all relevant rate constants over all temperatures. If rate constants of all reactions were known, a "physicochemical description of PCR" could be developed. Determining these rates would require precise sample temperature control and would be aided by reaction monitoring during temperature cycling.

The present invention also provides the advantages that accrue from continuous monitoring of a sample. Providing fluorescence monitoring every cycle for quantitative PCR provides benefits and works well with ethidium bromide. In once-per-cycle monitoring, fluorescence is acquired once per cycle during a combined annealing/extension phase and gives a relative measure of product concentration.

In accordance with the present invention, time, temperature and dsDNA fluorescence are acquired every 200 msec and fine details of product denaturation and reannealing can be observed during rapid temperature cycling. These details allow product identification by melting curves, and provide means for relative and absolute product quantification, mutation detection, and immediate feedback for temperature control. In addition with continuous monitoring, the melting of sequence specific probes can be determined by resonance energy transfer. Probe melting occurs at a characteristic temperature that can also be exploited for product quantification and single base mutation detection.

The application of the present invention to quantitative PCR will now be discussed. Prior art methods for quantitative PCR are labor intensive and expensive. Most prior art methods use endpoint analysis and require good initial estimates for accurate results. Monitoring the fluorescence of dsDNA once per cycle with dyes is an important advance that allows a wide dynamic range of initial template concentrations to be analyzed. However, the present invention's continuous monitoring within temperature cycles allows verification of product purity, simultaneous relative quantification with a competitor, and absolute product concentration determination. All this information can be obtained from one capillary sample tube with a generic dsDNA indicator within ten to twenty minutes of temperature cycling. Quantification based on internal sequence specificity is also possible using two color analysis and resonance energy transfer.

The application of the present invention for mutation detection will now be discussed. Screening for mutations in genes is a difficult task. Prior art methods (denaturing gradient gel electrophoresis, temperature gradient gel electrophoresis, single stranded conformational polymorphism, and sequencing) require PCR amplification followed by electrophoresis and subsequent detection. Melting curves with dsDNA dyes can identify heterozygous mutations that produce heteroduplexes during PCR. Analysis occurs during amplification without any need for extra sample handling or equipment. For example, detecting heterozygotes is important in assessing genetic susceptibility to breast cancer. See Shattuck-Eidens D., M. McClure, J. Simard, F. Labrie, S. Narod, F. Couch, D. Hoskins, B. Wever, L. Castilla, M. Erdos, L. Brody, L. Friedman, E. Ostermeyer, C. Szabo, M.C. King, S. Jhanwar, K. Offit, L. Norton, T. Gelewski, M. Lubin, M. Osborne, D. Black, M. Boyd, S. Steel, S. Ingles, R. Haile, A. Lindblom, H. Olsson, A. Borg, D.T. Bishop, B. Solomon, P. Radice, G. Spatti, S. Gayther, B. Ponder, W. Warren, M. Stratton, Q. Liu, F. Fujimura, C. Lewis, M.H. Skolnick, D.E. Goldgar, A collaborative survey of 80 mutations in the BRCA1 breast and ovarian cancer susceptibility gene, Implications for presymptomatic testing and screening, J. Amer. Med. Assoc. 273:535-541, 1995. Mutation detection by sequencing the BRCA1 and BRCA2 genes (>10,000 bases) may not be feasible as a clinical test. Test costs could be significantly lowered using heteroduplex screening methods in accordance with the present invention. Specific mutations can also be detected with sequence specific probes through resonance energy transfer during PCR.

Further discussion of the probes that can be advantageously utilized in accordance with the present invention will now be discussed. The best probes for continuous monitoring of PCR would be specific and inexpensive. Double-stranded DNA (dsDNA) dyes can be used in any amplification and are inexpensive. For example, SYBR® Green I generally costs 0.01 cents per 10 *µ*l reaction. In addition, product specificity can be obtained by analysis of melting curves. However, when sequence specificity is necessary, fluorescent oligonucleotide probes must be synthesized for each sequence under study. The "TaqMan" probes promoted by Perkin Elmer incorporate 2 fluorophores into each probe. Fluorescence is generated when the probe is hydrolyzed by polymerase exonuclease activity. These probes are difficult to make and expensive to buy; their current cost from the manufacturer is $1,200 for 0.1 *µ*mol of a custom probe. Two probes are necessary for competitive quantification of a target. In contrast, fluorescent hybridization probes that are not dependent on hydrolysis can be designed. These hybridization probes require only a single fluorophore per probe and are much easier to synthesize. Because their fluorescence results from hybridization, not hydrolysis, the temperature dependence of probe fluorescence can be used for mutation detection and quantification.

The previously available instrumentation used for detection presented many drawbacks. In the past, only endpoint detection was possible for the hydrolysis probes because of instrument limitations. Rapid, precise temperature cycling provided by the system of the present invention described herein. With the continuous fluorescence monitoring also provided by the system of the present invention, the temperature dependence of hybridization can be followed. By following hybridization during temperature cycling, the number of probes and/or spectral colors required can be minimized. In order to provide an embodiment of the present invention that is most cost effective, a high intensity light emitting diode is used instead of a laser for sample illumination, and photodiodes are used for detection. Samples are loaded into glass capillary sample tubes, or alternatively into composite glass/plastic sample tubes in a 96-well format that does not require heat sealing. The present invention thus provides real time fluorescence analysis in a cost effective manner. Real time fluorescence control of temperature cycling improves amplification quality. For example, if the temperature of samples is increased only until denaturation occurs, product exposure to high temperatures is minimized. This increases yield by limiting product and enzyme degradation and increases specificity by limiting amplification of products with a high melting temperature.

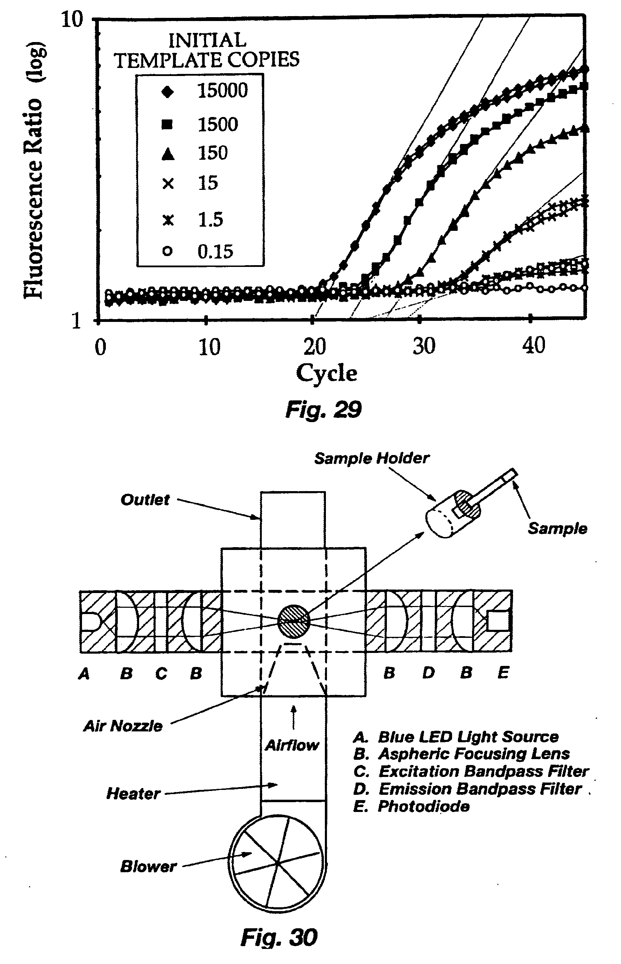

Figure 29 shows that monitoring over a 10-fold dynamic range in the log-linear portion of a routine amplification has been fully accomplished. Furthermore, it has been accomplished not only for routine amplifications (15,000 copies of genomic DNA/ sample tube), but also for more difficult amplifications with smaller amounts of template.

As shown in Figure 29, the fluorescence ratio increases with cycle number and with initial template concentration. The log-linear portions of each curve (straight, thin lines) span 4 cycles (about 24, or a 16-fold range in DNA concentration). Duplicate samples track very closely. Figure 29 shows that a single copy of template can be distinguished from no template (presumably, one of the 0.15 average copy samples included a single copy). At high initial template concentrations (15,000 and 1,500/sample tube) amplification efficiency is high and appears constant, but efficiency is reduced in samples with lower initial concentrations. All samples were simultaneously amplified and analyzed with quantitative display of the data each cycle. The forty-five cycle amplification was advantageously completed in under 20 minutes.

Reference will next be made to Figure 30, which shows an embodiment of the present invention configured for continuous monitoring of a single sample. In performing continuous monitoring, a single sample was placed at the intersection of a temperature-controlled air stream and a linear optical path. One side of the capillary was illuminated with a light emitting diode, and the other side was observed with a photodiode. Air was constantly forced over the sample and the temperature was controlled by an upstream heating coil. The geometry and orientation of the sample and container were studied with human genomic DNA stained with the dsDNA dye, SYBR® Green I.

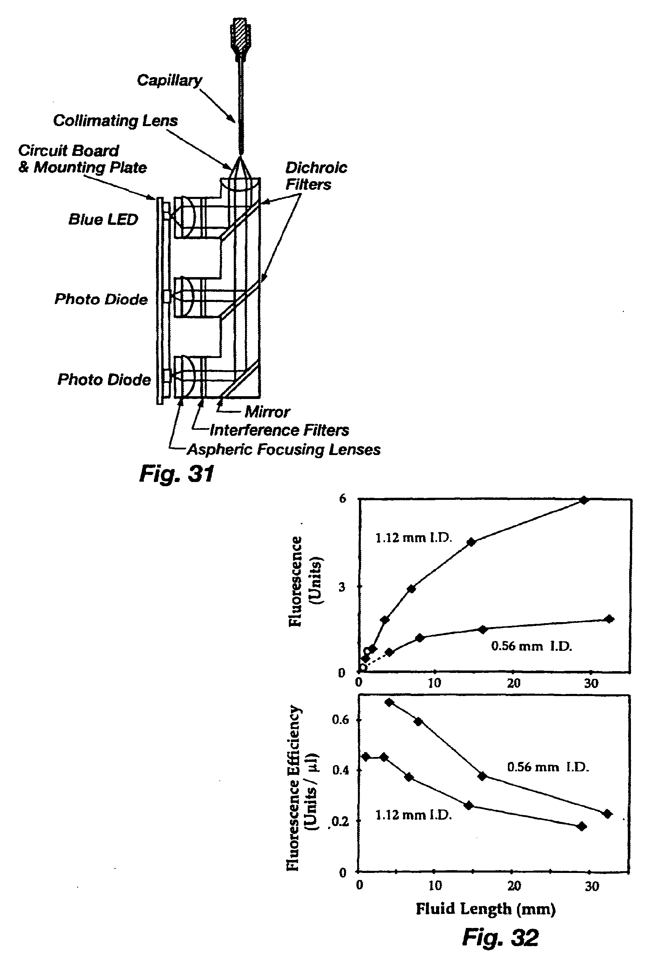

Figure 31 shows the optical layout of another embodiment in accordance with the present invention to provide continuous monitoring of a PCR sample. Total internal reflection ("light piping") along the length of the capillary sample tube was exploited to increase both excitation and emission yields. The optical path has been changed from linear, to epifluorescent, that is, both the excitation and emission light follow the same optical path. For maximal light piping, the optical axis is parallel to the length of the capillary (paraxial) with the capillary tip as the focal point. Assuming a refractive index of 1.33 for the sample, 12.3% of emitted light should be guided to the tip. Figure 32 displays charts showing the effectiveness of light piping as a 10-fold increase in signal intensity by viewing the tip (closed diamonds) rather than the side (open circles) of capillaries. As indicated in Figure 32, two different size capillary sample tubes are preferred which were filled to different lengths with dsDNA stained with SYBR® Green I. The observed fluorescence increases as more sample is added to the tube, although the fluorescence efficiency decreases.

Referring again to Figure 11, a rapid temperature cycler with fluorescence detection 300 can be used for continuous monitoring as will now be further explained.

The rapid temperature cycler with fluorescence detection 300 can advantageously be constructed using light emitting diodes (LEDs) and photodiodes in place of similarly functioning components represented in Figure 11. Thus, the fluorescence excitation source 328 can be carried out by light emitting diodes. The photomultiplier tubes 362A&B can also be replaced with photodiodes. Technique sensitivity is limited by background fluorescence, most of which comes from the probes, not the detection system. Stability is more important than absolute sensitivity.

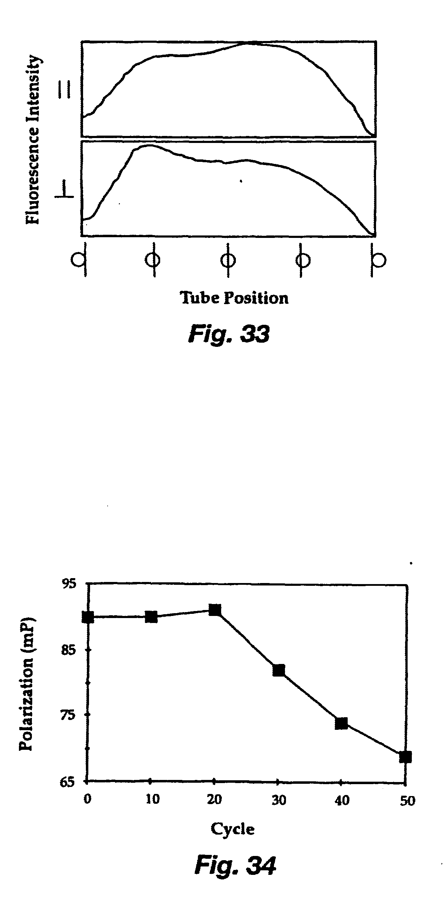

The present invention also overcomes the challenges which arise due to polarization effects when using capillary sample tubes. Observing the fluorescence polarization of a sample requires that the optical system not randomize polarized light. Epifluorescence monitoring from the tip of capillaries will randomize any polarization because of multiple reflections. When round glass capillaries are illuminated with polarized light from the side, the observed polarization depends on the precise positioning of the tube with respect to the excitation beam as set forth in Figure 33.

In accordance with the present invention, fluorescence polarization probes can be used. Polarization probes were synthesized as 30-mers with a 5'-fluorescein connected by aminolinkers of different lengths. During PCR, the 5'-exonuclease activity of the polymerase was expected to cleave the probe and decrease its polarization during amplification. The intact probes had a polarization of about 90 millipolarization units (mP) that was decreased to 35 mP when extensively hydrolyzed with phosphodiesterase I. Figure 34 shows the results obtained using one probe that was included at 0.2 µM in the amplification of a single copy gene with genomic DNA as template. Polarization decreased between 20 and 30 cycles and continued to decrease at least to 50 cycles. The decrease, expressed as a ratio of parallel to perpendicular fluorescence was about 11%. The length of the linker, the type of aminolinker and the presence/absence of a 5'-noncomplementary tail did not significantly alter the observed changes during PCR. Adding sucrose to increase the viscosity of the solution increased the polarization of both intact and hydrolyzed probes, but did not change the signal difference generated after amplification.

Instead of decreasing polarization by exonuclease hydrolysis, hybridization without hydrolysis increases polarization. In accordance with the present invention, a 15-mer peptide nucleic acid labeled with fluorescein was used. Because of its amide backbone, it cannot be hydrolyzed by exonuclease activity. When hybridized to a 10-fold excess of purified complementary single-stranded PCR product, its polarization only increased from 85 to 97 mP.

In summary, fluorescein-labeled probes do show polarization changes when hybridized or hydrolyzed. These changes can be used to monitor PCR, but the changes are small. The present invention can be used in a method for increasing the small polarization signal of hybridization for strand displacement amplification. A genetically altered form of the endonuclease EcoRI that lacks cleavage activity but retains binding specificity was used to increase the polarization of the complex.

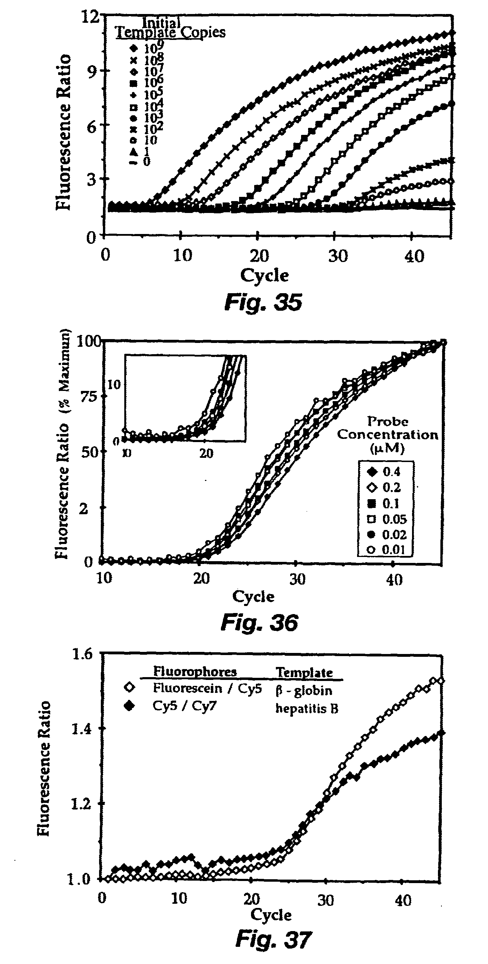

Resonance energy transfer probes can also be used in accordance with the present invention. Resonance energy transfer probes are superior to polarization probes for PCR monitoring because of greater signal intensity. Prior art uses of resonance energy transfer in PCR have been limited to only endpoint analysis. One probe design depends on exonuclease hydrolysis for signal generation. Dual-labeled fluorescein/rhodamine probes are cleaved during polymerase extension by 5'-exonuclease activity, separating the fluorophores and increasing the fluorescein/rhodamine emission ratio. Figure 35 shows fluorescence PCR results from a probe with five intervening bases between fluorescein and rhodamine labels. The forty-five cycle amplification was completed in 20 minutes using the rapid temperature cycler with fluorescence detection 300 of Figure 11. By monitoring the fluorescence ratio once per cycle, a 10⁹ fold range of initial template concentration could be distinguished. The amplification curves are shifted approximately 3-4 cycles for each 10-fold change in initial template concentration. Although efficiency decreases when the initial template concentration is low, a single copy can be distinguished from no template.

The signal generated by monitoring hydrolysis is cumulative and only indirectly related to product concentration. Hence, as shown by Figure 35, the fluorescence ratio continues to increase even after the amount of product has reached a plateau. Nevertheless, the fluorescence signal correlated well with the amount of PCR product as measured by ³²P-dATP incorporation, at least up to the plateau phase.

The concentration of the probe has very little effect on sensitivity as shown in Figure 36. changing the probe concentration by a factor of 40 only shifts the curve by about 2 cycles. Lower probe concentrations are slightly more sensitive, but are also more variable because of lower signal strengths. The data in Figure 36 are normalized to the maximum signal at each probe concentration. Although decreasing the probe concentration would give a greater signal for the same amount of hydrolyzed probe, this advantage is offset by lower hybridization rates. The rate of probe hybridization to target depends directly on the probe concentration.

The present invention provides that many different hydrolysis probes can be used, including those labeled with fluorescein and either rhodamine, eosin or BODIPY dyes (available from Molecular Probes). In general, as the spacing between fluorophores decreases, quenching and/or energy transfer increases, but hydrolysis during PCR decreases. Resonance energy transfer probes that require hydrolysis have some characteristics which must be noted. One characteristic is that some probes are resistant to hydrolysis. Another characteristic is that synthesis is difficult, requiring manual addition of at least one label and high pressure liquid chromatography for purification.

Cy5 is a red dye that recently became available as an amidite for direct incorporation into oligonucleotides. Working in the red/infrared region of the spectrum is advantageous when choosing optical compoments. Laser diodes can be used for illumination, photodiode detectors have excellent sensitivity, and most materials have minimal autofluorescence in the pertinent spectral region. A Cy5/Cy7 probe has been developed in connection with the present invention. Amplification was detectable with this probe, although the signal was weak. When the probe was completely hydrolyzed with phosphodiesterase, only a 2-fold increase in signal was observed. Between cycles 20 and 40 of PCR, a 1.3-fold increase in signal occurred as represented in Figure 37. Synthesis of the Cy5/Cy7 probe required manual addition of the Cy7.

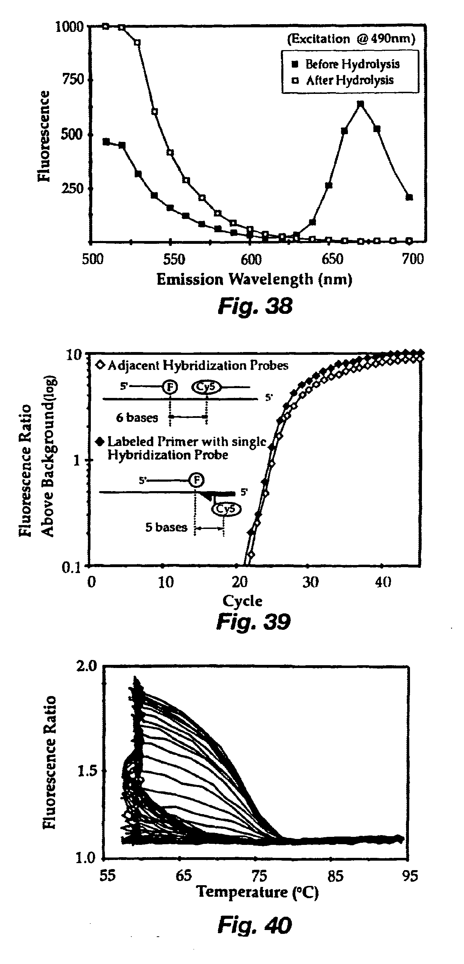

Dual-labeled, fluorescein and Cy5 probes can be easily made on automated oligonucleotide synthesizers. These fluorescein/Cy5 probes show excellent fluorescence ratio changes when completely hydrolyzed with phosphodiesterase. The emission wavelengths of fluorescein and Cy5 are well separated, and efficient resonance energy transfer occurs between the fluorophores as shown in Figure 38. Although the spectral overlap is small, the molar absorption coefficient and absorption wavelengths of Cy5 are high. Spectral overlap, acceptor absorptivity, and acceptor wavelength all contribute to the overlap integral that determines energy transfer rates. Because Cy5 has low absorbance at fluorescein excitation wavelengths, direct excitation of Cy5 is also minimal.

The fluorescein/Cy5 probes were not hydrolyzed efficiently during PCR. Even though a 200-fold change in fluorescence ratio is possible with complete hydrolysis, the preferred fluorescein/Cy5 probe gave only a 1.45-fold increase between cycles 20 and 40 of PCR (see Figure 37). Several fluorescein/Cy5 probes were synthesized with different spacing between fluorophores and with either fluorescein or Cy5 at the 5' end. All gave low signals with PCR. Some of our results with hydrolysis probes are summarized below:

**Fluorescence Signal Generation with Exonuclease Hydrolysis Probes**

| Fluorophores | | Increase in Fluorescence Ratio with: | |
|---|---|---|---|
| | | Complete Hydrolysis | Forty Cycles of PCR |
| Fluorescein | Rhodamine | 25-fold | 8-fold |
| Fluorescein | Cy5 | 200 | 1.45 |
| Cy5 | Cy7 | 2 | 1.3 |

The resistance of dual-labeled fluorescein/Cy5 oligonucleotides to hydrolysis was turned to an advantage by developing resonance energy transfer schemes that depend on probe hybridization instead of probe hydrolysis. Instead of hydrolyzing a dual-labeled probe, hybridization can be detected directly by resonance energy transfer during PCR. In contrast to dual-labeled exonuclease probes, the synthesis of single-labeled probes is relatively simple.

The present invention's feasibility using two different methods for resonance energy transfer detection of hybridization during PCR is shown in Figure 39. The first method uses two adjacent hybridization probes, one labeled 3' with fluorescein and the other labeled 5' with Cy5. As product accumulates during PCR, the probes hybridize next to each other during the annealing segment of each cycle. The second method uses a primer labeled with Cy5 and a single hybridization probe. The labeled primer is incorporated into the PCR product during amplification and only a single hybridization is necessary. In both methods, fluorescence energy transfer to Cy5 increases with hybridization and is plotted as a ratio of Cy5 to fluorescein fluorescence. The fluorescence is monitored once each cycle near the end of a 2-temperature annealing/extension segment. The sensitivity of these hybridization probes appears similar to hydrolysis probes (compare Figures 29 and 39).

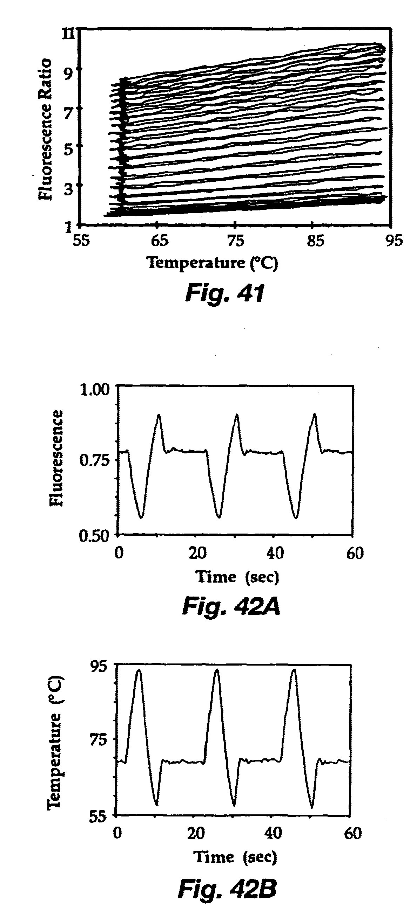

The temperature dependence of the fluorescence from hybridization probes is best demonstrated with fluorescence vs. temperature plots as shown in Figure 40. These plots are generated by monitoring a single sample every 200 msec of temperature cycling. During the annealing/extension phase, the probes hybridize to single stranded product and the fluorescence ratio (Cy5/fluorescein) increases. During heating to product denaturation temperatures, the probes dissociate around 70-75°C, returning the fluorescence ratio to background levels. In contrast, hydrolysis probes do not show this temperature dependence (see Figure 41). As expected, fluorescence vs. temperature plots with hydrolysis probes show only the linear dependence of fluorescence with temperature. The ability to monitor hybridization with fluorescence during temperature cycling is a powerful tool. The melting temperature of sequence-specific probes can identify and discriminate products during PCR.

Double strand specific dyes can also be used in accordance with various aspects of the present invention. The strand status of PCR products can be followed with dyes that fluoresce in the presence of dsDNA. When SYBR® Green I is present during amplification, fluorescence increases as more dsDNA is made. However, temperature cycling introduces a confounding effect because fluorescence is inversely proportional to temperature as shown in Figures 42A and 42B. When fluorescence is continuously monitored as a function of temperature and time during PCR, the data form a three dimensional spiral represented in Figure 12. As discussed earlier, the 3D curve represented in Figure 12 can be projected onto 2D plots of temperature vs. time, fluorescence vs. time, and fluorescence vs. temperature. The temperature vs. time projection of Figure 12 repeats each cycle; its familiar form is more clearly shown in the bottom of Figure 42B. The fluorescence vs. time projection of Figure 12 is a scaled mirror image of the temperature vs. time plot at early cycles shown in Figure 42B. As product accumulates, the fluorescence increases except at denaturation temperatures, where the fluorescence returns to baseline as shown in Figure 12.

Fluorescence vs. temperature projections show the temperature dependence of strand status during PCR (see Figure 24 and Figure 24A). As amplification proceeds, temperature cycles appear as rising loops between annealing and denaturation temperatures. As the sample is heated, fluorescence is high until denaturation occurs. As the sample cools, fluorescence from dsDNA formation increases, reflecting product reannealing. When the temperature is constant during extension, increasing fluorescence correlates with additional DNA synthesis. The fluorescence vs. temperature data can be transformed to remove the effect of temperature on fluorescence (see Figure 24). In accordance with the present invention, product Tₘ and melting curves are used to assess amplification specificity. In many cases, this method eliminates the need for synthesizing a hybridization probe. Discrimination based on hybridization temperatures is a powerful tool for identification and quantification, and can be used in conjunction with or instead of spectral discrimination by multicolor analysis.

Summary of probes for continuous monitoring of PCR. The following table compares dsDNA dyes. hydrolysis probes, and hybridization probes useful in continuous monitoring of PCR. The fluorescence of dsDNA dyes depends on the strand status of the DNA. The dual-labeled hydrolysis probes are quenched while intact. The fluorescence of the quenched fluorophore increases when the probe is hydrolyzed. Hybridization probes depend on increased resonance energy transfer when hybridization brings 2 fluorophores closer together.

**Summary of Fluorescent Probes for Continuous Monitoring of PCR**

| | dsDNA dye | Fluorescent Probe | |
|---|---|---|---|
| | | Hydrolysis | Hybridization |
| Mechanism | Strand status | Quenching | Transfer |
| Probe Synthesis | Unnecessary | Difficult | Simple |
| specificity | Product Tₘ | Sequence | Sequence |
| Melting Analysis | Yes | No | Yes |
| Multicolor Analysis | No | Yes | Yes |

Those versed in the art will appreciate that the rapid temperature cycler with fluorescence detection 300 represented in Figure 11 includes the beneficial characteristics of a fluorimetry device with rapid temperature control, a combination nowhere suggested or taught in the art. PCR can be performed and analyzed during ten to twenty minutes of temperature cycling. The present invention's combination of 1) continuous fluorescence monitoring within each temperature cycle and 2) analysis of the temperature and time dependence of hybridization provides advantages not otherwise obtainable.

The present invention also provides single-color fluorescence methods to monitor product purity, quantify template, and detect mutations during PCR. Dyes that monitor DNA strand status are added to PCR reactions for observation during temperature cycling. SYBR® Green I is a sensitive dye that is highly fluorescent only when complexed with double stranded DNA. As a PCR reaction is heated from extension to denaturation temperatures, denaturation can be observed as a drop in the fluorescence of SYBR Green I. The melting curve is characteristic of the product being denatured. For small PCR products, the melting transition occurs over a narrow temperature range; the midpoint of melting is referred to as the Tₘ. The fluorescence melting curves of three different purified PCR products are shown in Figure 43A. The data of Figure 43A were obtained by fluorescence monitoring with SYBR® Green I while increasing the temperature at a rate of 0.2°C/sec. The relative melting positions can be predicted from empirical equations relating GC content and length to Tₘ. The apparent Tₘ of PCR products is dependent on the heating rate such as shown in Figure 43. As the heating rate decreases, the melting curve shifts to lower temperatures and becomes sharper. For maximum detail of melting curves and accurate estimates of Tₘ, the kinetic effects of heating rate need to minimized. The apparent Tₘ of PCR products is also dependent on the dsDNA dye concentration as shown in Figure 44. Higher concentrations of dye increase the stability of the duplex and the observed Tₘ.

The characteristics of different dsDNA dyes will be discussed, first, to select an optimal dye for melting curves during PCR, and then a discussion of the melting curves to assess PCR product purity and to detect mutations during amplification will be provided. Finally, the use of dsDNA dyes in competitive quantitative PCR and absolute product quantification will be explained.

Different dsDNA dyes will now be further discussed. There are many double strand-specific dyes which can be used in accordance with the present invention. One preferred dye is SYBR® Green I which is more preferred over the more common ethidium bromide because its excitation spectrum is similar to fluorescein. A variety of double strand dyes were tested for: 1) inhibition of PCR, 2) detection sensitivity, 3) heating rate effects on the melting curve, and 4) dye concentration effects on the melting curve. First, the highest concentration of dye compatible with rapid cycle PCR was determined. This concentration can be used to assess detection sensitivity by continuous monitoring during PCR with optimal optical filters. The effect of transition rates and dye concentration on melting curves are dye specific. Data will be acquired and presented as in Figures 43 and 44. The dyes listed below will be compared to SYBR Green I:

**Alternate dsDNA dyes for melting curves (See Fu Y-H, DPA Kuhl, A Pizzute, M Pieretti, JS Sutcliffe, S Richards, AJMH Verkerk, JJA Holden, RG Fenwick, ST Warren, BA Oostra, DL Nelson, and CT Caskey, Variation of the CGG repeat at the fragile X site results in genetic instability: resolution of the Sherman paradox, Cell 67:1047-1058, 1991, which is now incorporated herein by reference.)**

| Dye | Excitation | Emission | Comment |
|---|---|---|---|
| Pico Green | 490 nm | 520 nm | Promoted for quantificatio n |
| Ethidium Bromide | 520 | 590 | Inexpensive, common intercalater |
| Acridine Orange | 490 | 550 | Single strand emission at 620 nm |
| Thiazole Orange | 510 | 540 | High fluorescence on binding DNA |
| YO-PRO-1 | 490 | 510 | AT preference |
| Chromomycin A3 | 440 | 550 | GC preference |

For SYBR Green I, it is preferred to use dilutions of 1:7,000-1:30,000 with heating rates of 0.1-0.5°C/second. Melting curves of purified PCR products with high GC content and low GC content are obtained for each dye. Melting curves with different dyes may vary depending on their AT/GC preference and mode of binding. Dyes may redistribute during the melting of different DNA domains, as is known for ethidium bromide. In accordance with the present invention, the best dye, its concentration, and the heating rate required for melting curves can be determined.

Product purity will now be discussed. Because of heterogeneity in size and GC content, PCR products can melt over a 40-50°C range. For example, primer dimers should melt at low temperatures, heterogeneous nonspecific products should melt over a large range, and pure PCR product should melt sharply at a specific Tₘ. These characteristics are demonstrated herein by comparing fluorescent melting curves to agarose gel electrophoresis of PCR amplifications that have been optimized to give the desired product(s). Long PCR products may have internal melting domains that could be used as a fingerprint for identification, but smaller DNA fragments (<300 bp) usually melt in one transition. Depending on sequence, DNA melting domains may vary from 40 to 600 base pairs.

Mutation detection should also be considered in accordance with the present invention. The ability of melting curves to detect genetic polymorphism and mutations will be determined. Repeat polymorphisms (VNTRs, dinucleotide repeats) vary in size but not significantly in sequence. Homozygous point mutations (base substitutions) in DNA fragments can be detected by denaturing gradient gel electrophoresis and temperature gradient gel electrophoresis, but the temperature shift is usually < 1°C, a difference which can only be detected using precision techniques. However, when the DNA target in PCR is heterozygous, heteroduplexes are formed during cooling, and the observed temperature shifts are 1-5°C. A majority of heterozygous mutations can be detected by simple melting curves during PCR using the present invention. The region surrounding the 3-base pair deletion site of the most common cystic fibrosis mutation (ΔP₅₀₂) is amplified from previously typed DNA in accordance with the present invention. Melting curves from homozygous wild type, heterozygous ΔF_{502S}, and homozygous ΔF₅₀₂ is also compared herein. The homozygous samples will be nearly indistinguishable but the heterozygote curve shows a low T, heteroduplex melt followed by a normal "homoduplex" melt. The effect is more pronounced with smaller amplicons.

The most common known genetic risk factor for thrombosis is a single point mutation in the factor V gene. Previously typed homozygous and heterozygous DNA surrounding the mutation is amplified and melting curves obtained. Homozygous mutations will be detected by coamplification with an equal amount of wild type DNA. The observed mixed melting curve should appear identical to that from a heterozyote amplification. If wild type DNA is routinely mixed with test samples in a 1:2 ratio, heterozygotes should show a 2:1 wild type:mutant melt and a homozygous mutant would be 1:2 wild type:mutant.

BRCA1 is the first breast cancer susceptibility gene identified, accounting for about 5% of breast cancers. Most mutations are substitutions, insertions or deletions of only one or two bases and susceptibility is conferred by being heterozygous for a mutation. Heterozygotes can be detected by automated sequencing, but a clinical test that sequences over 5,000 coding bases is expensive and difficult using prior art apparatus and methods. Ten known BRCA1 mutations are tested using the present invention by amplifying the affected amplicons, observing melting curves, and comparing to wild type. The primers and known DNA samples are available in the art from Myriad Diagnostics.

Competitive quantitative PCR is also obtained using the present invention. The ability to detect differences in product melting temperature is exploited by the present invention in quantitative PCR to differentiate between a PCR product and a competitor. Initial template copy number will be quantified by competitive amplification with a control template that uses the same primers, but differs in melting temperature from the natural amplicon. The benefits of this approach is demonstrated in Figures 45 and 46. Figure 45 shows the melting curve obtained when two purified PCR products differing in Tₘ by about 2°C are mixed. By correcting for the effect of temperature on fluorescence and plotting the derivative of the fluorescence over temperature, the relative amount of each PCR product can be quantified as shown in Figure 46.

In accordance with the present invention, a competitive template is generated for amplification of a 180 base pair fragment of the hepatitis B surface antigen gene from primers previously described. A competitive template with a 3°C difference in Tₘ can be obtained by either changing the template length, GC content, or a combination of both. By PCR amplification of smaller hepatitis B fragments and subsequent ligation through terminal overlaps the following templates were synthesized:

**Competitive Hepatitis B PCR Templates with a 3°C Tm Difference from Wild Type**

| Template | GC Content | Length | Estimated Tₘ shift (°C) |
|---|---|---|---|
| Wild Type | 50.0 | 180 | 0 |
| Alternate #1 | 50.0 | 87 | -3 |
| Alternate #2 | 57.3 | 180 | +3 |
| Alternate #3 | 42.7 | 180 | -3 |

The alternate templates were quantified by their A₂₆₀ and amplification efficiencies determined by monitoring with SYBR® Green I once each cycle. Competitive quantification of wild type DNA by all three alternative templates will be compared herein to a standard clinical method using hybridization.

Absolute quantification of product is also advantageously carried out using the present invention. Continuous monitoring of double stranded DNA formation allows direct, absolute DNA quantification by reannealing kinetics. The sample temperature is quickly dropped from the denaturation temperature and held constant at a lower temperature that is still high enough to prevent primer annealing. The rate of product reannealing then follows second order kinetics. When different concentrations of DNA are tested, the shape of the reannealing curve is characteristic of the DNA concentration (see Figure 26). For any given PCR product and temperature, a second order rate constant can be measured. Once the rate constant is known, any unknown DNA concentration can be determined from experimental reannealing data. The principle is demonstrated in Figure 27. The curves can be fit by non-linear least squares regression during temperature cycling in real time using the LabView programming environment explained previously. Cooling is not instantaneous, and some reannealing occurs before a constant temperature is reached, but regression analysis allow for this in accordance with the present invention (see Figure 27). The technique requires pure PCR product, but such can be assured by melting curves also obtained during temperature cycling. Quantification by reannealing kinetics is independent of signal level and not affected by minor sample differences.

The assumption of second order kinetics for product reannealing is confirmed by determining the rate constant at different DNA concentrations. Some fluorescent dyes affect reannealing in a concentration dependent manner. The relationship will be defined and quantification is possible. Quantification by reannealing kinetics is compared to quantification using the fluorescence at the end of each extension period.

The present invention also provides dual-color fluorescence methods that depend on probe hybridization (not hydrolysis) for sequence-specific detection during PCR. In accordance with the present invention, the resonance energy transfer between adjacent hybridization probes labeled with fluorescein and Cy5 can be used to monitor amplification. However, the effect of probe length, probe concentration, and optimal distance between the probes should also be considered when utilizing embodiments of the present invention.

The present invention also provides for optimization of adjacent hybridization probes. The effect of distance between the probes is determined. In accordance with the present invention, synthesis of 5, 3'-fluorescein-labeled 30-mers and 5, 5'-Cy5-labeled 30-mers with fluorescein controlled pore glass (available from Glen Research) and Cy5 amidites (available from Pharmacia). The probes are designed to hybridize to the same strand in the β-globin region with inter-probe distances between fluorescein and Cy5 of 0 to 25 bases. High Performance Liquid Chromotography (HPLC) with tandem absorbance and fluorescence monitors are used for purification. Purity is preferably checked by analytical gel electrophoresis and A₂₆₀:A₄₉₀ or A₂₆₀:A₆₅₀ ratios. Probes will be included at 0.2 µM during PCR, and 2-temperature cycling will be used. The fluorescence ratio change during PCR is be plotted against the inter-probe distance to determine the optimal distance between probes.

Using the optimal inter-probe distance determined above, probe lengths of 20, 25, and 35 bases are synthesized and compared to the 30-mer probes. The melting temperature of these probes is observed during PCR and plotted against probe length. Use of longer probes allows use of a 3-temperature cycle with a probe annealing step preceding a primer annealing step. The effect of probe concentration on detection sensitivity and signal strength during PCR is measured.

Using a labeled primer for resonance energy transfer to a hybridization probe is in accordance with one aspect of the present invention. One of the adjacent resonance energy transfer probes can be incorporated into a PCR primer as shown in Figure 39. In the illustrated case, one labeled probe hybridizes to a single stranded PCR product that incorporates the labeled primer. The labeled primers are synthesized with a modified dT amidite with an aminolinker (available from Glen Research) for manual coupling to Cys (available from Amersham/BDS). The present invention also includes determining how close the modified Cy5-dT can be to the 3' end of the primer without inhibiting PCR. This determination requires several labeled primers with the labeled base at different positions. Next, the optimal distance between fluorophores by synthesizing and testing several 3'-fluorescein-labeled probes is found.

It is also within the scope of the present invention to utilize other formats for detecting hybridization by resonance energy transfer. In one preferred method, the adjacent hybridization probe scheme is referred to as a "noncompetitive format." This is in contrast to a "competitive format" where two complementary oligonucleotides are singly labeled with fluorophores. The probes either hybridize to each other (resulting in energy transfer or quenching) or to a competing target, which in our case would be accumulating PCR product. Synthesis and testing of 20-, 25-, and 30-mer 5'-Cy5-labeled and 3'-fluorescein-labeled complementary probes in PCR can be carried out in accordance with the present invention.

The "intercalater format" of Morrison requires a labeled probe that interacts with a double strand-specific dye when it hybridizes. See Morrison L.E., Detection of energy transfer and fluorescence quenching, in L.J. Kricka (ed.), Nonisotopic DNA probe techniques, Academic Press, San Diego, pp. 311-352, 1992 which is now incorporated herein by reference. Synthesize of 20-, 25-, and 30-mer probes dual-labeled with Cy5 at the 3' and 5' ends and use of SYBR® Green I as the double strand-specific dye is within the scope of the present invention. PCR product formation results in hybridization observable as increased emission of Cy5. This method has the advantage that both sequence-specific product and double-stranded DNA can be monitored at the same time.

The present invention also applies dual-color, sequence-specific methods for mutation detection, competitive quantitative PCR, and product quantification. The Tₘ of hybridization probes shifts about 10°C if a single base mismatch is present. If a hybridization probe is placed at a mutation site, single base mutations are detectable as a 10°C shift in probe melting. Heterozygous mutations should give an intermediate melting curve. Single base discrimination will be demonstrated using the factor V mutation mentioned above and adjacent fluorescein/Cy5 probes.

Competitive PCR templates can be designed with internal base changes. If detection probes are used that sequentially melt off different targets at different temperatures, relative quantification is possible. Absolute product quantification is possible by following probe annealing kinetics. If one labeled primer and one hybridization probe is used, the annealing kinetics should be pseudo first order, a prediction that will be tested with known template concentrations.

Figure 47 is a schematic representation of a rapid temperature cycler with fluorescence detection at the tip of capillary sample tubes, which is generally indicated at 400. Provided below is a comparison of the different embodiments of the features of the present invention disclosed herein which include fluorescence detection.

| Characteristic | Single Sample Embodiment | Multiple Sample Embodiments | Multiple samnle Embodiments |
|---|---|---|---|
| Capacity | Single samples | Multiple samples | Multiple samples |
| Light Source | LED | Xenon Arc | LED |
| Detector | Photodiode | PMTs | Photodiode |
| Optical Path | Linear | Separate | Epifluorescent |
| Emission Bands | 1, 2 | 1, 2 | 1, 2, 3, continuous |
| Capillary Illumination | Side/Tip | Side | Tip |
| Polarization Capable | Yes | No | No |

The rapid temperature cycler with fluorescence detection at the tip of capillary sample tubes represented in Figure 47 is particularly advantageous. The temperature within the sample chamber is controlled with a 400 W heating cartridge (available from Reheat, Inc.) and a DC rare earth brush motor (available from Escap AG., RPM MAX=15,000, estimated time to failure 10,000 hours) which drives a chamber fan. For heating, the cartridge is proportionally controlled and the fan is run at a low speed (12 V, 0.5 A) to provide temperature homogeneity throughout the sample chamber. For cooling, the heating cartridge is disabled and the fan is run at maximum speed (27 v, 1.4 A). The fan forces air in the central opening and out the exhaust ports. The heating and cooling elements are symmetrical around a central axis, as are up to 24 samples that are placed in a circular carousel that is positioned with a stepper motor. The stepper motor is microstepped (using devices available from New England Affiliated Technologies) to provide over 10,000 steps per revolution of the carousel.

The optical design of the embodiment represented in Figure 47 is based on paraxial epifluorescent illumination of the capillary tip as previously described (see Figure 31). The excitation source is a "super bright" blue light emitting diode (available from LEDtronics). Fluorescence signals are acquired from integrated detector/filter modules (available from Ealing Electrooptics - 0.5 inch interference filters with high performance silicon photodiodes in TO5 packages). The excitation and detection components are mounted directly on one circuit board with associated electronics. All optics are 0.5 inches in diameter.

The samples are loaded into composite plastic/glass sample containers as shown in Figures 47A-D. A 5 µℓ sample is added to each tube (Figure 47A) and centrifuged at low speed to place the samples at the capillary tips as 1 cm fluid columns (Figure 47B). The entrance port of the composite plastic/glass container is then sealed with a plastic plug (Figure 47C) and placed in the rapid temperature cycler with fluorescence detection at the tip of capillary sample tubes 400 (Figure 47D). As can be discerned from Figures 47A-D, the addition of plastic loading and sealing structures to the capillary sample tubes is a great advantage providing efficient use of glass capillary tubes while retaining the desirable thermal characteristics. Samples can be added to the composite plastic/glass sample containers and centrifuged in a 96-well format, but are loaded into the instrument individually. The composite plastic/glass sample containers fit into brass sleeves that align the tip to the optical focal point as represented in Figure 47. This fit is off-axis, allowing precise alignment of each sleeve at the time of fabrication.

Advantageously, the composite plastic/glass sample containers provide a convenient, inexpensive sample holder is provided. Glass capillary tubes having 0.8 mm ID, 1.0 mm OD, which are sealed on one end and flared at the other end (for receiving a plastic sealing device shown in Figures 47A-D) can be fabricated inexpensively. The plastic components can also be fabricated inexpensively. It is preferred that these components are assembled and placed into 96-well racks for protection and ease of loading. The shape of the tip of the capillary tube is optimized for optical efficiency. Flat tips as well as tips with various outside curvatures and inside curvature are all within the present invention.

With the embodiment of Figure 47 acquiring fluorescence from each sample 1-10 times/seconds, the samples need to be rotated relatively rapidly. With stepper motor positioning of 24 samples, each sample would be allotted 4.2-42 msec.

The user interface and instrument control for the embodiment represented in Figure 47 is the same or similar to that explained in connection with the embodiment of Figure 11. The controller for the embodiment represented in Figure 47 provides digital I/O control of the light emitting diode, the direction of the stepper motor, the heater, and the chamber fan. An efficient user interface is provided.

Figure 48 illustrates useful temperature vs. time segments for fluorescence hybridization monitoring. Product melting curves are obtained during a slow temperature increase to denaturation. By quickly lowering the temperature after denaturation to a constant temperature, product, probe, or primer annealing can optionally be followed. Probe melting curves are obtained by slowly heating through temperatures around the probe Tₘ. The embodiment represented in Figure 47 provides all analysis during temperature cycling with immediate real time display.

While embodiments of the present invention utilizing only 1- or 2-color analysis, the embodiment represented in Figure 47 is particularly adapted for 3-color analysis by adding another dichroic and photodiode/filter assembly to the collection optics (see Figure 31). Moreover, it is within the scope of the present invention to allow simultaneous separation of wavelengths onto a linear photodiode array for multicolor acquisition. When the linear photodiode array is used, the collection optics represented in Figure 47 will be replaced with a prism or diffraction grating, a lens, and a photodiode array or CCD for multiple wavelength detection. The linear photodiode array collects 15-30 wavelength bins of 10-20 nm each between 500 and 800 nm. Various configurations (such as the Littrow autocollimating configuration for gratings used in most monochrometers) are used to reach a compromise between collection efficiency, spectral resolution and spatial requirements.

The present invention also utilizes fluorescence feedback for real time control and optimization of amplification. Real time fluorescence monitoring is used to control temperature cycling. With double strand-specific dyes, extension can be terminated each cycle after fluorescence stops increasing. For denaturation, the temperature needs to increase only until the product is completely melted. Finally, temperature cycling can be automatically terminated after a certain amount of product is made. These real time controls will be programmed as options in the fluorescence temperature cycler.

Real time monitoring and control of annealing is also provided by the present invention. When one of the primers is 3'-labeled with Cy5, no extension can occur. If labeled primer (1-10%) is mixed with unlabeled primer (90-99%), amplification efficiency will be slightly decreased, but annealing is observable as fluorescence energy transfer from a double strand-specific dye to Cy5. This is the "intercalater format" previously described above. The primer with the lowest Tₘ (as determined by nearest neighbor thermodynamics) will be labeled with SYBR® Green I as the double strand-specific dye. Amplification efficiency and Cy5 fluorescence during annealing are compared against the percentage of labeled primer.

Direct monitoring with the embodiment of the present invention can be carried out directly with a Cy5-labeled primer or indirectly with equivalent complementary oligonucleotides. An oligonucleotide of the same length and Tₘ as the lowest melting primer is designed with no complementarity to the amplified sequence. This oligonucleotide is 5'-labeled with Cy5 and its complement is 3'-labeled with fluorescein. Hybridization of this pair is followed by resonance energy transfer to Cy5. The fluorescein-labeled oligonucleotide (0.005 *µ*M) and Cy5-labeled oligonucleotide (0.5 *µ*M) anneals with pseudo first order kinetics that approximates primer annealing kinetics. In this way, the efficiency of annealing can be monitored and used to control annealing temperature and times. A control of annealing conditions by monitoring annealing efficiency is optionally included in the control device.

Conventional PCR is performed by programming all cycling parameters before amplification. For continuous monitoring, determination of temperature cycling requirements occurs during amplification, based on continuous observation of annealing, extension, and denaturation. Using complementary oligonucleotides equivalent to the lowest melting primer, the annealing efficiency is controlled even during early cycles. However, extension and denaturation can only be monitored with dsDNA dyes during later cycles when enough product has been made. This is not usually a problem because denaturation and extension conditions are made permissive enough to amplify most products, and data from the first amplification can be used to optimize subsequent runs.

Further examples of product differentiation by analysis of DNA melting curves during the polymerase chain reaction will now be provided.

PCR was performed in 50 mM Tris, pH 8.5 (25°C), 3 mM MgCl2, 500 µg/ml bovine serum albumin, 0.5 mM of each deoxynucleoside triphosphate, 0.5 µM primers, and 0.2 U of Taq polymerase per 5 µℓ sample. SYBR® Green I was also included at a 1:20,000 dilution unless otherwise specified.

Amplification product used as templates were purified by phenol/chloroform extraction and ethanol precipitation, followed by repeated washing through a Centricon 30 microconcentrator (available from Amicon of Danvers, Massachusetts). Template concentrations were determined by absorbency at 260 nm and had A₂₆₀/A₂₈₀ ratios greater than 1.7.

Primers were synthesized by standard phosphoamidite chemistry (available from Pharmacia Biotech Gene Assembler Plus of Piscataway, New Jersey). The primers for the 180 base pair hepatitis B surface antigen gene amplification were 5'-CGTGGTGGAC TTCTCTCAAT-3' (SEQ ID NO:1) and 5'-AGAAGATGAG GCATAGCAGC-3'(SEQ ID NO:2). The primers for the 292 base pair prostate specific antigen gene amplification were 5'-CTGTCCGTGA CGTGGATT-3' (SEQ ID NO:7) and 5'-AAGTCCTCCG AGTATAGC-3' (SEQ ID NO:8). The primers for the 536 base pair human β-globin gene amplification were RS42 and KM29.

DNA melting curves were acquired on a microvolume fluorimeter integrated with a 24 sample thermal cycler using the embodiment of Figures 8A&B and fitted with an optics module (model no. 2210 available from Idaho Technology of Idaho Falls, Idaho) for acquisition of fluorescence from SYBR® Green I. PCR reagents (5 µℓ volume) were pipetted into the open plastic reservoir of composite glass/plastic reaction tubes, centrifuged at low speed to place the sample at the tip of the glass capillary, and sealed inside with a plastic plug. PCR was performed in one of the embodiments previously described. Fluorescence data for melting curves was acquired by integrating the signal over 0.25 - 2.0 seconds during a linear temperature transition to 95°C at 0.1 - 10.0°C/second. The data was continuously displayed using the control devices discussed earlier.

A series of transforms were used to convert the melting curve into melting peaks (see Figure 49). Background fluorescence was removed (T1) by performing linear regression (L1) on the melting curve above the melting temperature of purified PCR product. To a close approximation (R2 = 0.96 for the melting curve in Figure 49), this line expresses the background fluorescence as a function of temperature. A linear regression (L2) on the melting curve below the melting temperature of the product is used to remove the effect of temperature on fluorescence. Finally, the data are remapped from a melting curve to a melting peak (T2) by plotting the derivative of fluorescence with respect to temperature (-dF/dT) by methods similar to those used to convert spectrophotometric melting curves to melting peaks. Integrating the melting peak curve over the expected melting range of the specific product, divided by the integral over the entire melting peak, gives an upper limit to the ratio of intended to nonspecific product.

Plotting fluorescence from dsDNA specific dyes as a function of temperature (see Figure 50) during PCR allowed monitoring of strand status during individual cycles of the reaction. Early temperature cycles appeared as a background fluorescence across the bottom of the plot. As amplification product accumulated, the fluorescence rose higher with each cycle during the transition to the annealing temperature and as polymerase activity synthesized new product. During heating toward the denaturation temperature, the fluorescence suddenly fell to background levels over the span of a few degrees indicating product denaturation. This sequence resulted in the clockwise spiral structure characteristic of fluorescence vs. temperature plots of PCR.

After 25 temperature cycles a melting curve acquisition cycle was initiated (see Figure 51). The product was denatured, fully annealed and then slowly heated through the denaturation temperature at 0.2°C/second. Melting began at 85°C and fluorescence fell to background levels by 87°C.

The absolute position of PCR product melting curves were affected by the concentration of SYBR® Green I (see Figure 52A). As the concentration of dye was increased, the melting curves were shifted to higher temperatures. Concentrations above 1 to 7,000 prevented amplification by PCR.

Rapid temperature transitions through the denaturation temperature shifted melting curves to higher apparent Tₘ because of kinetic effects (see Figure 52B). Temperature transition rates greater than 5°C/sec shifted the apparent T, by nearly 3°C. Apparent Tₘ was shifted 0.2°C by increasing the transition rate from 0.1 to 0.2°C/second. Subsequent melting curve analysis was done at a transition rate of o.2°C/second. Melting curve analysis at this rate added 30 seconds to the 8 minute reaction represented in Figure 50.

Melting curves were acquired for three different purified PCR products (see Figure 53). All three products had sharp melting curves, melting within 2°C. The apparent Tₘ of the 180 base pair hepatitis B fragment (50.0% GC) was about two degrees lower than the Tₘ of the 536 base pair β-globin fragment (53.2% GC), and these were easily differentiable. The apparent Tₘ of the 292 base pair prostate-specific antigen fragment (60.3% GC) had a melting temperature nearly five degrees higher than the larger β-globin fragment.

When purified PCR products were mixed, their melting curves overlapped (see Figure 54). After conversion into melting peaks, it was possible to resolve mixtures of reaction products that differed in Tₘ by less than 2°C into separate peaks (see Figure 55).

Melting curve analysis was used to differentiate intended product from nonspecific products such as primer dimers (see Figure 56). Primer dimers had broad melting peaks in the 75-85°C range, very different from the sharp melting peaks of specific PCR amplification products. Larger heterogeneous products which resulted from running many cycles at low annealing stringency had lower and broader melting curves when compared with pure PCR product.

The present invention's approach to product purity determination was used to improve quantitative PCR based on once-per-cycle monitoring of fluorescence of dsDNA specific dyes. Fluorescence was acquired once each cycle after polymerase extension of the product for a series of reactions varying in initial template concentration (see Figure 57). The log-linear increase above background fluorescence begins at a cycle number dependent on initial template concentration. The plots of the five reactions ranging from 10⁶ to 10² copies per reaction were separated by about four cycles. The reaction with an average 10² copies per reaction showed a decrease in reaction efficiency, and the reactions with initial copy number below 100 gave fluorescence profiles that were less useful. The fluorescence profiles for the reactions containing 10 and 1 (average) copies rise in reverse order, and the negative control showed amplification after about 30 cycles. This is due to amplification of primer dimers and other nonspecific amplification products that cannot be distinguished from the intended product by once-per-cycle fluorescence monitoring of dsDNA specific dyes.

Melting peaks were acquired for each sample (see Figure 58) and these were found to correlate well with electrophoresis results (Figure 60). The reaction containing zero and one average initial template copies produced no discernible electrophoresis band at the expected 536 base pair location. The reactions containing 10 and 100 initial copies of template show weak electrophoresis bands. This agrees well with the melting peak analysis, which shows no DNA melting in the range of the intended product (90 - 92°C) for the reactions containing zero and one initial copies and small peaks in this temperature range for 10 and 100 copies. Strong electrophoresis bands for the reactions containing 10³ - 10⁶ initial copies correlate well with large melting peaks in the expected 90 - 92°C range.

The ratio of intended product to total product, determined by melting peak integration, ranged from 0.28 for 10⁵ copies to 0.0002 for zero initial template copies. Each data point of Figure 57 was multiplied by the appropriate ratio to give the corrected plot (Figure 59). This procedure extends the effective dynamic range of quantitation to between 10 and 1 initial template copies.

As will be appreciated from an understanding of the foregoing, ideally, PCR product detection and analysis would occur simultaneously with temperature cycling during amplification. If a fundamental property of DNA such as product size, sequence, or melting profile could specifically identify the product during PCR, no further analysis would be required.

Sequence-specific detection during amplification can be obtained with fluorescent probes. A single probe can be labeled with two fluorescence resonance energy transfer (FRET) dyes. Hydrolysis of the probe by polymerase exonuclease activity releases the donor dye from quenching. As the amplification proceeds, the fluorescence from the donor dye increases cumulatively. Alternately, FRET dyes can be placed on 2 single-labeled probes which hybridize adjacently. Both hydrolysis and hybridization techniques allow simultaneous amplification and sequence-specific product detection. However, unique probes are required for each target. Probe synthesis and purification are not trivial, particularly for dual-labeled probes.

Helix/coil stability can also be used to specifically identify PCR products. For example, single-strand conformation polymorphism, denaturing gradient gel electrophoresis, and temperature gradient gel electrophoresis, all separate products based on the stability of different conformations. Hybridization stability can be monitored in real time with FRET. Monitoring of melting curves has also been used as an additional discriminating dimension in sequencing by hybridization techniques.

Ethidium homodimer and ethidium bromide is used to monitor DNA denaturation. PCR is compatible with many dsDNA specific dyes, including ethidium bromide and SYBR® Green I. These dyes are readily available and inexpensive; probe synthesis is not required. If ethidium bromide is included in PCR, an increase in fluorescence from samples in glass capillary sample tubes occurs. It is within the scope of the present invention to monitor PCR reactions as they occurred with ultraviolet illumination and a CCD camera. As product identity is established, monitoring with these generic dyes would eliminate the need for a separate analysis step after amplification. Absolute sequence specificity is seldom needed, but product differentiation is usually required.

PCR products can be differentiated during amplification by analysis of melting curves whose shape is a function of GC content, length, and sequence. The temperature at which PCR products melt varies over a large range. Using empirical formulas known in the art, the effect of GC content on the melting temperature (Tₘ) of DNA predicts that a 0% GC duplex would melt 41°C lower than a 100% GC duplex. Given the same GC content, a 40 base pair primer dimer would melt 12°C below a 1000 bp product. Hence, the range of Tₘ for potential PCR products spans at least 50°C. This wide range allows most PCR products to be differentiated by melting curves. Because of length differences, primer dimers usually melt at a lower temperature than desired PCR products. Heterogeneous products melt over a wider range than pure PCR products. Long PCR products may have internal melting domains that could be used as a fingerprint for identification, although smaller PCR products, including those tested here (see Figure 53), melt in one major transition.

Unlike gel electrophoresis, melting curve analysis can distinguish products of the same length but different GC/AT ratio. In addition, two products with the same length and GC content, but differing in their GC distribution (equally distributed vs a GC clamp on one end) would have very different melting curves. Small sequence differences may even be observable for example, in the amplification of heterozygous DNA where heteroduplexes are formed. Single base differences in heterozygous DNA result in temperature shifts of 1-5°C for heteroduplexes compared to the fully complementary DNA.

PCR products separated by less than 2°C can be distinguished within mixtures using the present invention (see Figure 56). Furthermore, these melting curves can be resolved into product peaks for estimates of the relative amount of different products present (see Figure 61). The ability to determine the relative amounts of different PCR products is very useful. For example, knowledge of the relative amount of intended product compared to total dsDNA can correct for nonspecific amplification when reactions are monitored by dsDNA dyes. Using ethidium bromide or SYBR® Green I, quantitation over 6-8 orders of magnitude of initial template concentration is possible. However, sensitivity is limited by nonspecific amplification at low initial template copy number (see Figure 57). Melting curves (see Figure 58) show multiple PCR products, particularly at low initial copy number, and this correlates well with the multiple products observed after gel electrophoresis (see Figure 60). The ratio of product melting in the expected range to total product can be used to correct for nonspecific amplification (see Figure 59). This ratio gives an upper limit to the amount of expected versus total product. Just as it is possible to misread electrophoresis results because of comigration of two or more products, it is possible that some of the product melting in the expected range may not be intended product. However, if no dsDNA melts in the range of the expected product, it can conclusively be said that none of the expected product is present.

Melting curve analysis can be used to extend the dynamic range of quantification to lower initial copy number. More importantly, melting peak analysis provides a greater level of certainty that the fluorescence derives from the intended product rather than nonspecific amplification. The usefulness of fluorescence monitoring in PCR is limited if gel electrophoresis is required to check the identity of the product. Melting peak analysis allows product identification during PCR without subsequent electrophoresis.

The ability to resolve the relative amounts of 2 PCR products should is also useful in competitive quantitative PCR. Competitive templates with the same primers are designed in accordance with the present invention with a melting temperature 2 - 3°C away from the natural amplicon by changing the GC/AT ratio or the length of the product. Known amounts of competitor would be added to unknown amounts of natural template, the templates amplified, melting curves acquired, and product peaks resolved. Assuming that the products amplify with the same efficiency, the ratio between products is the ratio between the initial templates.

Melting curve analysis has several limitations. The absolute position and width of melting curves are affected by temperature transition rates (see Figure 52B) and dye concentration (Figure 52A). The addition of intercalaters like ethidium bromide increases the melting temperature and broadens the melting transition. Although melting curves are often obtained at rates of about 0.5°C/minute to ensure equilibrium, rates of 12°C/minute (0.2°C/second) can be used to differentiate products differing by **2°C** or less in melting temperature (see Figure 53). Finer resolution is possible with slower transition rates and/or greater sample temperature homogeneity.

The melting peak analysis of the present invention offers a product differentiation method fully integrated with PCR. Similar to sizing by gel electrophoresis, melting peak analysis measures a fundamental characteristic of DNA and can be used to identify amplified products. The combination of fluorescence monitoring of PCR with melting curve analysis provides a promising approach for simultaneous amplification, detection, and differentiation of PCR products.

Information regarding an on-line DNA Analysis System with Rapid Thermal Cycling is found in U.S. Patent Application serial no. 08/381,703 filed January 31, 1995 which is now incorporated herein in its entirety.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

The Programming Code Appendix follows on the next page.

### PROGRAMMING CODE APPENDIX

NEW
1 GOTO 60:REM ***I/O stepper (I=$(0), O=z(72))***
3 IF (XBY(65507).AND.64)=0 THEN Z(0)=XBY(65506) : GOTO 3
4 DO : WHILE (XBY(65507).AND.128)=128
5 I=0 : DO : I=I+1 : XBY(65506)=ASC($(0),I)
6 WHILE ASC($(0),I)<>13 : RETURN
7 DO : WHILE (XBY(65507).AND.64)=64
8 I=0 : DO : Z(I)=XBY(65506)
9 I=I+1 : UNTIL Z(I-1)=32 : RETURN

10 REM ***error check***
15 IF TX<0 THEN TX=0
16 RETURN
20 IF TX<-251 THEN TX=-251
21 RETURN
25 IF TX>251 THEN TX=251
26 RETURN

30 GOSUB 50:TP1=TP:T1=TIME
32 DO:UNTIL TIME>T1+0.2
34 GOSUB 50:TP2=TP:T2=TIME
36 SL=(TP2-TP1)/(T2-T1):IF SL<>0 THEN IT=(TG-TP2)/SL
38 PRINT USING(###.##),"SL=",SL,"IT=",IT,"AT TEMP=",TP2
40 IF IT>1 GOTO 30
42 RETURN

49 REM ***temperature measurement***
50 E=XBY(65504):F=XBY(65505):G=XBY(65504):TP=(F*256+G+300)/38.3: RETURN

60 CLEAR : STRING 150,72 : DIM Z(72)
72 DTEMP=95:DTIM=1:DS=10
74 ATEMP=60:ATIM=1:AS=20
76 ETEMP=74:ETIM=15:ES=1:CYCLE=100
80 PRINT USING(###),"#",TAB (20),"TEMP",TAB (30),"TIME",TAB (40),"SLOPE"
82 PRINT "1) DENATURATION", TAB(20),DTEMP,TAB(30),DTIM,TAB(40),DS
84 PRINT "2) ANNEALING",TAB(20),ATEMP,TAB(30),ATIM,TAB(40),AS
86 PRINT "3) EXTENSION",TAB(20),ETEMP,TAB(30),ETIM,TAB(40),ES
88 PRINT "4) CYCLES ",CYCLE:PRINT
110 INPUT "TYPE # TO CHANGE OR 0 IF OK (CHOICES ARE 0,1,2,3, OR 4)?",J
114 IF J=0 THEN GOTO 170
116 ON J GOSUB ,120,130,150,160
118 PRINT:GOTO 80
120 INPUT "ENTER DENATURATION Temp, Time, Slope: ",DTEMP,DTIM,DS
125 IF DTEMP>98 GOTO 120
126 RETURN
130 INPUT "ENTER ANNEALING Temp, Time, Slope: ",ATEMP,ATIM,AS
140 IF AS<0 GOTO 130
142 IF AS>21 GOTO 130
145 RETURN
150 INPUT "ENTER EXTENSION Temp, Time, Slope: ",ETEMP,ETIM,ES:RETURN
160 INPUT "ENTER Number of Cycles:",CYCLE:RETURN
170 $(0)="msr1 500;sp1 200;hf1 2;rt2 1;esr" : GOSUB 3 : GOSUB 7
175 FOR I=0 TO 10 : PRINT CHR(Z(I)), : NEXT I : PRINT

200 $(0)="go1 nd;hf1 2;wfo =.x..;s1":GOSUB 3: rem open
210 PRINT "WAIT FOR COOLING":XBY(65504)=3
220 DO : GOSUB 50 : UNTIL TP<45
230 PRINT "TEMP=",TP," LOAD SAMPLES AND THEN PRESS A KEY"
240 W=GET : IF W=0 GOTO 240

300 TMC=0 :WS=0: CLOCK 0 : TIME=0 : DBY(71)=0 : CLOCK 1
305 $(0)="hf1 1;sp1 100;go1;wfo =x...;s1":GOSUB 3: REM CLOSE
310 FOR V=1 TO CYCLE
315 PRINT "D", : TG=DTEMP:XBY(65509)=4:IF V=1 THEN TG=DTEMP+(DS/4)

320 WS=(DS*10+WS*9)/10:TX=INT(WS+0.5):GOSUB 15
325 XBY(65508)=TX:GOSUB 50:IF TP>TG GOTO 340
330 IF TX<>DS*10 GOTO 320
333 GOSUB 30
335 GOSUB 50:IF TP<TG GOTO 335
340 TG=DTEMP:EB=TIME
345 WS=(6*WS+10*(TG-TP))/7 :TX=INT(WS+0.5)
350 IF TX>=0 THEN GOSUB 25:XBY(65508)=TX:XBY(65509)=4
355 IF TX<0 THEN GOSUB 20:XBY(65508)=0:XBY(65509)=4-TX
360 IF V=1 THEN IF DTIM+5>(TIME-EB) GOTO 380
370 IF DTIM<(TIME-EB) GOTO 500
380 GOSUB 50 : GOTO 345

500 PRINT "A",:XBY(65508)=0:TG=ATEMP
845 $(0)="spl 200;gol nd;hfl 2;wfo =.x..;sl":GOSUB 3: rem open
855 XBY(65509)=AS*10
860 GOSUB 30
890 EB=TIME:WS=-AS*10
913 WS=(4*WS+20*(TG-TP))/5:TX=INT(WS+0.5)
915 IF TX>=0 THEN GOSUB 25:XBY(65508)=TX:XBY(65509)=4
918 IF TX<0 THEN GOSUB 20:XBY(65508)=0:XBY(65509)=4-TX
920 IF ATIM+1<(TIME-EB) GOTO 925
924 GOSUB 50 : GOTO 913

925 PRINT:PRINT "E ",:XBY(65509)=4:TG=ETEMP+1
930 $(0)="hfl 1;spl 50;gol;wfo =x...;sl":GOSUB 3:WS=0: REM CLOSE
935 WS=(ES*10+WS*9)/10:TX=INT(WS+0.5):GOSUB 15:GOSUB 25:XBY(65508)=TX
937 GOSUB 50:IF TP>TG-1 GOTO 970
940 IF TX<>ES*10 GOTO 935
950 GOSUB 50 : IF TP<TG-1 GOTO 950
970 EB=TIME
980 WS=(WS+5*(TG-TP))/2 :TX=INT(WS+0.5)
983 IF TX>=0 THEN GOSUB 25:XBY(65508)=TX:XBY(65509)=4
987 IF TX<0 THEN GOSUB 20:XBY(65508)=0:XBY(65509)=4-TX
990 IF ETIM<(TIME-EB) GOTO 1000
995 GOSUB 50 : GOTO 980
1000 CTIM=TIME-TMC : TMC=TIME : PRINT "CYCLE=",V,"TIME=",CTIM
1050 NEXT V

1055 XBY(65508)=0:XBY(65509)=255
1060 INPUT "STRAND REANNEALING (Y/N) ?",$(1)
1070 IF ASC($(1),1)=78 GOTO 1290
1100 PRINT "STRAND REANNEALING IN PROCESS..."
1110 TG=DTEMP:XBY(65509)=4
1120 WS=(DS*10+WS*9)/10:TX=INT(WS+0.5):GOSUB 15
1130 XBY(65508)=TX:GOSUB 50:IF TP>TG GOTO 1170
1140 IF TX<>DS*10 GOTO 1120
1150 GOSUB 30
1160 GOSUB 50:IF TP<TG GOTO 1160
1170 EB=TIME
1180 WS=(11*WS+10*(TG-TP))/12 :TX=INT(WS+0.5)
1185 IF TX>=0 THEN GOSUB 25:XBY(65508)=TX:XBY(65509)=4
1190 IF TX<0 THEN GOSUB 20:XBY(65508)=0:XBY(65509)=4-TX
1193 IF 5<(TIME-EB) GOTO 1200
1196 GOSUB 50 : GOTO 1180
1200 XBY(65508)=0:XBY(65509)=255:WS=0
1210 DO:GOSUB 50:UNTIL TP<75

1220 EB=TIME
1230 WS=(WS+5*(75-TP))/2 :TX=INT(WS+0.5)
1240 IF TX>=0 THEN GOSUB 25:XBY(65508)=TX:XBY(65509)=4
1250 IF TX<0 THEN GOSUB 20:XBY(65508)=0:XBY(65509)=4-TX
1260 IF 60<(TIME-EB) GOTO 1280
1270 GOSUB 50 : GOTO 1230

1280 XBY(65508)=0:XBY(65509)=255
1290 INPUT "ANOTHER RUN (Y/N) ?",$(1)
1300 IF ASC($(1),1)=78 GOTO 2000
1400 PRINT:$(0)="gol nd;hf1 9;wfo =.x..;s1":GOSUB 3:GOTO 80
2000 $(0)="gol nd;hf1 9;wfo =.x..;s1":GOSUB 3:END

## Claims

1. A device for monitoring the fluorescence of a sample, said device comprising
a chamber;
a capillary tube having a high surface area to volume ratio and comprising an optically transparent material;
a capillary tube holder;
a light source mounted in said chamber and positioned to illuminate the capillary tube through an end of the capillary tube; and
a light detector mounted in said chamber and positioned to measure fluorescence from the capillary tube through an end of the capillary tube.

2. The device of claim 1 wherein the capillary tube holder comprises a carousel for holding a plurality of capillary tubes, said carousel being rotatably mounted in said chamber, said device further comprising
a stepper motor for rotating said carousel; and
means for coupling said carousel to said motor.

3. The device of claims 1 or 2 wherein the chamber is further provided with a heater and a fan mounted in said device in air flow communication with the chamber to rapidly cycle the temperature of the chamber.

4. The device of claim 1 wherein the capillary tube comprises a sample delivery port and a funnel cap for filling said capillary tube, wherein the ratio of the surface area to the volume of the capillary tube is greater than or equal to 4mm⁻¹, said funnel cap having a first sample receiving orifice and a sample transfer orifice and means for releasably engaging the sample delivery port of the capillary tube so that the sample transfer port of the funnel cap and the sample delivery port of the capillary tube are in alignment.

5. The device of claim 4 further comprising a plug for frictional fit sealing engagement with the sample receiving port of the funnel cap.

6. The device of claim 1 wherein the capillary tube is one of a plurality of capillary tubes, the device flirther comprising
a heater and a fan in air flow communication with the chamber and a controller for cycling the temperature in the chamber according to initial predefined temperature and time parameters;
a carousel for holding the plurality of capillary tubes, said carousel being rotatably mounted in said chamber; and
means for displaying the status of the reaction based detected fluorescence; wherein the light source is positioned to illuminate at least one of said capillary tubes through an end of the capillary tube and the light detector is positioned to measure fluorescence from at least one of said capillary tubes through an end of the capillary tube;

7. The device of claim 6 further comprising means for adjusting the controller such that one or more reaction parameters are adjusted in real time.

8. The device of claim 6 or 7 wherein each of the capillary tubes has an inner diameter ranging from about 0.02mm to about 1.0mm.

9. The device of claim 6 or 7 further comprising a stepper motor for rotating the carousel to position sequentially said capillary tubes held by said carousel for illumination and fluorescence detection.

10. A method for analyzing a target DNA sequence of a biological sample for DNA sequence polymorphisms, heterozygosity or mutations, said method comprising the steps of
amplifying the target sequence by polymerase chain reaction in the presence of two nucleic acid probes that hybridize to adjacent regions of the target sequence, one of said probes being labeled with an acceptor fluorophore and the other probe labeled with a donor fluorophore of a fluorescence energy transfer pair such that upon hybridization of the two probes with the target sequence, the donor and acceptor fluorophores are within 0 to 25 nucleotides of one another, said polymerase chain reaction comprising the steps of adding a thermostable polymerase and primers for the targeted nucleic acid sequence to the biological sample and thermally cycling the biological sample between at least a denaturation temperature and an elongation temperature;
exciting the biological sample with light at a wavelength absorbed by the donor fluorophore and detecting the emission from the biological sample.

11. A method for analyzing a target DNA sequence of a biological sample for DNA sequence polymorphisms, heterozygosity or mutations, said method comprising the steps of
amplifying the target sequence by polymerase chain reaction in the presence of two nucleic acid probes that hybridize to adjacent regions of the target sequence, one of said probes being labeled with an acceptor fluorophore and the other probe labeled with a donor fluorophore of a fluorescence energy transfer pair such that upon hybridization of the two probes with the target sequence, the donor and acceptor fluorophores are within 0 to 25 nucleotides of one another, said polymerase chain reaction comprising the steps of adding a thermostable polymerase and primers for the targeted nucleic acid sequence to the biological sample and thermally cycling the biological sample between at least a denaturation temperature and an elongation temperature;
exciting the biological sample with light at a wavelength absorbed by the donor fluorophore; and
monitoring the temperature dependent fluorescence from the biological sample.

12. A method of real time monitoring of a polymerase chain reaction amplification of a targeted nucleic acid sequence in a biological sample, said method comprising the steps of
(a) adding to the biological sample an effective amount of two nucleic acid primers and a nucleic acid probe, wherein one of said primers and the probe are each labeled with one member of a fluorescence energy transfer pair comprising an acceptor fluorophore and a donor fluorophore, and wherein the labeled probe hybridizes to an amplified copy of the target nucleic acid sequence within 0 to 25 nucleotides of the labeled primer;
(b) amplifying the targeted nucleic acid sequence by polymerase chain reaction, said polymerase chain reaction comprising the steps of adding a thermostable polymerase and primers for the targeted nucleic acid sequence and thermally cycling the biological sample between at least a denaturation temperature and an elongation temperature;
(c) illuminating the biological sample with light of a selected wavelength that is absorbed by said doaor fluorophore and detecting the fluorescence emission of the sample.

13. The method of claim 12 further comprising the step of monitoring the temperature dependent fluorescence of the sample.

14. An improved method of amplifying a targeted nucleic acid sequence of a biological sample, said method comprising the steps of
(a) adding to the biological sample an effective amount of two nucleic acid probes that hybridize to adjacent regions of the target sequence, one of said probes being labeled with an acceptor fluorophore and the other probe labeled with a donor fluorophore of fluorescence energy transfer pair such that upon hybridization of the two probes with the target sequence, the donor and acceptor fluorophores are within 0 to 25 nucleotides of one another;
(b) amplifying the targeted nucleic acid sequence using polymerase chain reaction, comprising thermally cycling the biological sample using predetermined temperature and time parameters;
(c) illuminating the biological sample with a selected wavelength of light that is absorbed by said donor fluorophore during the polymerase chain reaction;
(d) monitoring fluorescent emissions from said sample; and
(e) adjusting the temperature and time parameters in accordance with the data generated from step (d) to optimize product yield or specificity.

15. The method of claim 10,11,12,13 or 14 wherein the resonance energy transfer pair comprises fluorescein as the donor and Cy5 as the acceptor.

16. A device for conducting PCR reactions said device comprising
a chamber;
a heater and a fan mounted in said device and in air flow communication with the chamber;
carousel for holding a plurality of capillary tubes, said carousel being rotatably mounted in said chamber;
said capillary tubes comprising an optically transparent material;
a light source mounted in said chamber and positioned to illuminate at least one of the capillary tubes through an end of a capillary tube; and
a light detector mounted in said chamber and positioned to measure fluorescence from at least one of the capillary tubes through an end of a capillary tube.

17. A method for enhancing detection and efficiency of acquisition of fluorescence in a sample comprising a fluorophore, said method comprising the steps of
placing the sample in a capillary tube comprising an optically transparent material; and
detecting the fluorescence through an end of the capillary tube.

18. The method of claim 17 wherein the fluorescence is induced by fluorophore-excitatory illumination of the sample and the method further comprises illuminating the sample through an end of the capillary tube.

19. The method of claim 17 wherein the fluorescence is induced by fluorophore-excitatory illumination of the sample and the method further comprises illuminating the sample along the optical path of the detected fluorescence.

20. The method of claim 17, 18 or 19 wherein the ratio of the surface area to the volume of the capillary tube is greater than or equal to 4mm⁻¹.

21. The method of claim 17 or 18 wherein the fluorescence of the biological sample is temperature dependent and the method further comprises the step of heating or cooling the biological sample during detection of fluorescence.

22. The method of claim 17,18 or 21 wherein the sample contains a fluorescence energy transfer pair and the fluorescence of at least one fluorophore of the fluorescence energy transfer pair is detected.

23. The method of claim 17 or 18 wherein the sample comprises nucleic acids including nucleic acid probes, one of said probes labeled with an acceptor fluorophore and the other probe labeled with a donor fluorophore of a fluorescence energy transfer pair and wherein the fluorescence detected is that of the donor fluorophore or the acceptor fluorophore or both.

24. A handling system for a sample comprising a capillary tube having a sample delivery port and a funnel cap for filling said capillary tube, said capillary tube comprising an optically transparent material, wherein the ratio of the surface area to the volume of the capillary tube is greater than or equal to 4mm⁻¹, said funnel cap having a first sample receiving orifice and a sample transfer orifice and means for releasably engaging the sample delivery port of the capillary tube so that the sample transfer port of the funnel cap and the sample delivery port of the capillary tube are in alignment.

25. The sample handling system of claim 24 further comprising a plug for frictional fit sealing engagement with the sample receiving port of the funnel cap.

26. A system for performing PCR and monitoring the reaction in real time comprising:
a chamber;
a heater and a fan mounted in said device and in air flow communication with the chamber and a controller for cycling the temperature in the chamber according to initial predefined temperature and time parameters;
a carousel for holding a plurality of capillary tubes, said carousel being rotatably mounted in said chamber, said capillary tubes comprising an optically transparent material, and having a high surface area to volume ratio that provides for rapid thermal transfer;
a light source mounted in said chamber and positioned to illuminate at least one of said capillary tubes through an end of the capillary tube; and
a light detector mounted in said chamber and positioned to measure fluorescence from at least one of said capillary tubes through an end of the capillary tube;
means for displaying the status of the reaction based detected fluorescence.

27. The system of claim 26 further comprising means for adjusting the controller such that one or more reaction parameters are adjusted in real time.

28. The system of claim 26 or 27 wherein each of the capillary tubes has an inner diameter ranging from about 0.02mm to about 1.0mm.

29. The system of claim 26 and 27 further comprising a stepper motor for rotating the carousel to position sequentially said capillary tubes held by said carousel for illumination and fluorescence detection.

30. A method of real time monitoring of a polymerase chain reaction amplification of a targeted nucleic acid sequence in a biological sample, said method comprising the steps of
amplifying the target sequence by polymerase chain reaction in the presence of two nucleic acid probes that hybridize to adjacent regions of the target sequence, one of said probes being labeled with an acceptor fluorophore and the other probe labeled with a donor fluorophore of a fluorescence energy transfer pair such that upon hybridization of the two probes with the target sequence, the donor and acceptor fluorophores are within 0 to 25 nucleotides of one another, said polymerase chain reaction comprising the steps of adding a thermostable polymerase and primers for the targeted nucleic acid sequence to the biological sample and thermally cycling the biological sample between at least a denaturation temperature and an elongation temperature;
exciting the biological sample with light at a wavelength absorbed by the donor fluorophore and detecting the emission from the biological sample; and
monitoring the temperature dependent fluorescence from the biological sample.

31. The method of claim 30 wherein the resonance energy transfer pair comprises fluorescein as the donor and Cy5 as the acceptor.

32. A method for analyzing a target DNA sequence of a biological sample for DNA sequence polymorphisms, heterozygosity or mutations, said method comprising the steps of
(a) adding to the biological sample an effective amount of two nucleic acid primers and a nucleic acid probe, wherein one of said primers and the probe are each labeled with one member of a fluorescence energy transfer pair comprising an acceptor fluorophore and a donor fluorophore, and wherein the labeled probe hybridizes to an amplified copy of the target nucleic acid sequence within 0 to 25 nucleotides of the labeled primer;
(b) amplifying the targeted nucleic acid sequence by polymerase chain reaction, said polymerase chain reaction comprising the steps of adding a thermostable polymerase and primers for the targeted nucleic acid sequence and thermally cycling the biological sample between at least a denaturation temperature and an elongation temperature;
(c) illuminating the biological sample with light of a selected wavelength that is absorbed by said donor fluorophore and detecting the fluorescence emission of the sample.

33. The method of claim 32 further comprising the step of monitoring the temperature dependent fluorescence of the sample.

34. The method of claim 32 wherein the resonance energy transfer pair comprises fluorescein as the donor and Cy5 as the acceptor.


SEQUENCE LISTING

(1) GENERAL INFORMATION:

(i) APPLICANT: University of Utah Research Foundation

(ii) TITLE OF INVENTION: SYSTEM AND METHOD FOR CARRYING
OUT AND MONITORING BIOLOGICAL
PROCESSES

(iii) NUMBER OF SEQUENCES: 6

(iv) CORRESPONDENCE ADDRESS:
(A) ADDRESSEE: University of Utah Foundation
(B) STREET: 210 Park Building
(C) CITY: Salt Lake City
(D) STATE: Utah
(E) COUNTRY: United States of America
(F) ZIP: 84112

(v) COMPUTER READABLE FORM:
(A) MEDIUM TYPE: Diskette, 3.5 inch, 1.4 Mb storage
(B) COMPUTER: Toshiba 200CDS
(C) OPERATING SYSTEM: DOS 7.0
(D) SOFTWARE: Word Perfect 7.0

(vi) CURRENT APPLICATION DATA:
(A) APPLICATION NUMBER:
(B) FILING DATE: June 4, 1997
(C) CLASSIFICATION:

(vii) PRIOR APPLICATION DATA:
(A) APPLICATION NUMBER:
(B) FILING DATE:

(viii) ATTORNEY/AGENT INFORMATION:
(A) NAME: Grant R. Clayton
(B) REGISTRATION NUMBER: 32,462
(C) REFERENCE/DOCKET NUMBER: 8616CIP5

(ix) TELECOMMUNICATION INFORMATION:
(A) TELEPHONE: (801)566-6633
(B) TELEFAX: (801)566-0750

(2) INFORMATION FOR SEQ ID NO:1:

(i) SEQUENCE CHARACTERISTICS:
(A) LENGTH: 20
(B) TYPE: nucleic acid

(C) STRANDEDNESS: single-stranded
(D) TOPOLOGY: linear

(xi) SEQUENCE DESCRIPTION: SEQ ID NO:1:

CGTGGTGGAC TTCTCTCAAT 20

(2) INFORMATION FOR SEQ ID NO:2:

(i) SEQUENCE CHARACTERISTICS:
(A) LENGTH: 20
(B) TYPE: nucleic acid
(C) STRANDEDNESS: single-stranded
(D) TOPOLOGY: linear

(xi) SEQUENCE DESCRIPTION: SEQ ID NO:2:

AGAAGATGAG GCATAGCAGC 20

(2) INFORMATION FOR SEQ ID NO:3:

(i) SEQUENCE CHARACTERISTICS:
(A) LENGTH: 35
(B) TYPE: nucleic acid
(C) STRANDEDNESS: single-stranded
(D) TOPOLOGY: linear

(xi) SEQUENCE DESCRIPTION: SEQ ID NO:3:

CAAACAGACA CCATGGTGCA CCTGACTCCT GAGGA 35

(2) INFORMATION FOR SEQ ID NO:4:

(i) SEQUENCE CHARACTERISTICS:
(A) LENGTH: 30
(B) TYPE: nucleic acid
(C) STRANDEDNESS: single-stranded
(D) TOPOLOGY: linear

(xi) SEQUENCE DESCRIPTION: SEQ ID NO:4:

AAGTCTGCCG TTACTGCCCT GTGGGGCAAG 30

(2) INFORMATION FOR SEQ ID NO:5:

(i) SEQUENCE CHARACTERISTICS:
(A) LENGTH: 22

(B) TYPE: nucleic acid
(C) STRANDEDNESS: single-stranded
(D) TOPOLOGY: linear

(xi) SEQUENCE DESCRIPTION: SEQ ID NO:5:

GGTTGGCCAA TCTACTCCCA GG 22

(2) INFORMATION FOR SEQ ID NO:6:

(i) SEQUENCE CHARACTERISTICS:
(A) LENGTH: 20
(B) TYPE: nucleic acid
(C) STRANDEDNESS: single-stranded
(D) TOPOLOGY: linear

(xi) SEQUENCE DESCRIPTION: SEQ ID NO:6:

GCTCACTCAG TGTGGCAAAG 20